(19) Europäisches Patentamt
European Patent Office
Office européen des brevets

(11) Publication number: **0 441 226 A1**

(12) # EUROPEAN PATENT APPLICATION

(21) Application number: **91101126.0**

(22) Date of filing: **29.01.91**

(51) Int. Cl.[5]: **C07D 401/04**, C07D 401/06, C07D 401/14, A61K 31/445, A61K 31/495, A61K 31/535

(30) Priority: **29.01.90 ES 9000245**
**30.11.90 ES 9003307**

(43) Date of publication of application:
**14.08.91 Bulletin 91/33**

(84) Designated Contracting States:
**AT BE CH DE DK ES FR GB GR IT LI LU NL SE**

(71) Applicant: **J. URIACH & CIA. S.A.**
**Degà Bahi, 59-67**
**E-08026 Barcelona(ES)**

(72) Inventor: **Carceller, Elena**
**Arag n 117**
**E-08015 Barcelona(ES)**

Inventor: **Almansa, Carmen**
**Sta Eulalia 114**
**E-08902 Hosp. de Llobregat, Barcelona(ES)**
Inventor: **Pere J., Jimenez**
**Jaume I**
**E-08025 Flix, Tarragona(ES)**
Inventor: **Bartroli, Javier**
**Vives i Tut 55, 2o 2a**
**E-08034 Barcelona(ES)**

(74) Representative: **Zumstein, Fritz, Dr. et al**
**Dr. F. Zumstein Dipl.-Ing. F. Klingseisen**
**Bräuhausstrasse 4**
**W-8000 München 2(DE)**

(54) **New (cyanomethyl)pyridines.**

(57) 57 The present invention relates to new (cyanomethyl)pyridines of general formula **I**:

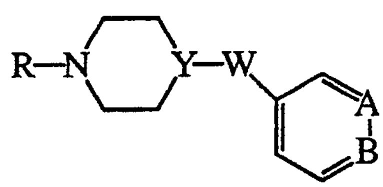

**I**

wherein **A** is nitrogen and **B** is -CH-, or **B** is nitrogen and **A** is -CH-; the group **Y-W-** represents a group of formula N-$CR_1$(CN)-, N-$CH_2$-CH(CN)-, CH-CH(CN)- or C=C(CN)- wherein $R_1$ is hydrogen or a $C_1$-$C_6$ alkyl group; **R** represents a group of formula $R_2$CO-, $R_3R_4$N-$(CH_2)_n$CO- or $R_5$-$(CH_2)_m$- wherein $R_2$ represents $C_1$-$C_{15}$ alkyl, aryl, aryl-($C_1$-$C_6$)-alkyl, aryl-($C_1$-$C_6$)-alkenyl, diaryl-($C_1$-$C_6$)-alkyl, diaryl-($C_1$-$C_6$)-alkenyl, arylhydroxy-($C_1$-$C_6$)-alkyl, diarylhydroxy-($C_1$-$C_6$)-alkyl, aryl-($C_1$-$C_6$)-alkoxy, diaryl-($C_1$-$C_6$)-alkoxy, or heteroaryl-($C_1$-$C_6$)-alkyl group; n is 0, 1, 2 or 3; $R_3$ is hydrogen, $C_1$-$C_6$ alkyl, aryl or aryl-($C_1$-$C_6$)-alkyl group; $R_4$ is $C_3$-$C_6$ cycloalkyl, aryl, aryl-($C_1$-$C_6$)-alkyl, diaryl-($C_1$-$C_6$)-alkyl, aryl-($C_1$-$C_6$)-alkylcarbonyl or diaryl-($C_1$-$C_6$)-alkylcarbonyl group; m is 0, 1 or 2; and $R_5$ is aryl-($C_1$-$C_6$)-alkyl, diaryl-($C_1$-$C_6$)-alkyl, diaryl-($C_1$-$C_6$)-alkylcarbonylamino or diaryl-($C_1$-$C_6$)-alkylaminocarbonyl group. These compounds are potent, orally active PAF antagonists and consequently, useful in the treatment of the diseases in which this substance is involved.

EP 0 441 226 A1

## NEW (CYANOMETHYL)PYRIDINES.

### Field of the invention.

The present invention relates to new (cyanomethyl)pyridines with potent antagonist activity of the platelet activating factor (PAF). The invention also relates to the pharmaceutical preparations which contain these compounds and their use in the treatment of the diseases in which PAF is involved, such as allergic and bronchial asthma, platelet aggregation disorders, septic shock, hypertension, inflammation, etc.

### Description of the prior art.

The platelet activating factor (PAF) or (1-*O*-alkyl-2-acetyl-*sn*-glyceryl-3-phosphorylcholine), also called acetyl glyceryl ether phosphorylcholine (AGEPC) or PAF-acether is a natural phospholipid synthesized by different cells (basophiles, macrophages, neutrophiles, platelets) and tissues (heart, lung and kidney) of the organism.

PAF was described for the first time as a potent platelet aggregating agent. Later on it was demonstrated to have other biological activities *in vivo*, such as peripheral vasodilatation, increase of the vascular permeability, induction of bronchoconstriction and hyperreactivity of the respiratory tract. PAF also produces immediate hypotension followed by pulmonary and renal hypertension in rats, guinea pigs, rabbits and dogs, and it has been rated as the most potent ulcerogenic agent described until now. Consequently, PAF is a mediator that is implicated in a large set of pathological processes such as asthma, septic shock, transplant rejection, thrombosis, ulceration, inflammation and renal diseases.

Even though its mechanism of action is still not known with precision, several studies show that the biological activities of PAF involve the existence of a specific receptor. Recently, the isolation of one of these receptors from human platelets has been achieved and it has been identified as a protein with a molecular weight of 160.000 daltons. On the other hand, the capacity to inhibit the binding of $^{3}$H-PAF to their receptors is well correlated with the amount of PAF needed to provoke the *in vitro* and *in vivo* observed effects. These facts indicate that the compounds that act as specific antagonists of PAF could result of interest for the treatment of all those pathological processes related directly or indirectly with PAF.

Until now, several PAF analogues with a potent PAF antagonist activity have been investigated. However, the ionic nature of these compounds usually is associated with a deficient and erratic oral absorption. Although, several non ionic compounds have been described in the literature and they show a potent antagonist activity of PAF when are administered orally (cfr. for example EP 0284359, EP 194416), the provision of good PAF antagonist suitable to be used in therapy is still an unresolved problem.

### Description of the invention.

This invention relates to new (cyanomethyl)pyridines which are potent, orally active PAF antagonist and represent a new structural type of PAF antagonist. Thus, the present invention provides (cyanomethyl)-pyridines of general formula **I**:

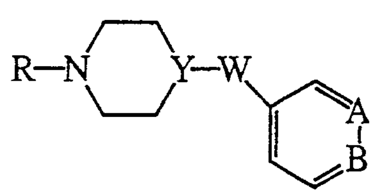

**I**

wherein **A** is nitrogen and **B** is -CH-, or **B** is nitrogen and **A** is -CH-;
the group

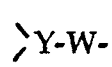

represents a group of formula

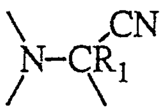 , 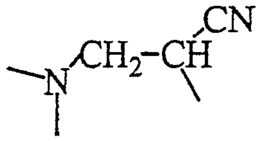 , 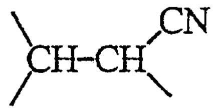 or 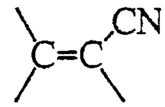

wherein $R_1$ is hydrogen or a $C_1$-$C_6$ alkyl group;
**R** represents a group of formula

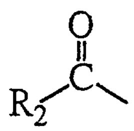 , 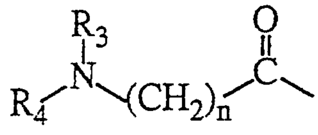 or 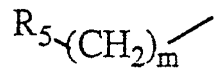

wherein $R_2$ represents $C_1$-$C_{15}$ alkyl, aryl, aryl-($C_1$-$C_6$)-alkyl, aryl-($C_1$-$C_6$)-alkenyl, diaryl-($C_1$-$C_6$)-alkyl, diaryl-($C_1$-$C_6$)-alkenyl, arylhydroxy-($C_1$-$C_6$)-alkyl, diarylhydroxy-($C_1$-$C_6$)-alkyl, aryl-($C_1$-$C_6$)-alkoxy, diaryl-($C_1$-$C_6$)-alkoxy, heteroaryl-($C_1$-$C_6$)-alkyl group or a group of formula

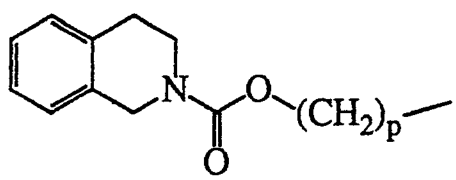

where p = 2 or p = 3; n is 0, 1, 2 or 3;
R3 is hydrogen, $C_1$-$C_6$ alkyl, aryl or aryl-($C_1$-$C_6$)-alkyl group;
$R_4$ is $C_3$-$C_6$ cycloalkyl, aryl, aryl-($C_1$-$C_6$)-alkyl, diaryl-($C_1$-$C_6$)-alkyl, aryl-($C_1$-$C_6$)-alkylcarbonyl or diaryl-($C_1$-$C_6$)-alkylcarbonyl group, or $R_3R_4N$- is a group of formula

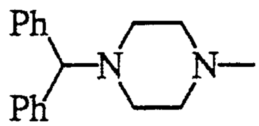 or 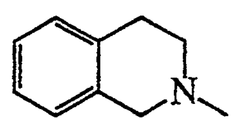

m is 0, 1 or 2;
$R_5$ is aryl-($C_1$-$C_6$)-alkyl, diaryl-($C_1$-$C_6$)-alkyl, diaryl-($C_1$-$C_6$)-alkylcarbonylamino or diaryl-($C_1$-$C_6$)-alkylaminocarbonyl group; being understood that the term "aryl" (or its symbol Ar) means phenyl or a radical from a fused or unfused benzenoid hydrocarbon (e.g. naphtyl, biphenyl, fluorenyl), and can be optionally substituted by one, two or three $C_1$-$C_6$ alkyl, $C_1$-$C_6$ haloalkyl, $C_1$-$C_6$ alkoxy, halogen, nitro, cyano, hydroxy, $C_1$-$C_6$ alkylcarbonyl, $C_1$-$C_6$ alkylcarbonyloxy, $C_1$-$C_6$ alkyloxycarbonyl, $C_1$-$C_6$ alkylsulfonyl, $C_1$-$C_6$ alkylsulfinyl and $C_1$-$C_6$ alkylthio group; and that the term "heteroaryl" means any radical from a 5 or 6 membered heterocycle, isolated or condensed with one benzene ring, with one ring heteroatom chosen from N, O or S, or with two ring heteroatoms chosen from the pairs N/N, N/O or N/S.

The compounds of the present invention can exist in the form of different stereoisomers each one of which, as well as their mixtures, are object of the present invention. The salts of compounds **I** with pharmaceutically acceptable acids are also the object of the present invention.

Preferred embodiments of the present invention are those in which the corresponding compounds are those of formula **Ia**

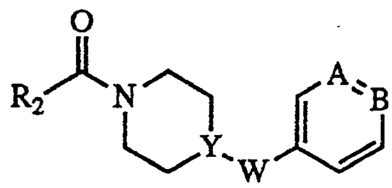

**Ia**

wherein **A**, **B** and the group

>Y-W-

have the previously defined meaning and $R_2$ is a $C_1$-$C_{15}$ alkyl radical, a group of formula $Ar(CH_2)_q$–, ArCHMe-, $(Ar)_2CH$-$(CH_2)_q$-, $(Ar)_2C(Me)$-, $ArCH_2$-CHAr-, $ArCH_2$-$CH_2$-CHAr-, $ArCH_2$-$CH(CH_2Ar)$-, ArCH = CH-, ArCH = CAr-, $(Ar)_2C$ = CH-, ArCH(OH)-$(CH_2)_q$-, $(Ar)_2C(OH)$-$(CH_2)_q$-, $(Ar)_2CH$-$(CH_2)_q$-O-, or Ar-$(CH_2)_q$-O- wherein q is an integer from 0 to 3 and Ar has the previously defined meaning, an heteroaryl-($C_1$-$C_6$)-alkyl group, or a group of formula

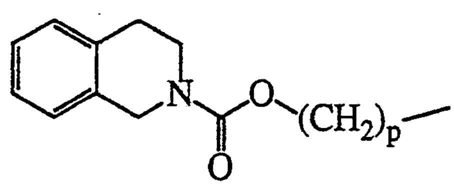

where p is 2 or 3; those of formula **Ib**

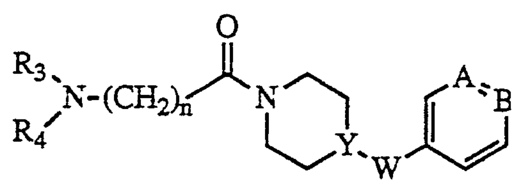

**Ib**

wherein **A**, **B**, the group

>Y-W-,

n and $R_3$ have the previously defined meaning and $R_4$ is a group of formula $Ar(CH_2)_q$-, ArCHMe-, $(Ar)_2CH$-, $(Ar)_2C(Me)$-, $(Ar)_2CH$-$(CH_2)_q$-, $ArCH_2$-CHAr-, Ar(C = O)-, or $(Ar)_2CH$-(C = O)-, wherein q and Ar have the previously defined meaning, cyclohexyl, or $R_3R_4N$- is a group of formula

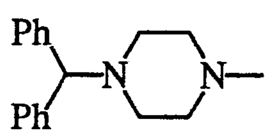

or

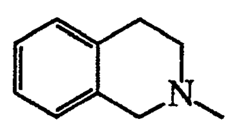

and those of formula **Ic**

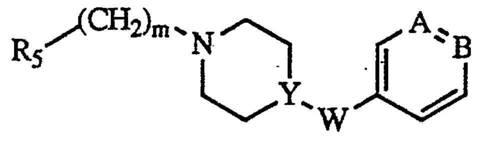

Ic

wherein **A**, **B**, the group

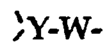

and m have the previously defined meaning and $R_5$ is a group of formula $Ar(CH_2)_q$-, $(Ar)_2CH-(CH_2)_q$-, $(Ar)_2CH-NH$-, $(Ar)_2CH-(CH_2)_q-(C=O)NH$-, or $(Ar)_2CH-(CH_2)_q-NH(C=O)$-, wherein q and Ar have the previously defined meaning.

Still more preferred are those embodiments in which the corresponding compounds are those of formula **Ia** wherein **A** and **B** have the previously defined meaning; the group

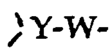

represents:

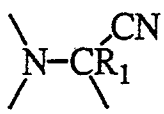 , 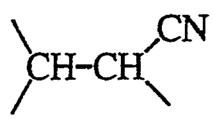 or 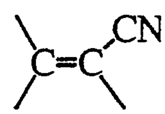

wherein $R_1$ have the previously defined meaning; and $R_2$ is a group of formula ArCHMe-, $(Ar)_2CH-(CH_2)_q$-, $(Ar)_2C(Me)$-, $ArCH_2-CHAr$-, $ArCH_2-CH_2-CHAr$-, $ArCH_2-CH(CH_2Ar)$-, ArCH = CH-, ArCH = CAr-, $(Ar)_2C=CH$-, $(Ar)_2C(OH)-(CH_2)_q$-, or $(Ar)_2CH-(CH_2)_q-O$-, wherein q and Ar have the previously defined meaning;

and those of formula **Ib** wherein **A**, **B**, and n have the previously defined meaning; the group

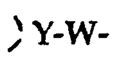

represents:

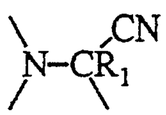 , 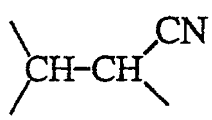 or 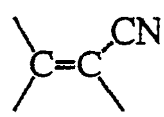

wherein $R_1$ have the previously defined meaning; $R_3$ is hydrogen, Ar or $Ar(CH_2)_q$-, and $R_4$ is a group of formula $Ar(CH_2)_q$-, ArCHMe-, $(Ar)_2CH$-, $(Ar)_2C(Me)$-, $(Ar)_2CH-(CH_2)_q$-, $ArCH_2-CHAr$-, Ar(C=O)-, or $(Ar)_2CH-(C=O)$-, wherein q and Ar have the previously defined meaning.

The most preferred are those embodiments in which the corresponding compounds are those of formula **Ia**

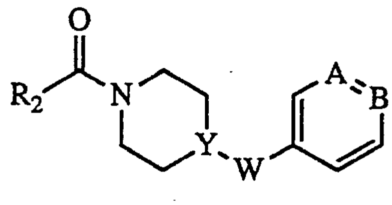

**Ia**

wherein **A** and **B** have the previously defined meaning; the group

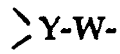

represents:

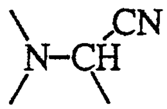 or 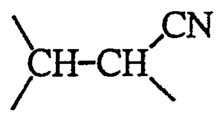

and $R_2$ is a group of formula $(Ph)_2CH$-, $(Ph)_2CH$-$CH_2$, $(Ph)_2C=CH$- or $(Ph)_2C(OH)$-$CH_2$-; and those of formula **Ib**

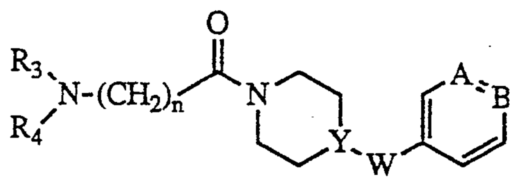

**Ib**

wherein **A** and **B** have the previously defined meaning; the group

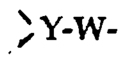

represents:

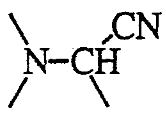 or 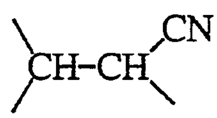

n is 1 or 2; $R_3$ is hydrogen or methyl and $R_4$ is PhCHMe- or $(Ph)_2CH$-.

In the above mentioned definitions, the term "$C_1$-$C_6$ alkyl" means, unless otherwise indicated, a linear or branched alkyl group that contain one to six carbon atoms. Consequently, this term include, among others methyl, ethyl, propyl, isopropyl, butyl, isobutyl, sec-butyl, tert-butyl, pentyl, isopentyl, neopentyl, hexyl, isohexyl, 1-methylpentyl, 2-methylpentyl, 3-methylpentyl.

"Aryl"group is preferred to be phenyl, or a radical from a fused or unfused benzenoid hydrocarbon (e.

g. naphthyl, biphenyl, fluorenyl). It has been indicated that this aryl group can be optionally substituted by one two or three substituents; examples of them are: 2-chlorophenyl, 3-chlorophenyl, 4-chlorophenyl, 4-fluorophenyl, 2-methoxyphenyl, 3-methoxyphenyl, 4-methoxyphenyl, 2,4-dimethoxyphenyl, 3,4,5-trimethoxyphenyl, 4-nitrophenyl.

The "aryl-($C_1$-$C_6$)-alkyl" group means a group resulting from sustitution of one hydrogen atom of the above mentioned "$C_1$-$C_6$ alkyl"group by an "aryl" group and includes among others, benzyl, 1-phenylethyl, 3-phenylpropyl, 2-phenylpropyl, 1-methyl-2-phenylethyl, 4-phenylbutyl, 3-phenylbutyl, 2-phenylbutyl, 1-phenylbutyl, 5-phenylpentyl, 4-phenylpentyl, 3-phenylpentyl, 2-phenylpentyl, 6-phenylhexyl, 5-phenylhexyl, 4-phenylhexyl, 1-naphthylmethyl, 2-naphthylmethyl, 2-(1-naphthyl)ethyl, 2-(2-naphthyl)ethyl, 1-(1-naphthyl)-ethyl 3-(1-naphthyl)propyl, 3-(2-naphthyl)propyl

The "aryl-($C_1$-$C_6$)-alkenyl"group means a group resulting from sustitution of one hydrogen atom of $C_1$-$C_6$ alkenyl group by an "aryl" group and include among others, 2-phenylethenyl, 3-phenyl-1-propenyl, 3-phenyl-2-propenyl, 4-phenyl-1-butenyl.

The "diaryl-($C_1$-$C_6$)-alkyl"group is preferred to be derived from a "$C_1$-$C_6$ alkyl" group by substitution of two optional hydrogen atoms by two "aryl" groups and include among others, diphenylmethyl, 2,2-diphenylethyl, 1,1-diphenylethyl, 1,2-diphenylethyl, 3,3-diphenylpropyl, 2,2-diphenylpropyl, 1,1-diphenylpropyl, 3,2-diphenylpropyl, 1,3-diphenylpropyl, 1,2-diphenylpropyl, 2-phenyl-2-naphthylethyl.

The "diaryl-($C_1$-$C_6$)-alkenyl" group means a group resulting from sustitution of two optional hydrogen atoms of the $C_1$-$C_6$ alkenyl group by two "aryl" groups and include among others, 2,2-diphenyletenyl, 1,2-diphenylethenyl, 1-benzyl-2-phenylethenyl.

The "$C_1$-$C_6$ cycloalkyl" group contains 3 to 6 carbon atoms and includes cyclopropyl, cyclobutyl, cyclopentyl and cyclohexyl.

The "heteroaryl-($C_1$-$C_6$)-alkyl" group is formed by the linkage of a heterocyclic group and an above mentioned "$C_1$-$C_6$ alkyl" group and include 2-indolylmethyl, 3-indolylmethyl, 3-pyridylmethyl, 2-thiazolylmethyl, 2-(2-indolyl)ethyl, 2-(3-indolyl)ethyl, 1-(3-indolylethyl, 2-(2-thiazolyl)ethyl, 2-(2-thienyl)ethyl, 2-(3-thienyl)ethyl, 2-(2-furyl)ethyl, 2-(3-furyl)ethyl.

The "arylhydroxy-($C_1$-$C_6$)-alkyl" and "diarylhydroxy-($C_1$-$C_6$)-alkyl" groups are preferentially derived from the substitution of one hydrogen atom of the alkylic chain of an "aryl-($C_1$-$C_6$)-alkyl" or "diaryl-($C_1$-$C_6$)-alkyl"group respectively, by a hydroxy group, and include among others, phenylhydroxymethyl, diphenylhydroxymethyl, 2,2-diphenyl-2-hydroxyethyl, 3,3-diphenyl-2-hydroxypropyl, 3,3-diphenyl-3-hydroxypropyl.

The "aryl-($C_1$-$C_6$)-alkoxy" and "diaryl-($C_1$-$C_6$)-alkoxy" groups are preferred to be derived from the union of an "aryl-($C_1$-$C_6$)-alkyl" or "diaryl-($C_1$-$C_6$)-alkyl" group to an oxygen atom of an ether functional group, such as, for instance, 2-phenylethoxy, 2,2-diphenylethoxy, 3,3-diphenylpropoxy, 1,2-diphenylethoxy, 2,3-diphenylpropoxy.

The "aryl-($C_1$-$C_6$)-alkylcarbonyl" and "diaryl-($C_1$-$C_6$)-alkylcarbonyl" groups are preferred to be derived from the union of an "aryl-($C_1$-$C_6$)-alkyl" or "diaryl-($C_1$-$C_6$)-alkyl" group, like the above mentioned, to a carbonyl group. Examples of those are phenylacetyl, 3-phenylpropanoyl, 4-phenylbutanoyl, diphenylacetyl, 3,3-diphenylpropanoyl, 2,2-diphenylpropanoyl, 2,3-diphenylpropanoyl, 2-phenyl-2-naphtylacetyl, 3-phenyl-3-naphtylpropanoyl.

The "diaryl-($C_1$-$C_6$)-alkylcarbonylamino" group is preferred to be formed by substitution of one hydrogen atom of an amino group by the "diaryl-($C_1$-$C_6$)-alkylcarbonyl" group above mentioned and include among others, diphenylacetylamino, 3,3-diphenylpropanoylamino, 2,2-diphenylpropanoylamino, 2,3-diphenylpropanoylamino, 2-phenyl-2-naphtylacetylamino, 3-phenyl-3-naphtylpropanoylamino.

The "diaryl-($C_1$-$C_6$)-alkylaminocarbonyl" group is preferred to be formed by the union of the "diaryl-($C_1$-$C_6$)-alkyl" group above mentioned to the nitrogen of -NH(C=OR)- diradical. Examples of those include, diphenylmethylaminocarbonyl, 2,2-diphenylethylaminocarbonyl, 1,1-diphenylethylaminocarbonyl, 1,2-diphenylethylaminocarbonyl, 3,3-diphenylpropylaminocarbonyl, 2,2-diphenylpropylaminocarbonyl, 1,1-diphenylpropylaminocarbonyl, 3,2-diphenylpropylaminocarbonyl, 1,3-diphenylpropylaminocarbonyl, 1,2-diphenylpropylaminocarbonyl, 2-phenyl-2-naphtylethylaminocarbonyl.

Furthermore, among all of them, they are still more preferred the specific compounds whose formulas are represented below, together with the number corresponding to the example in which their preparation is described:

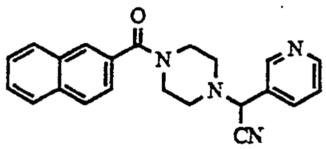

1

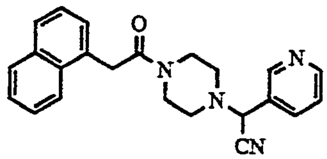

2

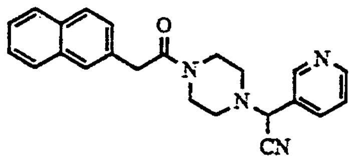

3

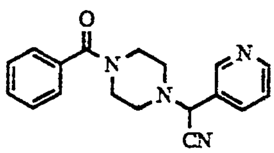

4

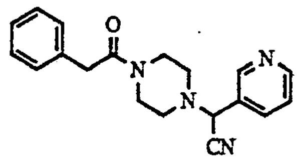

5

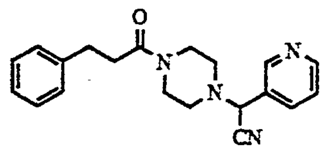

6

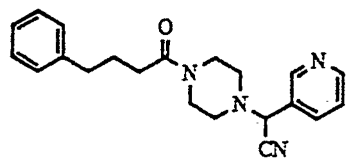

7

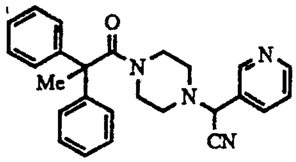
8

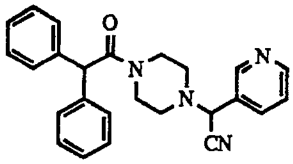
9

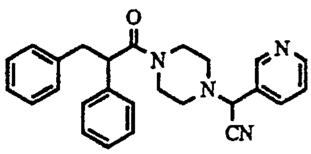
10

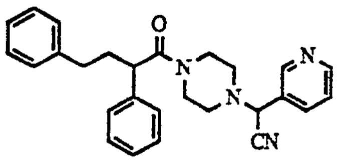
11

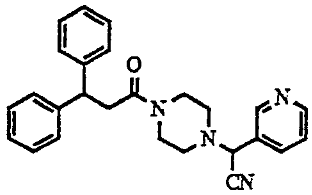
12

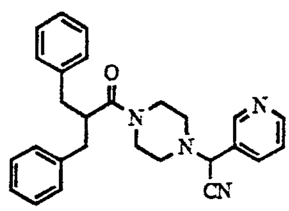
13

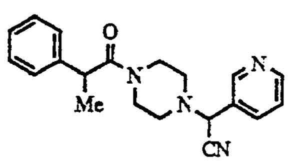
14

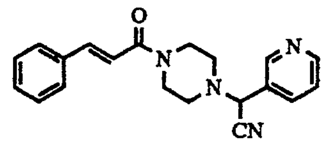
15

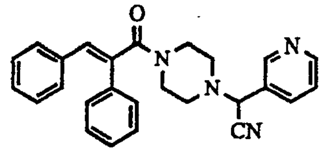
16

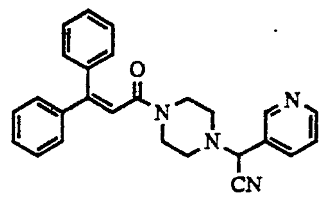
17

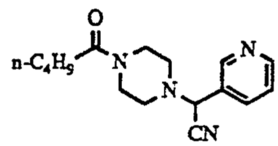
18

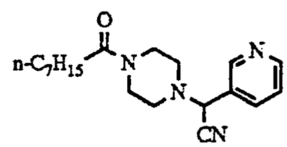
19

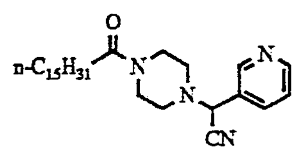
20

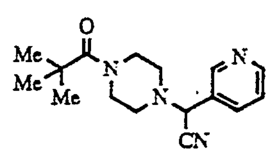
21

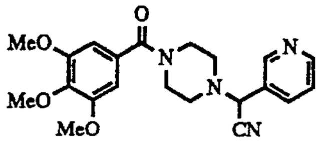

22

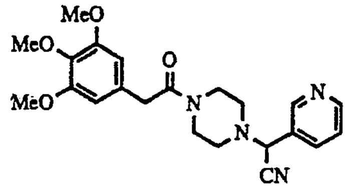

23

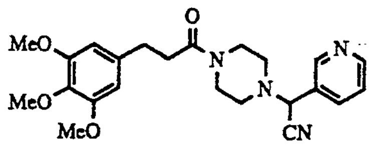

24

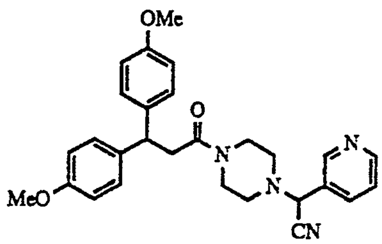

25

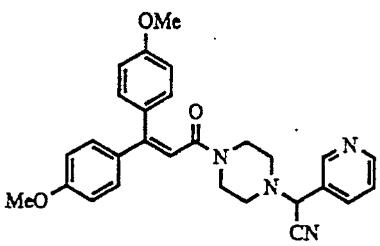

26

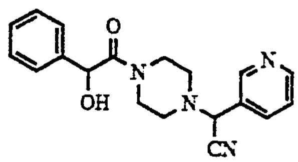

27

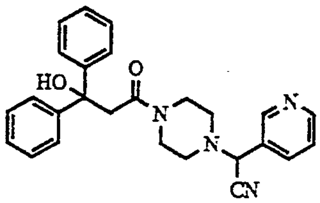

28

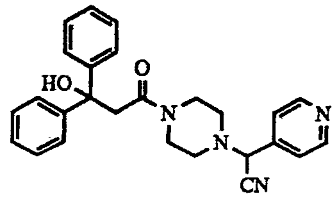

29

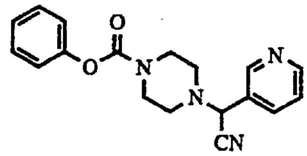

30

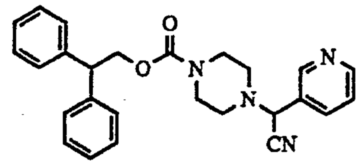

31

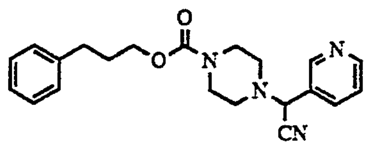

32

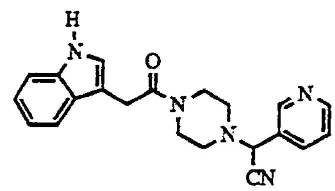

33

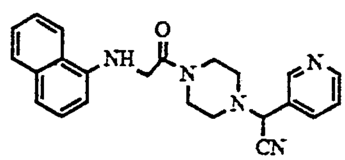

34

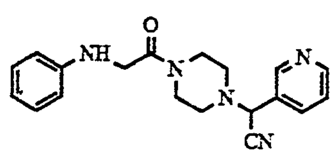

35

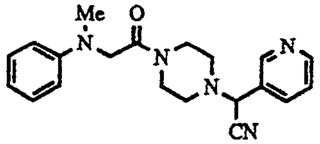

36

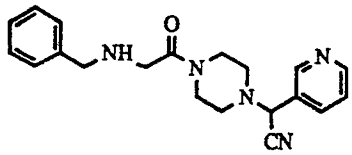

37

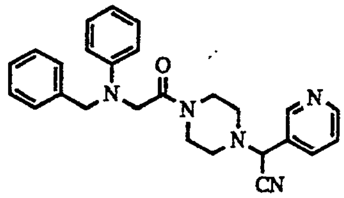

38

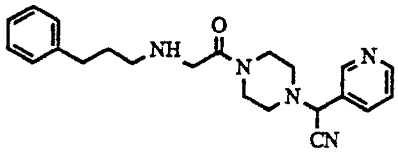

39

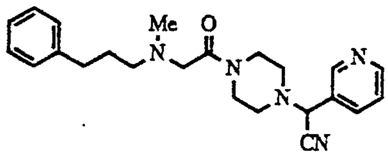

40

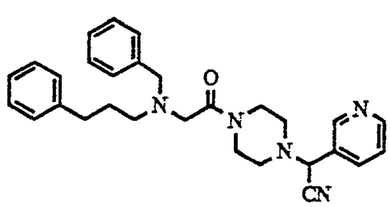

41

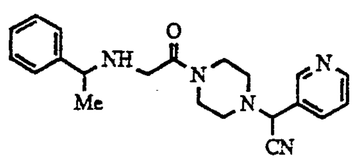

42

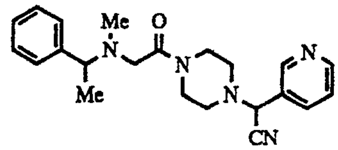

43

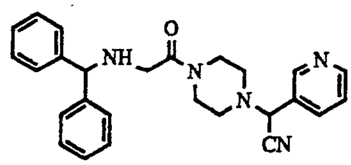

44

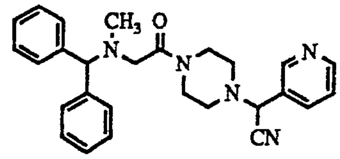

45

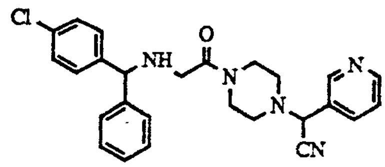

46

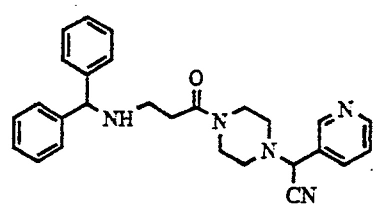

47

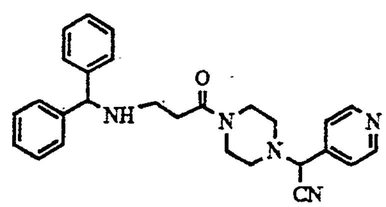

48

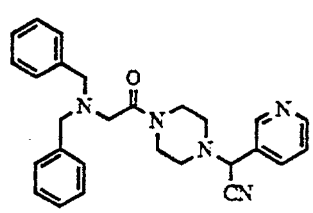

49

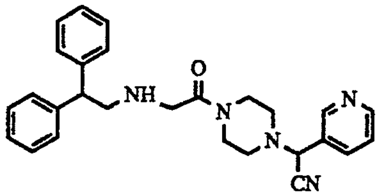
50

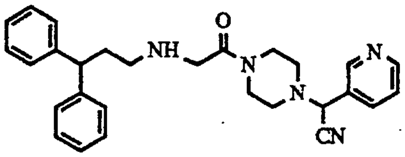
51

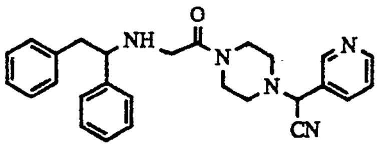
52

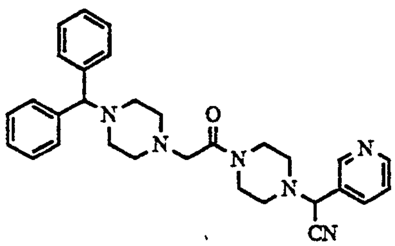
53

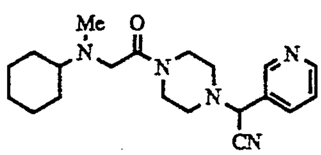
54

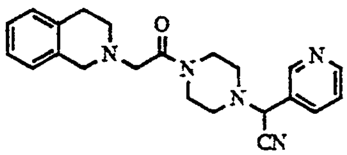
55

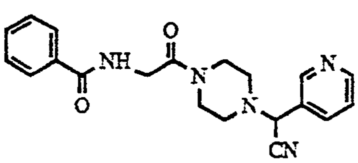
56

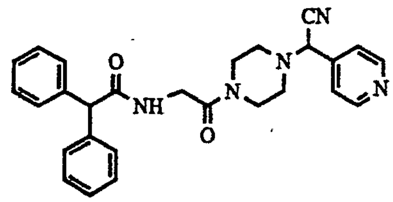
57

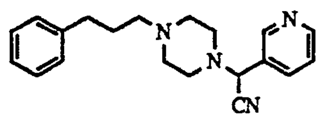
58

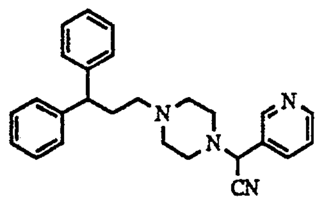
59

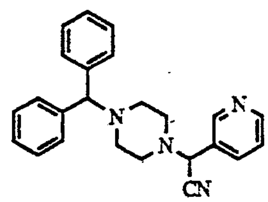
60

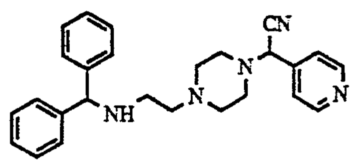
61

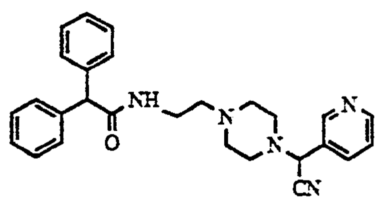
62

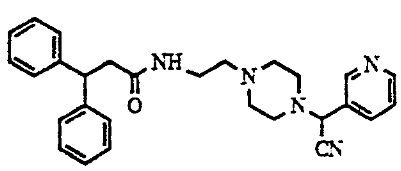
63

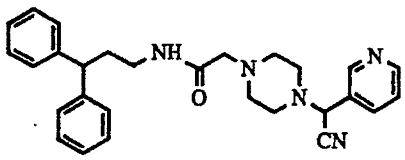

64

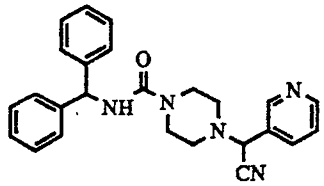

65

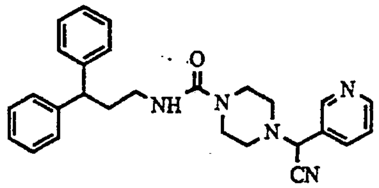

66

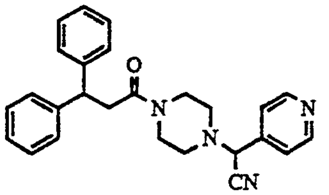

67

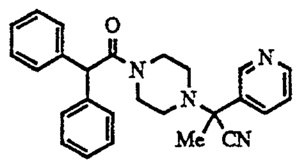

68

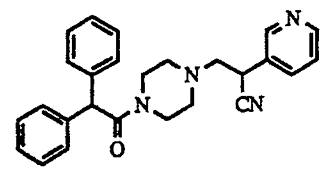

69

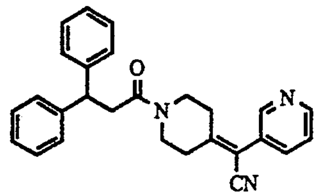

70

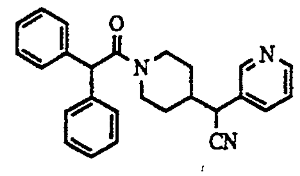

71

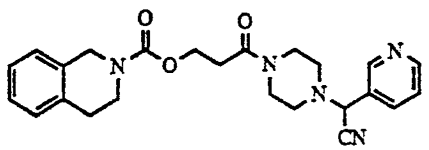

72

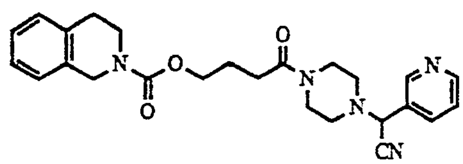

73

The compounds of the present invention have one or more basic nitrogens and, consequently, they can form salts, which are also part of the present invention. There is no limitation on the nature of these salts but pharmaceutically acceptable ones are preferred. Examples of these salts include: salts with an inorganic acid such as hydrochloric acid, hydrobromic acid, hydriodic acid, nitric acid, perchloric acid, sulfuric acid, or phosphoric acid; and salts with an organic acid, such as methanesulfonic acid, trifluoromethanesulfonic acid, ethanesulfonic acid, benzenesulfonic acid, *p*-toluenesulfonic acid, fumaric acid, oxalic acid or maleic acid.

The compounds of the present invention can exist as different diastereoisomers and/or optical isomers because the $\alpha$ carbon to the nitrile group is chiral and in some cases, there is an additional chiral center. Diastereoisomers can be separated by conventional techniques such as chromatography or fractional crystallization. The optical isomers can be separated by resolution using any of the usual techniques of optical resolution to give optically pure isomers. Such a resolution can be done in any chiral synthetic intermediate as well as in the products of general formula **I** . The optically pure isomers can also be individually obtained by using enantiospecific synthesis. The present invention covers the individual isomers as well as their mixtures (racemic mixtures, for instance), being irrelevant if they have been obtained by synthesis or have been prepared physically mixing them up.

The invention also provides processes for preparing the compounds of formula **I**.

Compounds of formula **Ia** (**I**, **R** = $\mathbf{R_2CO}$) are prepared by a process of dehydration between the amines **II-V** (**A**, **B**,

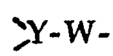

have the previously defined meaning) and the carboxylic acids of general formula **$R_2$COOH** wherein **$R_2$** has the previously defined meaning.

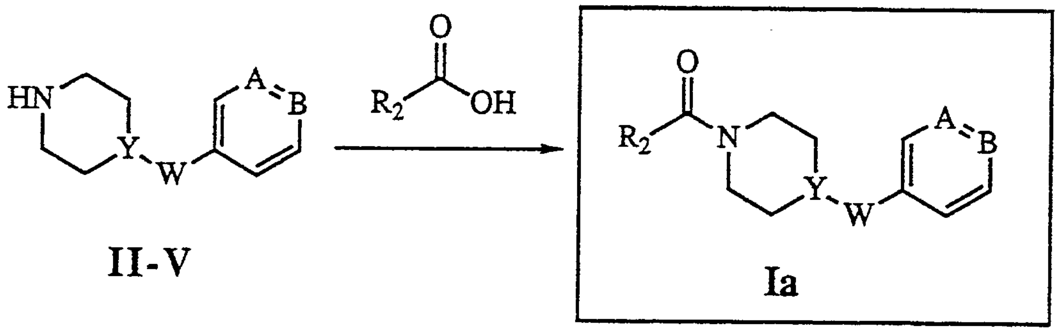

II-V

Ia

This process of dehydration can be conducted by applying any conventional method of amide bond formation such as the following processes:

a) By reaction of the amines **II-V** with an acid of general formula **$R_2$COOH** in the presence of dicyclohexylcarbodiimide and 1-hydroxybenzotriazole in a suitable solvent; as examples of suitable solvents can be mentioned: dioxane, tetrahydrofuran, acetonitrile, chloroform and *N,N*-dimethylformamide. The reaction is carried out at a temperature ranging from 0 to 60 °C during a reaction time from 6 to 24 hours.

b) By reaction of the amines **II-V** with an acid chloride, or anhydride, derived from an acid of general formula **$R_2$COOH** in the presence of a proton scavenger amine, such as pyridine or triethylamine, in a suitable solvent, such as methylene chloride or chloroform. The reaction is carried out at a temperature ranging from 0 °C to the boiling point of the solvent, during a reaction time from 6 to 24 hours.

Compounds of general formula **Ia** (**I, R = $R_2$CO**) wherein **$R_2$** is an arylalkoxy or diarylalkoxy group, can be prepared by reaction of amines **II-V** with a carbonic acid derivative of formula **$R_2$(C = O)$G_1$**, wherein **$G_1$** is a good leaving group, such as phenoxy or chloro. The reaction is carried out in the presence of a base, such as potassium carbonate, sodium bicarbonate, sodium hydroxide, pyridine, triethylamine, in a suitable solvent, such as acetonitrile, chloroform, methylene chloride, toluene or *N,N*-dimethylformamide.

Compounds of formula **Ib** (**I, R = $R_3R_4N(CH_2)_nCO$**) are prepared from amines **II-V** (**A, B,**

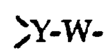

are as defined above) and carboxylic acids of general formula **$R_3R_4N(CH_2)_nCOOH$**, where **$R_3$**, **$R_4$** and **n** have the previous defined meaning, by a dehydration process that can be performed in identical conditions to those indicated for the preparation of compounds of general formula **Ia**.

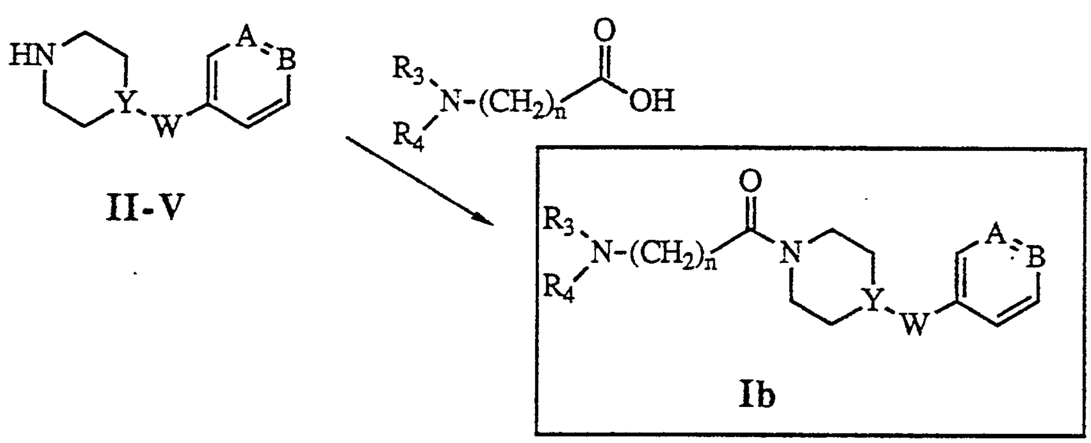

II-V

Ib

Alternatively, compounds of general formula **Ib** (**I**, **R** = $R_3R_4N(CH_2)_nCO$) can be produced by alkylation of amines $R_3R_4NH$ with an alkyl halide **VI** (**A**, **B**,

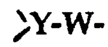

and **n** are as defined above and $G_3$ is a good leaving group, such as halide, p-toluenesulfonate, methanesulfonate etc. ), in the presence of a tertiary or aromatic amine, such as triethylamine or pyridine, in a suitable solvent, such as methylene chloride or chloroform. The reaction temperature ranges from 0° C to the boiling point of the solvent, and the reaction time goes from 6 to 24 hours.

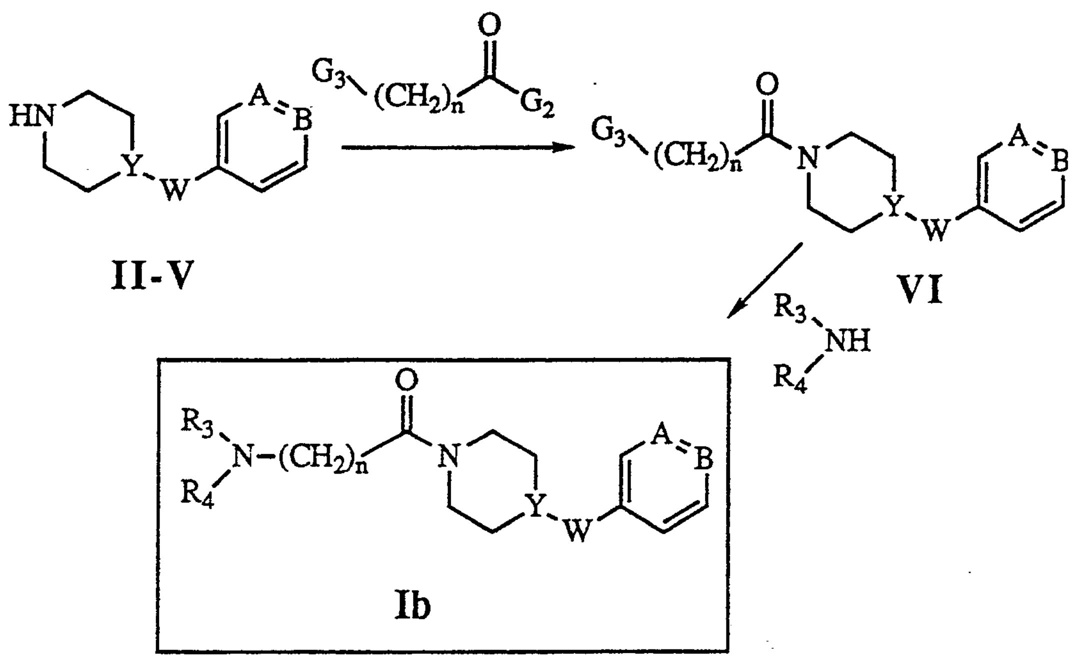

Alkyl halide **VI** may be obtained from the amines **II-V** (**A**,**B**, and

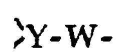

have the previous defined meaning) by treatment with $G_3(CH_2)_nC(=O)G_2$, wherein **n** and $G_3$ are as defined above, and $G_2$ is chloro or bromo, in the experimental conditions already mentioned for the formation of an amide bond from an acid halide.

Compounds of general formula **Ib** (**I**, **R** = $R_3R_4N(CH_2)_nCO$) wherein **n** is 0 are prepared by allowing to react amines **II-V** with a carbamic acid derivative of formula $R_3R_4N(C=O)G_1$, wherein $G_1$ is a good leaving group, such as phenoxy or chloro. The reaction is carried out in a suitable solvent, such as pyridine, at a temperature ranging from room temperature to the boiling point of the solvent, during a reaction time from 1 to 4 days.

Compounds of formula **Ic** (**I**, **R** = $R_5(CH_2)_m$) may be obtained from the amines **II-V** (**A**,**B**,

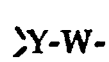

have the previous defined meaning) by treatment with alkyl halides of formula $R_5(CH_2)_mG_3$, where $G_3$, $R_5$ and **m** are as defined above. The reaction is conducted in the presence of a proton scavenger amine, such as pyridine or triethylamine, in a suitable solvent, such as methylene chloride or chloroform. The reaction temperature ranges from 0° C to the boiling point of the solvent, and the reaction time from 6 to 48 hours.

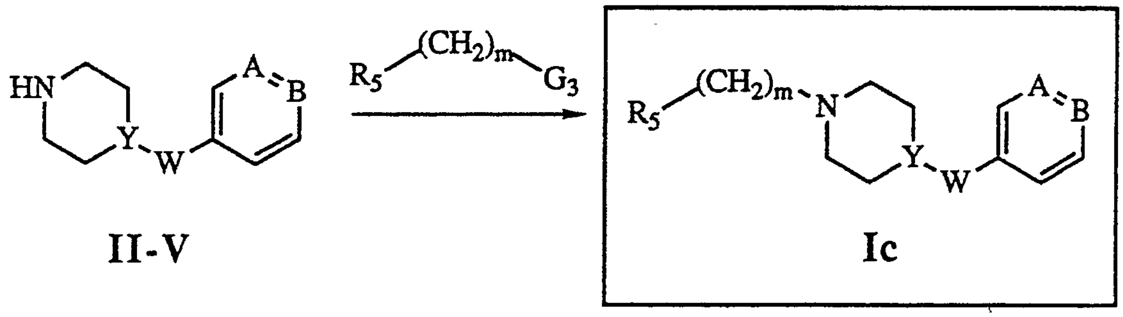

II-V

Ic

(1-Piperazinyl)-(3-pyridyl)acetonitrile (**II**, $R_1$ = H, A = N and B = CH) and (1-piperazinyl)-(4-pyridyl)-acetonitrile (**II**, $R_1$ = H, A = CH and B = N)) can be obtained by reaction of 3-pyridinecarboxaldehyde and 4-pyridinecarboxaldehyde respectively, with an excess of piperazine, in the presence of potassium cyanide, in a mixture of methanol and phosphate buffer close to pH = 7.1. The reaction is carried out at room temperature with a reaction time that goes from 6 to 24 hours. The desired compound can be isolated by celite filtration of the crude followed by chloroform extraction. The compound obtained in that way is in general pure enough but, in any case, can be purified by flash chromatography.

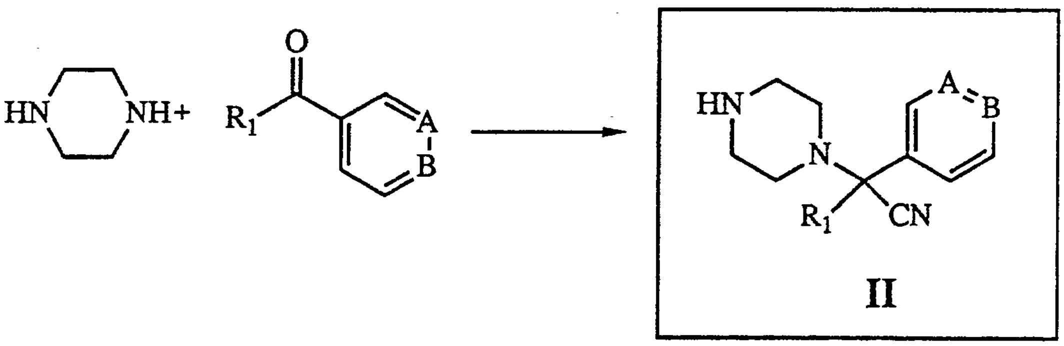

II

Amines **II** (**A** and **B** have the previously defined meaning), wherein $\mathbf{R_1}$ is a $C_1$-$C_6$ alkyl group, can be prepared also via the Strecker reaction, starting from the corresponding 3-(1-oxoalkyl)pyridine or 4-(1-oxoalkyl)pyridine. Due to the lower reactivity of ketones, in some cases, it is convenient to change the previous procedure in the following way:

3-(1-Oxoalkyl)pyridine or 4-(1-oxoalkyl)pyridine are allowed to react with an excess of piperazine in the presence of an acid catalyst, for example p-toluenesulfonic acid, in a suitable solvent, for example toluene or benzene, in a Dean-Stark at the temperature of the boiling point of the solvent. The reaction time will be enough to separate one equivalent of water. Then, once the solvent has been removed, the crude is treated with an equimolar amount, relative to the ketone, of potassium cyanide, in acetic acid as a solvent, at a temperature ranging from room temperature to the boiling point of the solvent, during a reaction time from 6 to 48 h. In other cases, allowing to react 3-(1-oxoalkyl)pyridine or 4-(1-oxoalkyl)pyridine with an excess of piperazine in the presence of one equivalent of potassium cyanide in acetic acid as a solvent at a temperature ranging from 20 °C to the boiling point of the solvent, during a reaction time from 6 to 48 h.

On the other hand, compounds of general formula **Ia**, **Ib** and **Ic** wherein **A**, **B**, $\mathbf{R_2}$, $\mathbf{R_3}$, $\mathbf{R_4}$, $\mathbf{R_5}$, **n** and **m** have the previous defined meaning and

$$>Y\text{-}W\text{-} \text{ is } >N\text{-}CR_1CN\text{-}$$

can be also prepared changing the order of the synthetic steps, starting from amines of formula **VIIa**, **VIIb** and **VIIc** respectively, wherein $\mathbf{R_2}$, $\mathbf{R_3}$, $\mathbf{R_4}$, $\mathbf{R_5}$, **n** and **m** have the previous defined meaning, and following the same procedures described in the preparation of amines **II**, but using in this case, an equimolar relation between the amine and carbonylic compound.

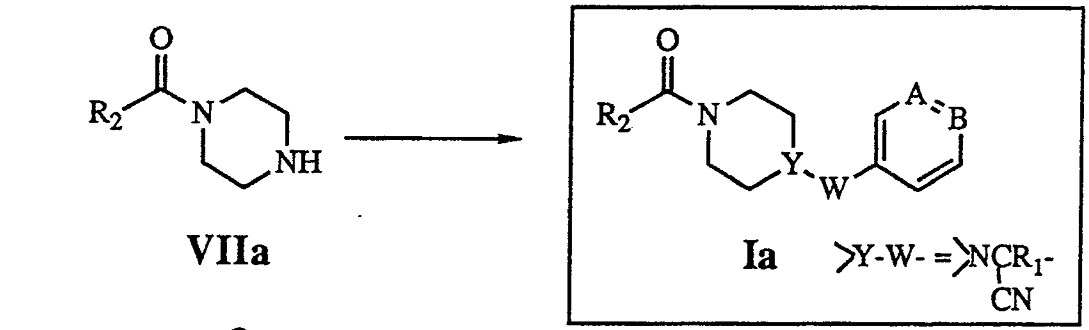

VIIa

Ia

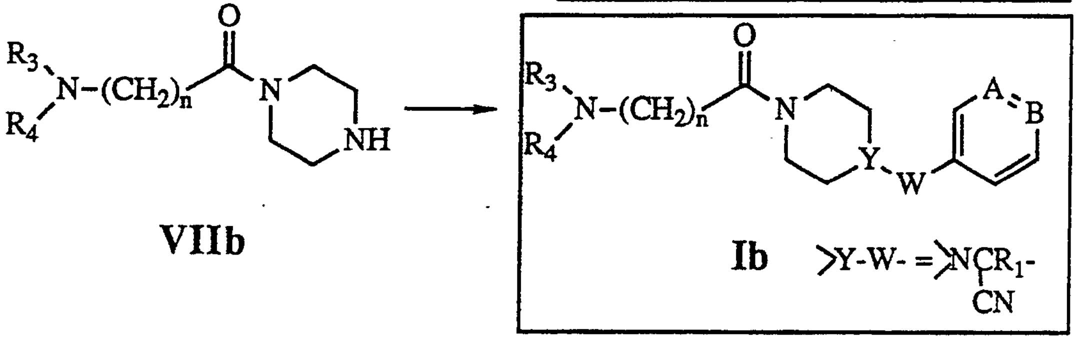

VIIb

Ib

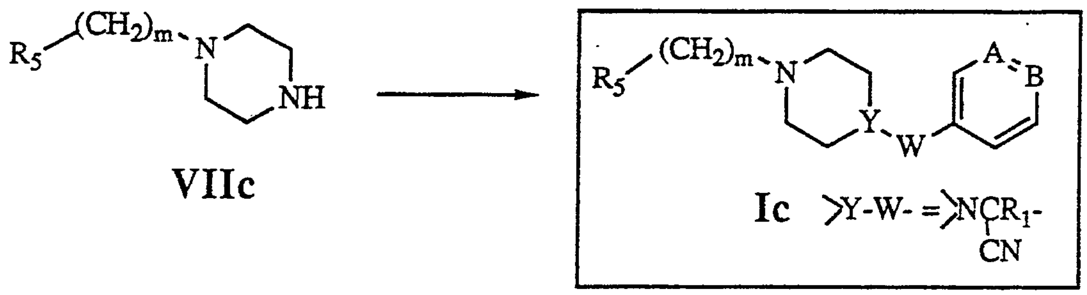

VIIc

Ic

Amines **III** (**A** and **B** have the previously defined meaning) are prepared by alkylation of 3-pyridineacetonitrile or 4-pyridineacetonitrile with a piperidine derivative **VIII** wherein **G**$_3$ has the previously defined meaning and **P**$_1$ is an amine protecting group. The anion in the $\alpha$ carbon to the nitrile is generated by treatment with a strong base such as sodium or potassium hydride, potassium tert-butoxide, lithium dialkylamide, sodium amide etc. in a suitable solvent such as *N,N*-dimethylformamide, dimethylsulfoxide, tetrahydrofuran, diethyl ether, toluene, etc. The temperature of the reaction ranges from 0 °C to the boiling point of the solvent, and the reaction time goes from 1 to 48 hours. As the nitrogen protecting groups it may be mentioned urethane-forming protective groups such as benzyloxycarbonyl, tert-butoxycarbonyl or ethoxycarbonyl groups, acyl-type protective groups such as formyl, acyl, trifluoroacetyl, benzoyl, chloroacetyl or tosyl, or alkyl-type protective groups such as trityl, benzyl or trimethylsilyl. Once the alkylation reaction is finished, the amine protecting group can be removed using different methods depending on the nature of the protecting group. For instance, benzyl and benzyloxycarbonyl groups can be deprotected by catalytic reduction, urethane type by saponification or treatment with trimethylsilyl iodide, and the tert-butoxycarbonyl group by treatment with an acid such as trifluoroacetic or hydrochloric acids.

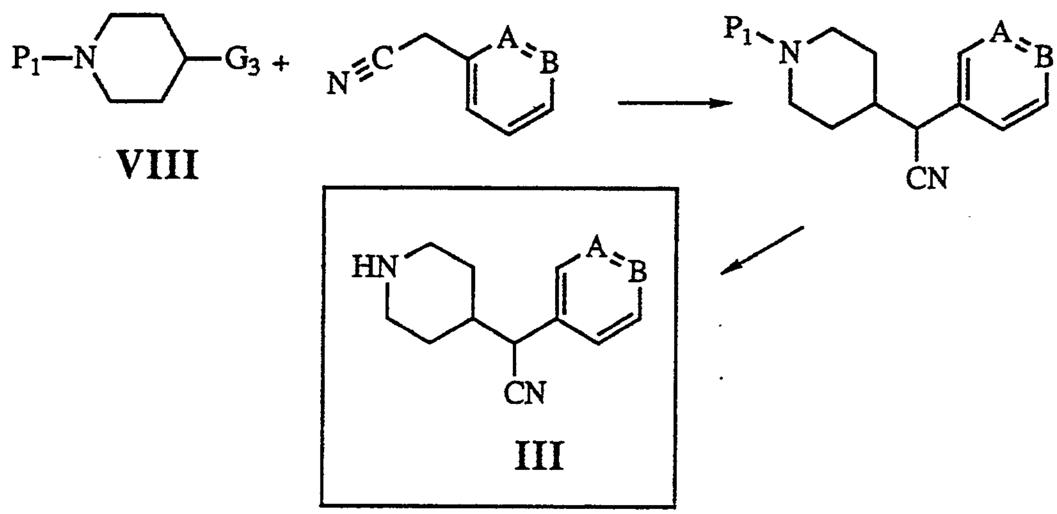

VIII

III

Amines **IV** (**A** and **B** have the previously defined meaning) are prepared by an aldol type reaction followed by dehydration from 3-pyridineacetonitrile or 4-pyridineacetonitrile and 4-piperidone conveniently protected in the nitrogen. The reaction conditions can be, for example, sodium ethoxide in ethanol as a solvent at a temperature that can range from 40 °C to the boiling point of the solvent during a reaction time from 1 to 48 h. As protecting groups and deprotection conditions it can be used the same options suggested for the preparation of amines **III**.

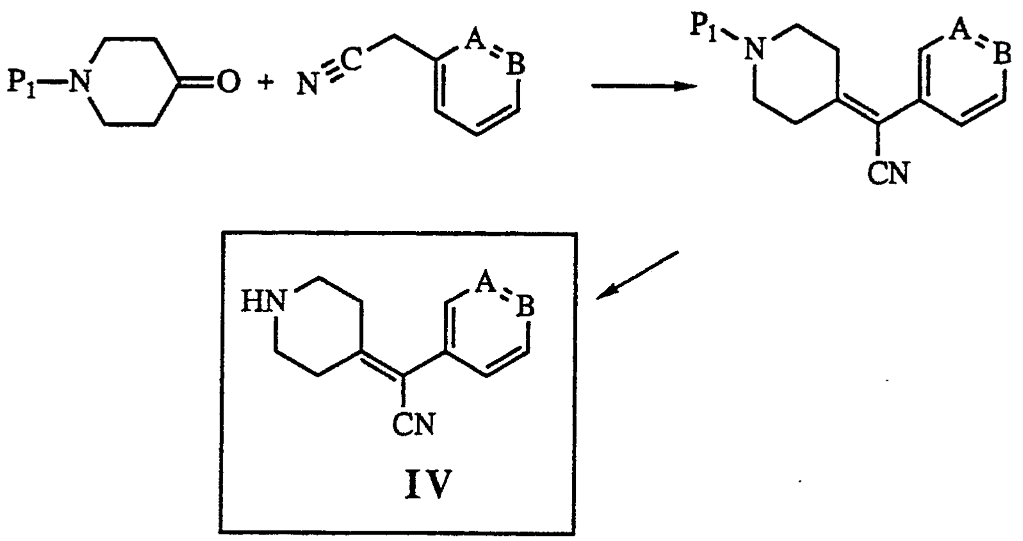

IV

Amines **V** (**A** and **B** have the previously defined meaning) are prepared by allowing the monoprotected piperazine to react with 3-(2-bromoacetyl)pyridine hydrobromide or 4-(2-bromoacetyl)pyridine hydrobromide in the presence of a tertiary or aromatic amine, for example triethylamine or pyridine, in a suitable solvent, for example methylene chloride or chloroform. The reaction is carried out at a temperature ranging from 0 °C to the boiling point of the solvent, during a reaction time from 6 to 24 hours. The ketone **IX** is reduced by sodium borohydride treatment in a suitable hydroxylic solvent such as methanol, ethanol, water or mixtures of them, at a temperature ranging from 0 °C to room temperature and during a reaction time from 30 min to three hours, to give intermediate **X**. Then, the hydroxy group present in **X** is converted in a previously defined good leaving group $G_3$ to give intermediate **XI**. This transformation depends on the nature of $G_3$, for instance, if $G_3$ is chloro, alcohol **IX** is treated with one equivalent of thionyl chloride in a suitable solvent, among which are chloroform, methylene chloride, toluene or tetrahydrofuran, at a temperature ranging from 0 °C to the boiling point of the solvent, during a reaction time from half to 6 hours, yielding **XI** as the hydrochloride. The free base can be obtained by treatment with a tertiary amine such as triethylamine, in a solvent such as chloroform, followed by an aqueous work up. Finally, intermediate **XI** is

treated with potassium cyanide in a suitable solvent, among which can be mentioned dimethylsulfoxide or *N,N*-dimethylformamide at a temperature ranging from 40° C to the boiling point of the solvent, during a reaction time from 1 to 48 hours. As protecting groups and removing conditions, the options suggested in the preparation of amines **III** can be used.

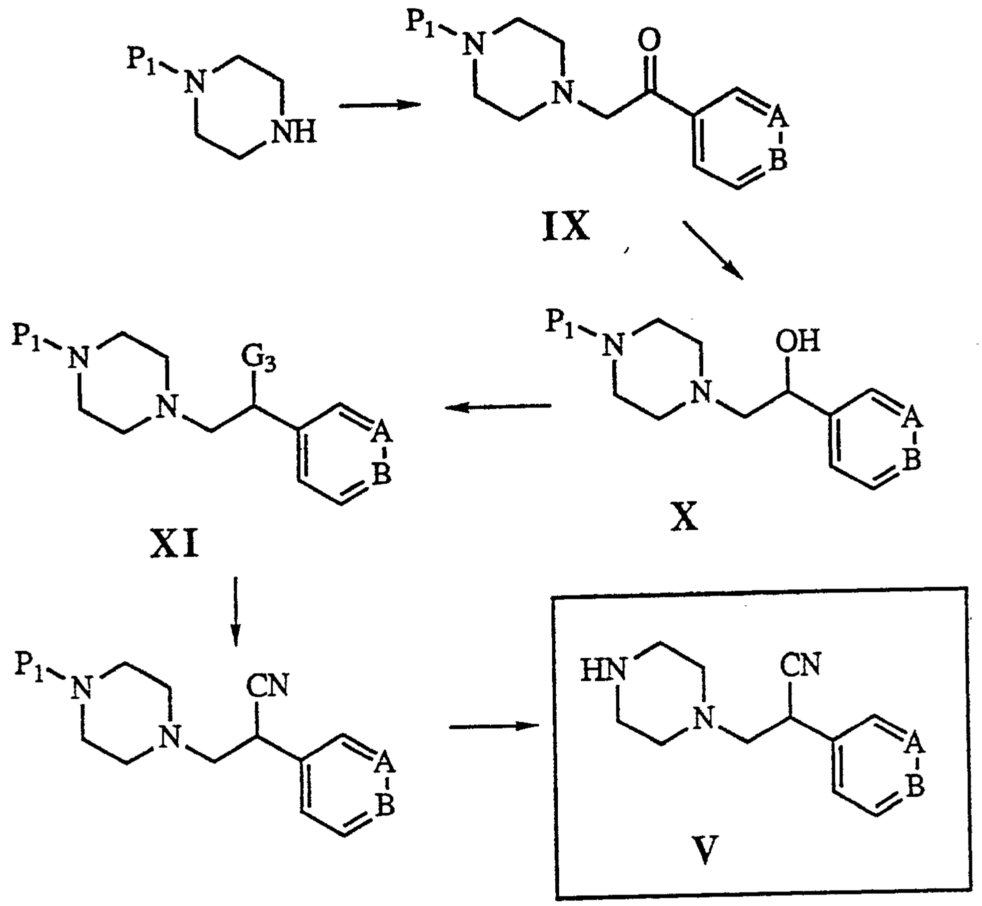

**IX**

**X**

**XI**

**V**

Compounds of general formula **Id** (**I**, **A**, **B** and **R** have the previous meaning and

$$\succ Y\text{-}W\text{- is } \succ N\text{-}CH_2\text{-}CH(CN)\text{-})$$

can be also prepared from amines **VIIa**, **VIIb** or **VIId**, following a sequence of reactions analogous to that described in the preparation of amines **V**

**VIIa**

**VIIb**

**VIIc**

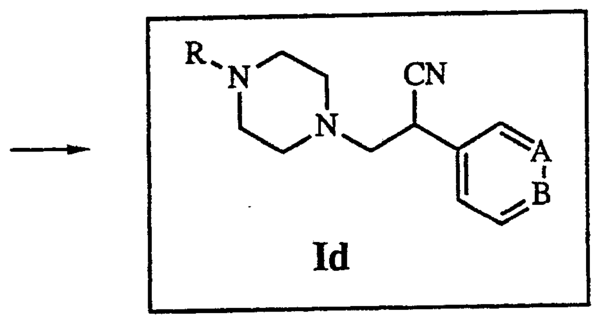

**Id**

Acids of general formula **$R_2$COOH**, **$R_3R_4N$-$(CH_2)_n$-COOH** and amines **VIIa**, **VIIb** and **VIIc** are commercially available, widely described in the literature or can be prepared by methods analogous to those described starting from commercially available products.

A production process of acids **$R_3R_4N$-$(CH_2)_n$-COOH** is resumed in the following scheme:

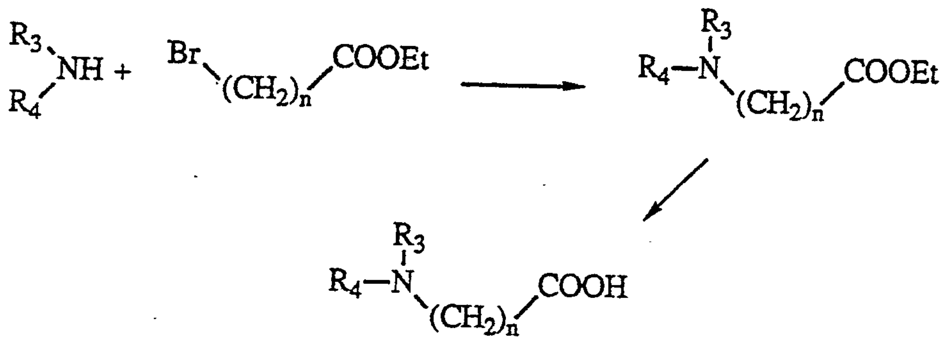

In the first step, similar conditions to those previously described for the alkylation of amines $R_3R_4NH$ with alkyl halides **VI** can be used. The second step, the hydrolisis of an ester to a carboxylic acid, can be performed in acid or basic media, in the usual conditions for those transformations, for example, in the presence of potassium carbonate, sodium or potassium hydroxide etc. in a hydroxylic solvent such as water, methanol, ethanol or mixtures of them.

When $R_2COOH$ has the structure indicated in **XII**, those acids are prepared according to the following scheme:

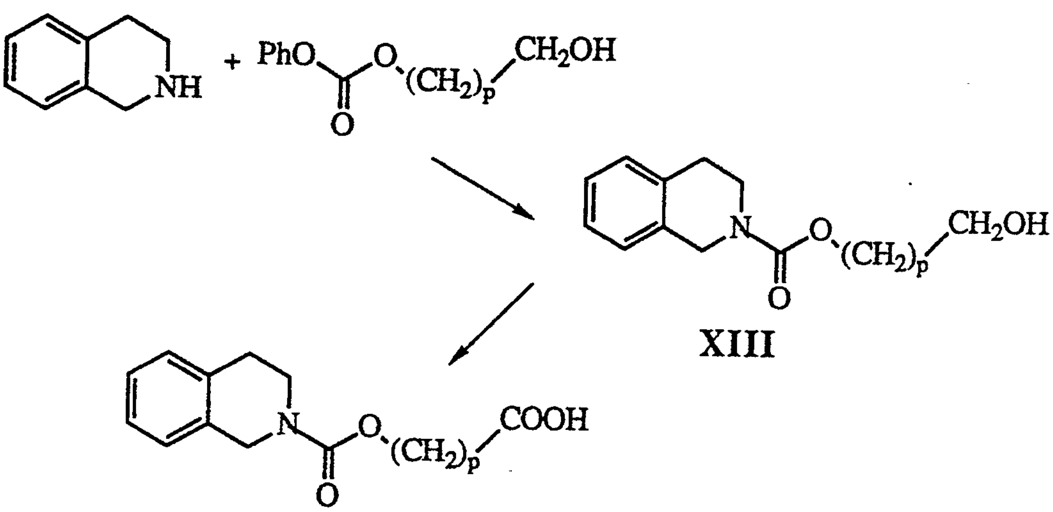

XIII

XII

Urethane **XIII** is produced in similar conditions to those described for the preparation of compounds of general formula **Ia** wherein $R_2$ is an arylalkoxy or diarylalkoxy group and the oxidation of the alcohol to carboxylic acid is carried out in the usual conditions for those transformations, for example by treatment with an acid solution of chromic acid in acetone (Jones' reagent).

Compounds of general formula **I**, being potent PAF antagonists, are useful as preventive and therapeutic drugs for the treatment of circulatory diseases caused by PAF, such as thrombosis, cerebral apoplexy ( e.g.. cerebral hemorrhage, cerebral thrombosis), myocardial infarction, angina pectoris, thrombotic phlebitis, thrombocitopenic purple, nephritis (e.g. glomerular nephritis), diabetic nephrosis, shock (e.g. endotoxin shock observed after severe infection or postoperatively, intravascular agglutination syndrome caused by endotoxin, anaphylactic shock, hemorrhagic shock); digestive tract diseases caused by PAF (e.g. gastric ulcer); diseases related to allergy and inflammation (e.g. asthma, dermatitis, urticaria, psoriasis); pneumonia; rejection due to increased PAF production after implantations of organs; and postoperative organodisfunction (e.g. in heart, liver and kidney). It can also be used for contraception of female mammals by suppressing cell division and/or ovoimplantation on the uterus, in the treatment of endometriosis and for the prevention or treatment of hyperendothelinemia induced by excess secretion of endothelin.

According to the activity of the compounds, the present invention relates also to compositions that contain one or more of the compounds of the present invention, together with an excipient or other auxiliary

agents if it were necessary. Compounds of the present invention can be administered on the form of any conventional formulation, the nature of which will, as it is well known, depend on the route of administration and the nature of the pathology to be treated.

Thus, solid compositions according to the present invention for oral administration include compressed tablets, dispersible powders, granules and capsules. In tablets, one or more of the active component(s) is admixed with at least one inert diluent such as lactose, starch, mannitol, microcrystalline cellulose or calcium phosphate; granulating and desintegrating agents for example corn starch, gelatine, microcrystalline cellulose or polyvinylpyrrolidone; and lubricating agents for example magnesium stearate, stearic acid or talc. The tablets may be coated by known techniques to delay disintegration and absorption in the gastrointestinal tract and, thereby, provide a sustained action over a longer period. Gastric film-coated or enteric film-coated can be made with sugar, gelatin, hydroxypropylcellulose, or acrylic resins. Tablets with a sustained action may also be obtained using an excipient which provides regressive osmosis, such as the galacturonic acid polymers. Formulations for oral use may also be presented as hard capsules of absorbable material, such as gelatin, wherein the active ingredient is mixed with an inert solid diluent and lubricating agents, or pasty materials, such as ethoxylated saturated glycerides that could exhibit controled liberation. Soft gelatin capsules are possible wherein the active ingredient is mixed with water or an oily medium, for example peanut oil, liquid paraffin or olive oil.

Dispersible powders and granules suitable for preparation of a suspension by the addition of water provide the active ingredient in admixture with a dispersing or wetting agent, a suspending agent, such as sodium carboxymethylcellulose, methylcellulose, hydroxypropylmethylcellulose, sodium alginate, polyvinylpirrolidone, gum tragacanth, xantham gum, gum acacia, and one or more preservatives, such as methyl or *n*-propyl-*p*-hydroxybenzoate. Additional excipients, for example sweetening, flavoring and coloring agents may also be present.

Liquid compositions for oral administration include emulsions, solutions, suspensions, syrups and elixirs containing commonly used inert diluents, such as distilled water, ethanol, sorbitol, glycerol, or propylene glycol. Such compositions may also comprise adjuvants such as wetting agents, suspending agents, sweetening, flavoring, perfuming, preserving agents and buffers.

Other compositions for oral administration include spray compositions, which may be prepared by known methods and which comprise one or more active compound(s). The spray compositions will contain a suitable propellent.

Preparations for injection according to the present invention for parenteral administration include sterile aqueous or non-aqueous solutions, suspensions or emulsions, in a non-toxic parentally-acceptable diluent or solvent. Examples of aqueous solvents or suspending media are distilled water for injection, the Ringer's solution, and isotonic sodium chloride solution. Examples of non-aqueous solvents or suspending media are propylene glycol, polyethylene glycol, vegetable oils such as olive oil, or alcohols such as ethanol. These compositions may also include adjuvants such as wetting, preserving, emulsifying and dispersing agents. They may be sterilized by one of the known methods or manufactured in the form of sterile solid compositions which can be dissolved in sterile water or some other sterile injectable medium immediately before use. When all of the components are sterile, the injectables will maintain the sterility if they are manufactured in sterile environment.

A compound of the invention may also be administered in the form of suppositories for rectal administration of the drug, or as creams, ointments jellies, solutions or suspensions for topical use and pessaries for vaginal administration.

Following are some representative preparations for tablets capsules, syrups, aerosols and injectables. They can be prepared following standard procedures and they are useful as inhibitors of the platelet activating factor.

**Tablets**

| | | |
|---|---|---|
| Compound of formula **I** | 100 | mg |
| Dibasic calcium phosphate | 125 | mg |
| Sodium starch glycolate | 10 | mg |
| Talc | 12,5 | mg |
| Magnesium stearate | 2,5 | mg |
| | 250,0 | mg |

**Hard gelatin capsules**

| | | |
|---|---|---|
| Compound of formula **I** | 100 | mg |
| Lactose | 197 | mg |
| Magnesium stearate | 3 | mg |
| | 300 | mg |

**Syrup**

| | | |
|---|---|---|
| Compound of formula **I** | 0,4 | g |
| Sucrose | 45 | g |
| Flavoring agent | 0,2 | g |
| Sweetening agent | 0,1 | g |
| Water to | 100 | mL |

**Aerosol**

| | | |
|---|---|---|
| Compound of formula **I** | 4 | g |
| Flavoring agent | 0,2 | g |
| Propylene glycol to | 100 | mL |
| Suitable propellent to | 1 | unit |

**Injectable preparation**

| | | |
|---|---|---|
| Compound of formula **I** | 100 | mg |
| Benzylic alcohol | 0,05 | mL |
| Propylene glycol | 1 | mL |
| Water to | 5 | mL |

The compounds of the present invention are useful for the manufacture of medicaments for the treatment of PAF-mediated illnesses, as illustrated in the following examples of pharmacological tests

**EXAMPLE OF PHARMACOLOGICAL TEST 1**

Inhibition of platelet aggregation induced by PAF.
The blood is obtained by cardiac puncture of male New Zealand albino rabbits (between 2 and 2.5 Kg of weight) and coagulation is prevented by adding 1 part of 3.16% sodium citrate dihydrate in 9 parts of blood. The platelet rich plasma (PRP) is prepared by blood centrifugation at 250xg for 10 min. at 4° C and it is diluted with platelet poor plasma (PPP) obtained by additional centrifugation at 3000xg for 10 min. The amount of platelets is adjusted to $3x10^5/mm^3$. The platelet aggregation induced by PAF ($C_{18}$, prepared in our laboratory) (16 nM, final) is determined by the Born nephelometric technique (*J. Physiol.*, **1962**, 162, 67) using a aggregometer Chrono-log 500. The activities of the inhibitors are expressed as $IC_{50}$, that is to say the concentration of the drug needed to inhibit the platelet aggregation in a 50%. The results are shown in table I:

TABLE I

| Compound Nº | $IC_{50}$ (μM) |
|---|---|
| 1 | 22 |
| 2 | 0.078 |
| 3 | 0.7 |
| 4 | 4.1 |
| 5 | 2.5 |
| 6 | 0.44 |
| 7 | 0.27 |
| 8 | 0.0021 |
| 9 | 0.009 |
| 10 | 0.2 |
| 11 | 2.7 |

| | |
|---|---|
| 12 | 0.02 |
| 13 | 0.011 |
| 14 | 0.019 |
| 15 | 2.1 |
| 16 | 0.0067 |
| 17 | 0.0093 |
| 19 | 0.79 |
| 22 | 2.2 |
| 25 | 0.018 |
| 26 | 1.6 |
| 27 | 0.5 |
| 28 | 0.015 |
| 29 | 0.002 |
| 31 | 0.023 |
| 33 | 0.41 |
| 34 | 2.8 |
| 38 | 0.024 |
| 41 | 0.74 |
| 44 | 0.0075 |
| 45 | 0.048 |
| 46 | 0.064 |
| 47 | 0.054 |
| 48 | 0.066 |
| 49 | 0.030 |
| 52 | 0.046 |
| 57 | 0.76 |
| 62 | 0.034 |
| 67 | 0.041 |

**EXAMPLE OF PHARMACOLOGICAL TEST 2**

Inhibition of the hypotensive effect induced by PAF in normotense rats.

Male Sprage Dawley rats, of 180-220 g of weight, anesthetized with sodium pentobarbital (50 mg/Kg, i.p. 1 mL/100 g) have been used. In order to measure the average arterial pressure, a polyethylene catheter is introduced into the carotid artery. The arterial pressure is recorded with the help of a transducer connected with a R611 Beckman polygraph. The tested compounds are administered through the femoral vein 3 min. before the injection of PAF (0.5 mcg/Kg, i.v.). The inhibition of the hypotension induced by PAF of the different compounds expressed as $ID_{50}$, that is to say, the amount of compound by weight of animal (dose) needed to inhibit the hypotension induced by PAF in a 50%, is shown in Table II.

TABLE II

| Compound Nº | $ID_{50}$ (mg/Kg i.v.) |
|---|---|
| 1 | 5 |
| 2 | 1.3 |
| 5 | 1.5 |
| 6 | 1.2 |
| 7 | 5 |
| 8 | 0.14 |
| 9 | 0.11 |
| 10 | 0.12 |
| 11 | 0.73 |
| 12 | 0.016 |
| 13 | 0.68 |
| 14 | 0.26 |
| 15 | 0.24 |
| 16 | 0.17 |
| 17 | 0.049 |
| 19 | 1.2 |
| 22 | 4.3 |
| 25 | 0.19 |
| 26 | 0.17 |
| 27 | 1.1 |
| 28 | 0.061 |
| 29 | 0.061 |

| | |
|---|---|
| 31 | 0.078 |
| 33 | 3.5 |
| 34 | 3.0 |
| 38 | 0.42 |
| 41 | 1.5 |
| 44 | 0.023 |
| 45 | 0.33 |
| 46 | 0.13 |
| 47 | 0.025 |
| 48 | 0.037 |
| 49 | 1.7 |
| 52 | 0.34 |
| 57 | 0.47 |
| 62 | 0.29 |
| 67 | 0.034 |

Finally the following examples illustrate but do not limit the preparation processes of the compounds of the present invention.

**PREPARATION 1**

**(1-Piperazinyl)-(3-pyridyl)acetonitrile**

To a solution of 13.95 g (0.162 mol) of piperazine and 5.4 g of potassium cyanide in 60 mL water and 60 mL of 1M pH = 7.1 of phosphate buffer solution, under argon atmosphere, a solution of 5.1 mL (0.054 mol) of pyridine-3-carboxaldehyde in 60 mL methanol was added dropwise. The solution was stirred 18 hours at room temperature, 80 mL of water were added and the solution was extracted with ethyl acetate. The organic phase was dried over sodium sulfate and after evaporation of the solvent, 17.4 g of crude were obtained. The crude was purified by chromatography on silica gel ($CHCl_3$ / MeOH / $NH_3$ : 60/2/0.2) yielding 8.5 g of a colorless oil (81 % yield).

IR (film)$\nu$: 3301, 2943, 2911, 2225, 1588, 1573, 1474, 1449, 1440, 1419, 1123 $cm^{-1}$;

$^1H$ NMR (80MHz, $CDCl_3$) $\delta$ (TMS):

8.77 (d, J=2.3 Hz, 1H, pyr),

8.59 (dd, $J_a$=4.8Hz, $J_b$=1.4Hz, 1H, pyr)

7.87 (broad d, J=6.3Hz, 1H, pyr),

7.36 (dd, $J_a$=6.3Hz, $J_b$=4.1Hz, 1H, pyr),

4.91(s, 1H, CHCN),

2.90 (m, 4H, pip),

2.55 (m, 4H, pip),

1.82(s, 1H, NH);

$^{13}C$ NMR (20.15 MHz, $CDCl_3$) $\delta$ (TMS): 149.41 (CH), 148.82 (CH), 134.83 (CH), 128,38 (C), 122.76 (CH), 113.83 (C), 59.81 (CH), 50.29 ($CH_2$), 45.01 ($CH_2$).

**PREPARATION 2**

**(1-Piperazinyl)-(4-pyridyl)acetonitrile**

Following the procedure described in preparation 1, but using pyridine-4-carboxaldehyde instead of pyridine-3-carboxaldehyde the title compound of this preparation was obtained as a colorless oil (82 %yield)

IR(film) : 3296,2943,2822,1594,1410, 1322$cm^{-1}$;
$^1H$ NMR (80MHz, $CDCl_3$) δ (TMS):
8.68 (d, J=6.2Hz, 2H, pyr),
7.50 (d, J=6.2Hz, 2H, pyr)
4.81 (s, 1H, CHCN)
2.96 (t, J=4.8Hz, 4H, pyr)
2.55 (t, J=4.8Hz, 4H, pyr),
1.78 (s, 1H, NH)

**PREPARATION 3**

**1-(2-chloroacetyl)-4-(3-pyridylcyanomethyl) piperazine**

To a solution of 2.50g (12.30mmol) of the product obtained in preparation 1, in 38 mL of dry chloroform, 1.25 mL(12.30 mmol) of triethylamine were added under argon atmosphere and the solution was cooled to 0°C. 1ml (12.30 mmol) of chloroacetyl chloride was added dropwise and the mixture was stirred 1 h. After dilution with chloroform the organic phase was washed twice with water, dried over sodium sulfate and evaporated, affording 3.32 g of a colorless oil ( 97%yield).
$^1H$ NMR ($CDCl_3$) δ ppm (TMS):
8.79 (d , J=2.3Hz, 1H, pyr),
8.67 (broad d, J=4.1 Hz, 1H, pyr),
7.87 (broad d, J=6.3 Hz, 1H, pyr),
7.39 (dd, $J_a$=6.3 Hz, $J_b$=4.1 Hz, 1H, pyr)
4.97 (s, 1H, CHCN),
4.08 (s, 2H, $CH_2Cl$),
3.61 (m, 4H, pip),
2.64 (m, 4H, pip)

**PREPARATION 4**

***N*-Diphenylmethyl phenylcarbamate**

To a mixture of 1.72 g (9,4 mmol) of diphenylmethylamine, 1.3 mL (9.4 mmol) of triethylamine and 40 mL dry dichloromethane, cooled to 0°C, under argon atmosphere, were added 1.2 mL (9,4mmol) of phenyl chloroformate. The mixture was stirred at room temperature for 1 hour, poured into water and the layers were separated. The organic phase was washed with water and dried over $Na_2SO_4$. The solvent was removed yielding 2.69 g of the product as a colorless oil (94% yield).
IR(film) ν :3331, 3054, 3019, 1702, 1518, 1485, 1233, 1217, 1196, 1023 $cm^{-1}$;
$^1H$ NMR (80MHz, $CDCl_3$) δ (TMS):
7.29 (m,15H),
6.04(d, J=8Hz, 1H, CH),
5.78 (m, 1H, NH)

**PREPARATION 5**

***N*-3,3-diphenylpropyl phenylcarbamate**

Following a procedure analogous to that described in preparation 4, but using 3,3-diphenylpropylamine instead of diphenylmethylamine, the title compound of this example was obtained as a colorless oil (99% yield).
IR(film) ν : 3318, 3050, 2925, 1699, 1525, 1487, 1260, 1209, 1161 $cm^{-1}$;
$^1H$ NMR (80MHz, $CDCl_3$) δ (TMS):
7.24 (m, 15H),
5.04 (m, 1H, NH),
3.98 (t, J=8Hz, 1H, $CHPh_2$),
3.20 (q, J=8Hz, 2H, $CH_2N$),
230 (q, J=8Hz 1H, $CH_2$).

**PREPARATION 6**

**Ethyl *N*-diphenylmethyl-*N*-methylaminoacetate**

To a solution of 0.5 g (2.5 mmol) of ethyl bromoacetate and 0.35 mL (2.5 mmol) of triethylamine, in 10 mL of dry chloroform, under argon atmosphere, 0.95 g (5 mmol) of *N*-methyldiphenylmethylamine were added. The solution was stirred 24 hours at room temperature, diluted with chloroform and washed with water. It was dried over magnesium sulfate and the solvent was removed, affording a crude that was purified by chromatography on silica gel eluting with hexane-ethyl acetate mixtures of increasing polarity. In that way, 0.39 g of the product were obtained as a colorless oil ( 55%yield)
$^1H$ NMR (80MHz, $CDCl_3$) δ (TMS):
7.29 (m, 10H),
4.82 (s, 1H, $CHPh_2$),
4.11 (q, J= 8Hz, 2H, $CH_2$),
3.26 (s, 2H, $CH_2N$),
2.35 (s, 3H, $NCH_3$),
1.20 (t, J=8Hz, 3H, $CH_3$).

**PREPARATION 7**

***N*-diphenylmethyl-*N*-methylaminoacetic acid**

To a solution of 1.59 g (5.6 mmol) of the product obtained in preparation 6 in 100 mL methanol, was added a solution of 3.86 g (28 mmol) of potassium carbonate in 50 mL of water and the mixture was stirred 1h at room temperature and 2 h. at 60° C. Methanol was removed under vacuum and the remaining solution was neutralized with hydrochloric acid to pH=6. The solution was extracted with $CHCl_3$, dried over $Na_2SO_4$ and the solvent was removed, yielding 1.37 g of the product as a white solid. ( 96%yield).
IR(KBr) ν : 3600-2200,1713, 1613, 1487, 1448, 1377, 1198, 747, 705 $cm^{-1}$;
$^1H$ NMR (80MHz, $CDCl_3$) δ (TMS):
9.54 (s, 1H, $CO_2H$),
7.35 (m, 10H, Ph),
5.21 (s, 1H, $CHPh_2$),
3.46 (s, 2H, $CH_2N$),
2.56 (s, 3H, $CH_3N$).

**PREPARATION 8**

**1-(2-Aminoethyl)-4-(3-pyridylcyanomethyl)piperazine**

Following the procedure described in preparation 1, but using 1-(2-aminoethyl)piperazine instead of piperazine, the title compound of this preparation was obtained as yellow liquid (24%yield)
IR(film) ν : 3361, 2941, 2880, 1527, 1573, 1451, 1420 $cm^{-1}$;
$^1H$ NMR (80MHz, $CDCl_3$) δ (TMS):
8.76 (d, J=2.4Hz, 1H, pyr),
8.62 (dd, $J_a$=1.6Hz, $J_b$=4.8Hz, 1H, pyr),
7.87 (d de t, $J_a$=1.6Hz, $J_b$=8.0Hz, 1H, pyr)
7.38 (dd, $J_a$=5.0Hz, $J_b$=7.6Hz, 1H, pyr)
4.96 (s, 1H, CHCN)
2.57 (m, 12H),
1.75 (m, 2H, $NH_2$).

**PREPARATION 9**

***N*-(3,3-Diphenylpropyl)-2-chloroacetamide**

Following the procedure described in preparation 3, but using 3,3-diphenylpropylamine instead of the compound obtained in preparation 1 the title compound of this example was obtained as a colorless oil (98%yield).

IR(film) ν : 3277, 3072, 2918, 2873, 1645, 1445, 1239 $cm^{-1}$;
$^1H$ NMR (80MHz, $CDCl_3$) δ (TMS):
7.25 (m, 10H, Ph)
3.95 (s, 2H, $COCH_2Cl$),
3.95 (t, J=7.6Hz, 1H, $Ph_2CH$)
3.32 (q, J=7.5Hz, 2H, $CH_2NHCO$),
2,36 (q, J=7.5, Hz, 2H, $Ph_2CHCH_2$).

**PREPARATION 10**

**1-Diphenylacetyl-4-formylpiperazine**

To a mixture of 9 mL (90 mmol) of formylpiperazine, 18.6g (90 mmol) of diphenylacetic acid and 13.5g (0.1 mol) 1-hydroxybenzotriazole in 200 mL anhydrous dimethylformamide, at 0 °C and under argon atmosphere, were added 21 g (0.1 mol) of dicyclohexylcarbodiimide. The mixture was stirred at room temperature overnight and diluted with 700 mL of ethyl acetate. The insoluble solid was filtered off and the filtrate washed with saturated $NaHCO_3$ solution and water. It was dried over $MgSO_4$ and the solvent was removed yielding 25.05 g of a crude that was directly used in the next reaction (90%yield).
IR(film) ν : 3019, 2923, 2857, 1672, 1658, 1624, 1432, 1225, 1211, 1007 $cm^{-1}$;
$^1H$ NMR (80MHz, $CDCl_3$) δ (TMS):
8.00 (s, 1H, CHO),
7.29 (m, 10H),
5.19 (s, 1H, $CHPh_2$),
3.23 (m, 8H, pip).

**PREPARATION 11**

**1-Piperazinyl α,α diphenylacetate**

To a solution of 25.05 g (81 mmol) of the product obtained in preparation 1, in 170 mL methanol, 300 mL 10% hydrochloric acid were added and the mixture heated 1h. at 100 °C. The solvent was removed and the residue was chromatographed on silica gel, eluting with $CHCl_3$/Methanol/$NH_3$ (60:2:0.2) to give 16.25 g of a white solid (71%yield). IR(film) ν : 3600-3200, 3021, 2998, 2913, 1632, 1428, 1223, 1031$cm^{-1}$;
$^1H$ NMR (80MHz, $CDCl_3$) δ (TMS):
7.24 (m, 10H, Ph),
5.18 (s, 1H, $CHPh_2$),
3.50 (m, 4H, pip),
2.63 (m, 5H, pip).

**PREPARATION 12**

**(1-Ethoxycarbonyl-4-piperidinylidene)-3-pyridylacetonitrile**

To a solution of 0.49 g sodium ethoxide in 30 mL ethanol, were added 0.75 mL (7 mmol) of 3-pyridylacetonitrile and 1.21 g (7 mmol) of 1-ethoxycarbonyl-4-piperidone and the resulting mixture was heated 1h. at reflux. It was allowed to cool and worked up with chloroform and water. The organic phase was separated, dried over sodium sulfate and the solvent was removed, yielding a crude that was purified on silica gel (chloroform: methanol 5%) to afford 1.03g of a colorless oil(55%yield).
$^1H$ NMR (80MHz, $CDCl_3$) δ (TMS):
8.55 (m, 2H, pyr),
7.68 (broad d, J = 7.0Hz, 1H, pyr),
7.45 (m, 1H, pyr),
4.16 (q, J = 7.1Hz, 2H),
3.69 (t, J = 5.7Hz, 2H, pip),
3.51 (t, J = 5.6Hz, 2H, pip),
2.81 (t, J = 6.0Hz, 2H, pip), .
2.44 (t, J = 6.0Hz, 2H, pip),
1.27 (t, J = 7.0Hz, 3H).

**PREPARATION 13**

**(4-piperidinylidene)-3-pyridylacetonitrile**

To a solution of 1.36g (5mmol) of the compound obtained in preparation 12 and 2.28 g sodium iodide in 10 mL acetonitrile, under $N_2$ atmosphere, were added 0.97 mL of trimethylsilyl chloride. The resulting mixture was heated at 50° C for 2 days. It was allowed to cool, diluted with chloroform and washed with 0.1N sodium hydroxide solution and 10% sodium thiosulfate solution. The organic phase was dried over sodium sulfate and the solvents were removed yielding 0.95 g crude that was purified by chromatography on silica gel (chloroform: methanol: ammonia, 60: 4: 0.2) to give 0.28 g of a colorless oil (28%yield).
IR(film) $\nu$ : 3309, 2949, 2208, 1612, 1582, 1410 $cm^{-1}$;
$^1H$ NMR (80MHz, $CDCl_3$) $\delta$ (TMS):
8.57 (m, 2H, pyr),
7.68 (d de t, $J_a$ = 8.0 Hz, $J_b$ = 1.8Hz, 1H, pyr),
7.42 (dd, $J_a$ = 7.3Hz, $J_b$ = 2.5Hz, 1H, pyr),
2.95 (m, 6H, pip),
2.38 (t, J = 5.8Hz, 2H, pip),
2.17 (broad s, 1H, NH).

**PREPARATION 14**

**1-Ethoxycarbonyl-4-((1-(3-pyridyl)-1-cyano)methyl)piperidine**

To a suspension of 168 mg (7 mmol) of sodium hydride in 10 mL dimethylformamide, cooled with an ice bath and under argon atmosphere, were added 0.54 mL (5 mmol) of 3-pyridylacetonitrile in 3mL dimethylformamide. The suspension was stirred 10 min. at room temperature and 1.6 g (5 mmol) of 1-ethoxycarbonyl-4-(p-toluenesulfonyloxy)piperidine dissolved in 5 mL dimethylformamide were added. The mixture was heated at 50° C for 18 h. The solvents were removed under vacuum and the residue was worked up with chloroform and 0.05 N sodium hydroxide solution. The organic phase was separated, dried and the solvents were removed affording 2.81g crude that was purified by chromatography on silica gel (hexanes: ethyl acetate: methanol, 10: 10: 0.5) to yield 0.47 g of a colorless oil (35 % yield).
IR(film) $\nu$ : 2977, 2238, 1686, 1470, 1424, 1377, 1277 $cm^{-1}$;
$^1H$ NMR (80MHz, $CDCl_3$) $\delta$ (TMS):
8.60 (m, 2H, pyr),
7.72 (broad d, J = 7.4Hz, 1H, pyr),
7.36 (m, 1H, pyr),
4.12 (m, 2H, pip),
4.01 (q, J = 7.0Hz, 2H, $OCH_2CH_3$)
3.84 (d, J = 6.6Hz, 1H, CHCN),
2.70 (m, 2H),
2.1-1.2 (complex signal 4H),
1.24 (t, J= 7.0Hz, 3H, $OCH_2CH_3$)
$^{13}C$ NMR (20.15 MHz, $CDCl_3$) $\delta$ (TMS):154.75 C, 149.21 CH, 148.73 CH, 134.94 CH, 116.13 C, 60.85 $CH_2$, 42.89 $CH_2$, 40.46 CH, 29.33 $CH_2$, 28.33 $CH_2$, 14.17 $CH_3$,

**PREPARATION 15**

**4-(1-(3-Pyridyl) cyanomethyl)piperidine**

Following the procedure described in preparation 13, but using the compound obtained in preparation 14, a colorless oil was obtained (95%yield)
IR(film) $\nu$ : 3309, 2932, 2237, 1572, 1423 $cm^{-1}$;
$^1H$ NMR (80MHz, $CDCl_3$) $\delta$ (TMS):
8.45 (m, 2H, pyr),
7.60 (d de t, Ja = Hz, Jb =Hz, 1H, pyr),
7.28 (dd, Ja = Hz, Jb = Hz, 1H, pyr),
3.62 (d, J = Hz, 1H, CHCN),
3.05 (m, 2H, pip),

2.55 (m, 2H, pip),
2.28 (m, 1H, NH),
1.9-1.1 (complex signal, 4H, pip).

**PREPARATION 16**

**4-(2-(1,2,3,4-Tetrahydroisoquinolyl))carbonyloxybutyric acid**

**a) Phenyl 4-hydroxybutyl carbonate.**

To a solution of 23.7 g (0.26 mol) of 1,4-butanediol in 200 mL $CH_2Cl_2$, under argon atmosphere, at 0 °C, were added 11 mL of pyridine and 8.6 mL (0.066 mol) of phenyl chloroformate. The solution was stirred at room temperature 1 h. It was washed with water and 1N hydrochloric acid and the organic phase was dried over magnesium sulfate. The solvent was removed yielding 14.13 g of a crude that was used directly in the next step (quantitative yield ).
$^1H$ NMR (80MHz, $CDCl_3$) $\delta$ (TMS):
7.20 (m, 5 H, Ph),
4.25 (t, J = 5.6 Hz, 2 H, $CH_2OCO$),
3.63 (t, J = 5.6 Hz, 2 H, $CH_2OH$),
2.09 (s, 2 H, OH),
1.70 (m, 4 H, 2 $CH_2$).

**b) 2-(1,2,3,4-Tetrahydroisoquinolyl) 4-hydroxybutylcarbamate.**

To a solution of 7.1 g (0.034 mol) of the product obtained in preparation 16 a, in 50 mL of pyridine, under argon atmosphere and at 0 °C, 4.32 mL (0.034 mol) of 1,2,3,4-tetrahydroisoquinoline were added. The mixture was stirred 1 day at reflux. The resulting solution was poured into 500 mL water and extracted with $CH_2Cl_2$. The combined organic phases were washed with 1N hydrochloric acid, 1 N NaOH and $H_2O$ and were dried over sodium sulfate. The solvent was removed yielding 7.55 g of a crude that was directly used in the next step (88 % yield).
$^1H$ NMR (80MHz, $CDCl_3$) $\delta$ (TMS):
7.17 (s, 4H, Ph),
4.60 (s, 2 H, $CH_2N$),
4.16 (t, J = 6.4 Hz, 2 H, $CH_2O$),
3.67 (m, 4 H),
3.03 (broad s,1 H, OH),
2.82 (t, J = 6.4 Hz, 2 H, $CH_2N$),
1.70 (m, 4 H).

**c) 4-(2-(1,2,3,4-Tetrahydroisoquinolyl))carbonyloxybutyric acid**

To a solution of 7.55 g (0.03 mol) of the product obtained in preparation 16 b, in 1060 mL acetone, under argon atmosphere at 0 °C, 18.1 mL (0.045 mol) of Jones' reagent were added. The mixture was stirred 3 h. at room temperature. The resulting solution was filtered and the solvent removed. The residue was dissolved in $CH_2Cl_2$ and washed with 10% NaOH. The aqueous phase was washed with $CH_2Cl_2$ and acidified with 2N hydrochloric acid. It was extracted with $CH_2Cl_2$ and dried over sodium sulfate. The solvent was removed yielding 4.35 g of a brown oil (overall yield from phenyl chloroformate: 47 %).
IR(KBr)$\nu$ : 3600-2400, 1695, 1580, 1433, 1379, 1364, 1338, 1294, 1234, 1123 $cm^{-1}$.
$^1H$ NMR (80MHz, $CDCl_3$) $\delta$ (TMS):
10.22 (broad s., 1 H, COOH),
7.13 (s, 4 H,Ph),
4.59 (s, 2 H,$CH_2N$),
4.19 (t, J = 5.6 Hz, 2 H, $CH_2O$),
3.67(t, J = 5.6 Hz, 2 H, $CH_2OH$),
2.83 (t, J = 5.6 Hz, 2 H, $CH_2N$),
2.64 (t, J = 5.6 Hz, 2 H, $CH_2Ph$),
2.00 (m, 2 H).

**PREPARATION 17**

**3-(2-(1,2,3,4-Tetrahydroisoquinolyl))carbonyloxypropanoic acid.**

**a)Phenyl 3-hydroxypropyl carbonate.**

Following a procedure analogous to the described in preparation 16 a but using 1,3-propanediol instead of 1,4-butanediol, the title compound of this example was obtained as a colorless oil.
IR(KBr)ν : 3600-3200, 2959, 2885, 1754, 1588, 1488, 1453, 1259, 1208, 1060 $cm^{-1}$.
$^1H$ NMR (80MHz, $CDCl_3$) δ (TMS):
7.26 (m, 5 H, Ph),
4.38 (t, J = 6.4 Hz, 2 H, $CH_2OCO$),
3.74 (t, J = 6.4 Hz, 2 H, $CH_2OH$),
2.37 (s, 1 H, OH),
1.95 (m, 2 H).

**b) 2-(1,2,3,4-Tetrahydroisoquinolyl) 3-hydroxypropylcarbamate.**

Following a procedure analogous to the described in preparation 16 b, the title compound of this example was obtained as a colorless oil (overall yield from phenyl chloroformate: 66 % ).
IR(KBr) ν : 3600-3200, 2925, 1672, 1427, 1364, 1294, 1232, 1122 $cm^{-1}$.
$^1H$ NMR (80MHz, $CDCl_3$) δ (TMS):
7.14 (s, 4 H, Ph),
4.61 (s, 2 H, $CH_2N$),
4.30 (t, J = 6.4 Hz, 2 H, $CH_2OCO$),
3.69 (t, J = 6.4 Hz, 4 H, $CH_2OH$),
2.83 (t, J = 6 Hz, 2 H, $CH_2Ph$),
1.88 (m, 2 H).

**c) 3-(2-(1,2,3,4-Tetrahydroisoquinolyl))carbonyloxypropanoic acid.**

Following a procedure described in preparation 16 c, the title compound of this example was obtained as a colorless oil (61 % yield ).
IR(KBr)ν : 3600-2400, 1695, 1580, 1433, 1361, 1295, 1234, 1192, 1123, 1100 $cm^{-1}$.
$^1H$ NMR (80MHz, $CDCl_3$) δ (TMS):
10.24 (broad s., 1 H COOH),
7.13 (s, 4 H, Ph),
4.59 (s, 2 H, $CH_2N$),
4.41 (t, J = 6 Hz, 2 H, $CH_2OCO$),
3.66 (t, J = 6 Kz, 2 H, $CH_2N$),
2.77 (m, 4 H).

**EXAMPLE 1**

**1-(2-Naphthoyl)-4-(3-pyridylcyanomethyl)piperazine, dihydrochloride**

**a) 1-(2-Naphthoyl)-4-(3-pyridylcyanomethyl)piperazine.**

To a mixture of 0.7 g (3.7 mmol) of the compound obtained in preparation 1, 0.7 g (4.1 mmol) of 2-naphthoic acid and 0.75 g (5.5 mmol) of 1-hydroxybenzotriazole in 20 mL of dimethylformamide, cooled to 0 °C and under $N_2$, were added 0.78 g (3.78 mmol) of dicyclohexylcarbodiimide and the mixture was stirred 18 hours at room temperature. Then, the solvents were removed in vacuo and the residue was stirred with ethyl acetate. The white solid was removed and the organic solution was washed with saturated sodium bicarbonate solution, water and brine. Drying with sodium sulfate and removal of the solvent afforded 1.4 g that were purified by chromatography yielding 0.79 g of a colorless oil (61% yield).
IR (film) ν : 3050, 2979, 2910, 1625, 1588, 1573, 1463, 1422, 1368 $cm^{-1}$;
$^1H$ NMR (80 MHz, $CDCl_3$) δ (TMS):
8.77 (broad s, 1H, pyr),

8.63 (d, J=3.9 Hz, 1H, pyr),
7.85 (m, 4H),
7.50 (m, 5H),
4.92 (s, 1H, CHCN),
3.98 (m, 2H, pip),
3.24 (m, 2H, pip),
2.76 (m, 2H, pip),
2.46 (m, 2H, pip).

**b) Title compound of the example.**

To a solution, cooled to 0 ° C, of 0.79 g of the compound obtained in example 1a in 5 mL of ethyl acetate, 1.5 mL saturated solution of HCl gas in diethyl ether were added, yielding 0.62 g of a white solid (65% yield).
mp: 118.3-128.5 ° C;
IR (KBr) $\nu$ : 3419, 3051, 1624, 1544, 1463, 1437, 1281 $cm^{-1}$; $^1H$ NMR (80MHz, $CDCl_3$) $\delta$ (TMS):
11.90 (broad s, 2H),
9.28 (s, 1H, pyr),
8.93 (d, J=4.7Hz, 1H, pyr),
8.66 (d, J=7.9 Hz, 1H, pyr),
7.88 (m, 4H),
7.42 (m, 4H),
5.92 (m, 1H, CHCN),
4.00 (m, 2H, pip),
3.25 (m, 2H, pip),
2.92 (m, 2H, pip),
2.65 (m, 2H, pip).
Analysis Calcd. for $C_{22}H_{20}N_4O.2HCl$: C 61.55 %; H 5.16%; N 13.05%.
Found: C 61.41%; H 5.07%; N 12.82%.

**EXAMPLE 2**

**1-(1-Naphthylacetyl)-4-(3-pyridylcyanomethyl)piperazine.**

Following the procedure described in example 1a but using 1-naphthylacetic acid instead of 2-naphthoic acid, the title compound of this example was obtained as a white solid (75% yield).
mp: 152.2-152.8 ° C;
IR (KBr) $\nu$ : 2912, 1639, 1572, 1430, 1418, 1229 $cm^{-1}$;
$^1H$ NMR (80MHz, $CDCl_3$) $\delta$ (TMS):
8.72 (d, J=2.3Hz, 1H, pyr),
8.60 (dd, $J_a$=4.8Hz, $J_b$=1.5Hz, 1H, pyr),
7.70 (m, 4H),
7.35 (m, 5H),
4.84 (s, 1H, CHCN),
4.12 (s, 2H, $CH_2CO$),
3.71 (m, 2H, pip),
3.43 (m, 2H, pip),
2.46 (m, 4H, pip).
Analysis Calcd. for $C_{23}H_{22}N_4O$: C 74.57%; H 5.99%; N 15.12%. Found: C 74.17%; H 5.93%; N 15.07%.

**EXAMPLE 3**

**1-(2-Naphthylacetyl)-4-(3-pyridylcyanomethyl)piperazine.**

To a solution of 1.0 g (4.9 mmol) of the compound obtained in preparation 1 and 1.2 mL of triethylamine in 20ml anhydrous chloroform, cooled to 0 ° C under argon atmosphere, was added dropwise a solution of 5.6 mmol of 2-naphthylacetyl chloride in 5 mL anhydrous chloroform. The mixture was stirred 18 hours at room temperature. The organic solution was washed with water, dried and the solvents were

removed, affording 2.1g of crude that were purified by chromatography on silica gel (AcOEt) to yield 0.8 g of a white solid (44% yield).
mp: 181.9-186.2° C; IR (KBr) ν : 3052, 2881, 2812, 1638, 1597, 1572, 1440, 1429, 1416, 1227 $cm^{-1}$;
$^1H$ NMR (80MHz, $CDCl_3$) δ (TMS):
8.68 (m, 2H, pyr),
7.72 (m, 5H),
7.43 (m, 4H),
4.85 (s, 1H, CHCN),
3.90 (s, 2H, $CH_2CO$),
3.73 (m, 2H, pip),
3.51 (m, 2H, pip),
2.58 (m, 2H, pip),
2.40 (m, 2H, pip).
Analysis Calcd. for $C_{23}H_{22}N_4O$. 1/4 $H_2O$: C 73.61%; H 5.87%; N 14.93%.
Found: C 73.46%; H 5.81%; N 14.62%.

**EXAMPLE 4**

**1-Benzoyl-4-(3-pyridylcyanomethyl)piperazine, dihydrochloride.**

**a) 1-Benzoyl-4-(3-pyridylcyanomethyl)piperazine.**

Following the procedure described in example 3 but using benzoyl chloride instead of 2-naphthylacetyl chloride, the title compound of this example was obtained as a colorless oil (27% yield).
IR (film) ν : 2913, 2850, 1624, 1572, 1422, 1279, 1258 $cm^{-1}$;
$^1H$ NMR (80MHz, $CDCl_3$) δ (TMS):
8.78 (d, J=2.2Hz, 1H, pyr),
8.63 (dd, $J_a$=4.7 Hz, $J_b$=1.5Hz, 1H, pyr),
7.58 (d de t, $J_a$=7.8Hz, $J_b$=1.6Hz, 1H, pyr),
7.39 (m, 6H),
4.99 (s, 1H, CHCN),
3.63 (m, 4H, pip),
2.59 (m, 4H, pip).

**b) Title compound of the example.**

Following the procedure described in example 1b, but starting with the compound obtained in example 4a, the title compound of this example was obtained as a white solid (82% yield)
mp: 35.1-4.55° C; IR (KBr) ν : 3412, 3051, 2851, 1619, 1566, 1491, 1461, 1279 $cm^{-1}$;
$^1H$ NMR (80MHz, DMSO-$d_6$) δ (TMS):
8.87 (m, 2H, pyr),
8.45 (broad d, J=8.0Hz, 1H, pyr),
7.99 (dd, $J_a$=8.0Hz, $J_b$=5.5Hz, 1H, pyr),
7.43 (broad s, 5H, Ph),
5.80 (s, 1H, CHCN),
4.12 (broad s, HCl, H2O),
3.54 (m, 4H, pip),
2.58 (m, 4H, pip).
Analysis Calcd. for $C_{18}H_{18}N_4O.2HCl$: C 57.00%; H 5.32%; N 14.77%.
Found: C 57.01%; H 5.61%; N 14.46%.

**EXAMPLE 5**

**1-(Phenylacetyl)-4-(3-pyridylcyanomethyl)piperazine**

Following the procedure described in example 3 but using phenylacetyl chloride instead of 2-naphthylacetyl chloride the title compound of this example was obtained as a white solid (35% yield).
mp: 150.3-151.5° C;

IR (KBr) ν : 2914, 2826, 1633, 1585, 1573, 1416, 1230 $cm^{-1}$;
$^{1}H$ NMR (80MHz, $CDCl_3$) δ (TMS):
8.73 (broad d, J = 1.6Hz, 1H, pyr),
8.61 (broad d, J = 3.6Hz, 1H, pyr),
7.80 (d of t, $J_a$ = 7.9Hz, $J_b$ = 1.6Hz, 1H, pyr),
7.25 (m, 6H, pyr), Ph),
4.89 (s, 1H, CHCN),
3.72 (s, 2H, $CH_2CO$),
3.60 (m, 2H, pip),
3.47 (m, 2H, pip),
2.59 (m, 2H, pip),
2.32 (m, 2H, pip).
Analysis Calcd. for $C_{19}H_{20}N_4O.1/2H_2O$: C 69.22 %; H 6.37%; N 17.00%.
Found: C 69.39%; H 6.12%; N 17.12%.

**EXAMPLE 6**

**1-(3-Phenylpropanoyl)-4-(3-pyridylcyanomethyl)piperazine, dichlorhydrate.**

**a ) 1-(3-Phenylpropanoyl)-4-(3-pyridylcyanomethyl)piperazine**

Following the procedure described in example 3 but using 3-phenylpropanoyl chloride instead of 2-naphthylacetyl chloride the title compound of this example was obtained as a colorless oil (40% yield).
IR (film) ν : 2911, 1633, 1448, 1434, 1421 $cm^{-1}$;
$^{1}H$ NMR (80MHz, $CDCl_3$) δ (TMS):
8.76 (d, J = 2.2Hz, 1H, pyr),
8.63 (dd, $J_a$ = 4.7Hz, $J_b$ = 1.5Hz, 1H, pyr),
7.84 (d de t, $J_a$ = 7.8Hz, $J_b$ = 1.6Hz, 1H, pyr),
7.23 (m, 6H),
4.91 (s, 1H, CHCN),
3.64 (m, 2H, pip),
3.42 (m, 2H, pip),
2.98 (m, 2H, $CH_2CO$),
2.60 (m, 6H).

**b) Title compound of the example.**

Following the procedure described in example 1b but starting with the compound obtained in example 6a, the title compound of this example was obtained as a white solid (85% yield)
mp: 34.7-49.7 ° C;
IR (KBr) ν : 3417, 2825, 1625, 1540, 1462, 1228 $cm^{-1}$;
$^{1}H$ NMR (80MHz, DMSO-$d_6$) δ (TMS):
8.95 (m, 2H),
8.50 (broad d, J = 8.0Hz, 1H, pyr),
8.04 (dd, $J_a$ = 8.1 Hz, $J_b$ = 5.5Hz 1H, pyr),
7.24 (s, 5H),
5.78 (m, CHCN, HCl),
3.49 (m, 4H, pip),
2.70 (m, 4H),
2.51 (m, 4H).
Analysis Calcd. for $C_{20}H_{23}N_4O.2HCl$: C 58.83 %; H 6.17%; N 13.72%.
Found: C 58.48%; H 6.25%; N 13.36%.

**EXAMPLE 7**

**1-(4-Phenylbutanoyl)-4-(3-pyridylcyanomethyl)piperazine.**

Following the procedure described in example 1a but using 4-phenylbutanoic acid instead of 2-naphthoic acid the title compound of this example was obtained as a white solid (64% yield)
mp: 153.2-160.7 °C;
IR (KBr) ν : 3441, 3021, 2919, 1633, 1421, 1234,$cm^{-1}$;
$^1H$ NMR (80MHz, $CDCl_3$) δ (TMS):
8.77 (broad s, 1H, pyr),
8.65 (broad d, J=4.5Hz, 1H, pyr),
7.85 (broad d, J=8.0Hz, 1H, pyr),
7.22 (m, 6H, pyr, Ph),
4.91 (s, 1H, CHCN),
3.61 (m, 2H, pip),
3.42 (m, 2H, pip),
2.7-1.8 (complex signal, 10H).
Analysis Calcd. for $C_{21}H_{24}N_4O.1/4H_2O$: C 71.49%; H 6.95%; N 15.89%.
Found: C 71.66%; H 6.64%; N 15.94%.

**EXAMPLE 8**

**1-(2,2-Diphenylpropanoyl)-4-(3-pyridylcyanomethyl)piperazine, dihydrochloride**

**a) 1-(2,2-Diphenylpropanoyl)-4-(3-pyridylcyanomethyl)piperazine**

Following the procedure described in example 1a but using 2,2-diphenylpropanoic acid instead of 2-naphthoic acid the title compound of this example was obtained as a colorless oil (60% yield)
IR (film) ν : 3051, 3027, 2985, 2931, 1632, 1592, 1487, 1444, 1416, 1236 $cm^{-1}$;
$^1H$ NMR (80MHz, $CDCl_3$) δ (TMS):
8.65 (m, 2H, pyr),
7.82 (broad d, J=6.4Hz, 1H, pyr),
7.32 (m, 11H),
4.70 (s, 1H, CHCN),
3.8 (m, 4H, pip),
2.18 (m, 4H, pip),
1.86 (s, 3H, $CH_3CH$).

**b) Title compound of the example.**

Following the procedure described in example 1b but starting with the compound obtained in example 8a the title compound of this example was obtained as a white solid(80% yield)
mp: 127.9-128.9 °C;
IR (KBr) ν : 3418, 3051, 2822, 1623, 1455, 1417, 1236, $cm^{-1}$;
$^1H$ NMR (80MHz, $CDCl_3$) δ (TMS):
9.32 (m, 1H, pyr),
8.72 (m, 4H, pyr, HCl),
8.03 (m, 1H, pyr),
7.34 (m, 10H, Ar),
5.93 (s, 1H, CHCN),
3.54 (m, 4H, pip),
2.40 (m, 4H, pip),
1.90 (s, 3H, $CH_3CH$).
Analysis Calcd. for $C_{26}H_{26}N_4O.2HCl$: C 64.60 %; H 5.84%; N 11.59%.
Found: C 64.76%; H 5.86%; N 11.48%.

**EXAMPLE 9**

**1-(Diphenylacetyl)-4-(3-pyridylcyanomethyl)piperazine, dihydrochloride**

**a) 1-(Diphenylacetyl)-4-(3-pyridylcyanomethyl)piperazine**

Following the procedure described in example 1a but using diphenylacetic acid instead of 2-naphthoic

acid the title compound of this example was obtained as a white solid (50% yield)
mp: 204.5-205.5 °C;
IR (KBr) ν : 3048, 3022, 2923, 1636, 1421, 1228 $cm^{-1}$;
$^1$H NMR (80MHz, $CDCl_3$) δ (TMS):
8.75 (broad s, 1H, pyr),
8.62 (dd, $J_a$=4.7Hz, $J_b$=1.2Hz, 1H, pyr),
7.79 (broad d, J=6.4Hz, 1H, pyr),
7.28 (m, 11H, pyr, Ar),
5.17 (s, 1H, $CHPh_2$),
4.83 (s, 1H, CHCN),
3.74 (m, 2H, pip),
3.49 (m, 2H, pip),
2.57 (m, 2H, pip),
2.26 (m, 2H, pip);
$^{13}$C NMR (20.15 MHz, $CDCl_3$) δ (TMS): 170.30 (C), 150.46 (CH), 149.32 (CH), 139.13 (C), 135.37 (CH), 128.95 (CH), 128.62(CH), 128.45 (C), 127.12 (CH), 123.46 (CH), 113.75 (C), 59.83 (CH), 54.94 (CH), 49.30 ($CH_2$), 45.52 ($CH_2$), 41.79 ($CH_2$).
Analysis Calcd. for $C_{25}H_{24}N_4O.1/4H_2O$: C 74.88%; H 6.11%; N 13.97%.
Found: C 75.15%; H 5.94%; N 13.89%.

**b) Title compound of the example.**

Following the procedure described in example 1b, but starting with the compound obtained in example 9a the title compound of this example was obtained as a white solid (63% yield)
mp: 125.4-135.4 °C;
IR (KBr) ν : 3406, 3054, 2823, 1632, 1546, 1454, 1428 $cm^{-1}$;
$^1$H NMR (80MHz, DMSO) δ (TMS):
8.85 (m, 2H, pyr),
8.46 (broad d, J=8.8Hz, 1H, pyr),
8.0 (dd, $J_a$=8.0Hz, $J_b$=5.5Hz 1H, pyr),
7.22 (s, 10H),
5.75 (s, 1H),
5.25 (s, 1H),
4.0 (s, $H^+$, $H_2O$),
3.55 (m, 4H, pip),
2.52 (m, 4H, pip).
Analysis Calcd. for $C_{25}H_{24}N_4O.2HCl.1/2H_2O$: C 62.76%; H 5.65%; N 11.71%. Found: C 62.76%; H 5.61%; N 11.42%.

**EXAMPLE 10**

**1-(2,3-Diphenylpropanoyl)-4-(3-pyridylcyanomethyl)piperazine**

Following the procedure described in example 1a, but using 2,3-diphenylpropanoic acid instead of 2-naphthoic acid the title compound of this example was obtained as a white solid (31% yield)
mp: 155-157 °C;
IR (KBr) ν : 2928, 1628, 1448, 1422, 1239 $cm^{-1}$;
$^1$H NMR (80MHz, $CDCl_3$) δ (TMS):
8.65 (m, 2H, pyr),
7.78 (broad d, J=7.8Hz, 1H, pyr),
7.23 (broad s, 11H),
4.77 (s, 1H, CHCN),
3.95 (t, J=7.0Hz, 1H, CHPh),
3.48 (complex signal, 5H),
2.93 (Half an AB system, $J_a$ =13.3Hz, $J_b$=7.0Hz 1H, $CH_2Ph$),
2.44(m, 2H, pip),
2.03 (m, 2H, pip).
Analysis Calcd. for $C_{26}H_{26}N_4O.3/4\ H_2O$: C 73.64 %; H 6.53%; N 13.21%. Found: C 73.80%; H 6.32%; N

13.19%.

**EXAMPLE 11**

**1-(2, 4-Diphenylbutanoyl)-4-(3-pyridylcyanomethyl)piperazine**

Following the procedure described in example 1a but using 2,4-diphenylbutanoic acid instead of 2-naphthoic acid the title compound of this example was obtained as a white solid (76% yield)
mp: 110.6-115.3 °C;
IR (KBr) ν : 3021, 2918, 2856, 1633, 1488, 1448, 1421, 1241 $cm^{-1}$;
$^1H$ NMR (80MHz, $CDCl_3$) δ (TMS):
8.68 (m, 2H, pyr),
7.79 (broad d, J = 7.9Hz, 1H, pyr),
7.20 (broad s, 11H),
4.80 (s, 1H, CHCN),
3.62 (m, 3H, CHPh, pip),
3.31 (m, 2H, pip),
2.8-1.9 (complex signal, 8H).
Analysis Calcd. for $C_{27}H_{28}N_4O$: C 76.38 %; H 6.64%; N 13.19%. Found: C 76.39%; H 6.59%; N 13.15%.

**EXAMPLE 12**

**1-(3,3-Diphenylpropanoyl)-4-(3-pyridylcyanomethyl)piperazine**

Following the procedure described in example 3 but using 3,3-diphenylpropanoyl chloride instead of 2-naphthylacetyl chloride the title compound of this example was obtained as a white solid (53% yield)
mp: 160.7-160.9 °C;
IR (KBr) ν : 3049, 3025, 2933, 1612, 1476, 1454, 1421, 1323, 1258 $cm^{-1}$;
$^1H$ NMR (80MHz, $CDCl_3$) δ (TMS):
8.67 (broad s, 2H, pyr),
8.63 (d, J = 4.3Hz, 1H, pyr),
7.82 (broad d, J = 7.9Hz 1H, pyr),
7.23 (m, 11H, pyr, Ar),
4.82 (s, 1H, CHCN),
4.64 (t, J = 7.6Hz, 1H, $CHPh_2$),
3.55 (m, 2H, pip),
3.35 (m, 2H, pip),
3.04 (d, J = 7.5Hz, 2H, $CH_2CO$),
2.32 (m, 4H, pip).
Analysis Calcd. for $C_{26}H_{26}N_4O$: C 76.07%; H 6.38%; N 13.65%. Found: C 76.06%; H 6.46%; N 13.60%.

**EXAMPLE 13**

**1-(2-Phenylmethyl-3-phenylpropanoyl)-4-(3-pyridylcyanomethyl) piperazine, dichlorhydrate**

**a) 1-(2-Phenylmethyl-3-phenylpropanoyl)-4-(3-pyridylcyanomethyl) piperazine**

Following the procedure described in example 1a but using 2-phenylmethyl-3-phenylpropanoic acid instead of 2-naphthoic acid the title compound of this example was obtained as a colorless oil (57% yield)
IR (film) ν : 3022, 2915, 1631, 1448, 1420, 1368, 1235 $cm^{-1}$;
$^1H$ NMR (80MHz, $CDCl_3$) δ (TMS):
8.63 (m, 2H, pyr),
7.72 (broad d, J = 7.8Hz, 1H, pyr),
7.21, (broad s, 11H),
4.67 (s, 1H, CHCN),
3.45 (m, 2H, pip),
3.3-2.6 (complex signal, 7H),
2.23 (m, 2H, pip),

1.70 (m, 2H, pip);
$^{13}C$ NMR (20.15MHz, $CDCl_3$) δ (TMS): 172.14 (C), 149.68 (CH), 148.62 (CH), 139.00 (C), 134.81 (CH), 128.26 (CH), 127.94(CH), 126.09 (CH), 122.89 (CH), 113.5 (C), 58.73 (CH), 48.53 ($CH_2$), 48.34 ($CH_2$), 45.17 (CH), 44.26 ($CH_2$), 40.40 ($CH_2$), 39.15 ($CH_2$).

**b) Title compound of the example.**

Following the procedure described in example 1b, but starting with the compound obtained in example 13a the title compound of this example was obtained as a white solid (65% yield)
mp: 82.3-91.2 °C;
IR (KBr) ν : 3414, 3021, 2916, 1624, 1448 $cm^{-1}$.
Analysis Calcd. for $C_{27}H_{28}N_4O.2HCl.1/2H_2O$: C 64.03 %; H 6.16%; N 11.06%. Found: C 63.81%; H 5.93%; N 10.92%.

**EXAMPLE 14**

**1-(2-Phenylpropanoyl)-4-(3-pyridylcyanomethyl)piperazine, dihydrochloride**

**a)1-(2-Phenylpropanoyl)-4-(3-pyridylcyanomethyl)piperazine.**

Following the procedure described in example 1a, but using 2-phenylpropanoic acid instead of 2-naphthoic acid, the title compound of this example was obtained as a colorless oil (65% yield)
IR (KBr) ν : 2925, 1633, 1422, 1232 $cm^{-1}$.
$^{1}H$ NMR (80MHz, $CDCl_3$) δ (TMS):
8.62 (m, 2H, pyr),
7.77 (broad d, J = 7.8Hz, 1H, pyr),
7.29, (broad s, 6H),
4.88 (s, 1H, CHCN),
3.87 (q, J = 6.9Hz, 1H, $CHCH_3$),
3.50 (m, 4H, pip),
2.48 (m, 4H, pip),
1.42 (d, J = 6.9Hz, 3H);
$^{13}C$ NMR (20.15MHz, $CDCl_3$) δ (TMS): 171.83 (C), 150.13 (CH), 148.99 (CH), 141.50 (C), 135.23 (CH), 128.79 (CH), 126.89(CH), 126.78 (CH), 123.29 (CH), 113.60 (C), 59.53 (CH), 49.04 ($CH_2$), 44.74 ($CH_2$), 43.04 (CH), 41.41 ($CH_2$), 20.35 ($CH_3$).

**b) Title compound of the example.**

Following the procedure described in example 1b, but starting with the compound obtained in example 14a the title compound of this example was obtained as a white solid (82% yield)
mp: 81.1-92.5 °C;
IR (KBr) ν : 3417, 3053, 2925, 1632, 1546, 1461, 1231 $cm^{-1}$.
Analysis Calcd. for $C_{20}H_{22}N_4O.2HCl$: C 58.97 %; H 5.94%; N 13.75%.
Found: C 58.76%; H 5.84%; N 13.52%.

**EXAMPLE 15**

**1-(3-Phenylpropenoyl)-4-(3-pyridylcyanomethyl)piperazine, dichlorhydrate**

Following the procedure described in example 3, but using cinnamoyl chloride instead of 2-naphthylacetyl chloride, the title compound of this example was obtained as a white solid (85% yield)
mp:180.0-180.5 °C;
IR (KBr) ν : 3022, 2911, 2812, 1637, 1595, 1455, 1433, 1411, 1222 $cm^{-1}$.
$^{1}H$ NMR (80MHz, $CDCl_3$) δ (TMS):
8.81 (m, 1H, pyr),
8.65 (m, 1H, pyr),
7.87 (broad d, J = 7.8Hz, 1H, pyr),
7.68 (d, J = 15.4Hz, 1H, CH = C),

7.42 (m, 11H, pyr, Ph),
6.83 (d, J = 15.4Hz, 1H, CH = C),
4.94 (s, 1H, CHCN),
3.74 (m, 4H, pip),
2.63 (m, 4H, pip);
Analysis Calcd. for $C_{20}H_{20}N_4O$: C 72.26 %; H 6.06%; N 16.85%. Found: C 72.58%; H 5.84%; N 17.01%.

**EXAMPLE 16**

**1-(2,3-Diphenylpropenoyl)-4(3-pyridylcyanomethyl)piperazine.**

Following the procedure described in example 1a, but using 2,3-diphenylpropenoic acid instead of 2-naphthoic acid, the title compound of this example was obtained as a white solid (53% yield)
mp: 132.7-133.2 °C;
IR (KBr) ν : 3050, 3018, 2912, 1624, 1454, 1422, 1245 $cm^{-1}$.
$^1H$ NMR (80MHz, $CDCl_3$) δ (TMS):
8.68 (m, 2H, pyr),
7.83 (broad d, J = 7.8Hz 1H, pyr),
7.35 (m, 1H, pyr),
7.28 (s, 5H, Ph),
7.14 (s, 5H, Ph),
4.91 (s, 1H, CHCN),
3.63 (m, 4H, pip),
2.47 (m, 4H, pip);
Analysis Calcd. for $C_{26}H_{24}N_4O.1/4H_2O$: C 75.63 %; H 5.93%; N 13.57%.
Found: C 75.57%; H 5.84%; N 13.48%.

**EXAMPLE 17**

**1-(3,3-Diphenylpropenoyl)-4-(3-pyridylcyanomethyl)piperazine.**

Following the procedure described in example 1a, but using 3,3-diphenylpropenoic acid instead of 2-naphthoic acid, the title compound of this example was obtained as a white solid (74% yield)
mp: 69.0-71.7 °C;
IR (KBr) ν : 3051, 2978, 2911, 1626, 1571, 1456, 1430, 1245 $cm^{-1}$;
$^1H$ NMR (80MHz, $CDCl_3$) δ (TMS):
8.68 (m, 2H, pyr),
7.75 (broad d, J = 7.8Hz, 1H, pyr),
7.30, (broad s, 11H),
6.28 (s, 1H, CH = C),
4.79 (s, 1H, CHCN),
3.57 (m, 2H, pip),
3.31 (m, 2H, pip),
2.38 (m, 2H, pip),
1.95 (m, 2H, pip).
Analysis Calcd. for $C_{26}H_{24}N_4O$: C 76.45 %; H 5.92%; N 13.72%. Found: C 76.88%; H 5.83%; N 13.39%.

**EXAMPLE 18**

**1-(Pentanoyl)-4-(3-pyridylcyanomethyl)piperazine**

To a solution of 0.7 g (3,7 mmol) of the compound obtained in preparation 1 and 0.6 mL of triethylamine in 5 mL anhydrous chloroform, under argon atmosphere, 0.9 mL (4.81 mmol) of pentanoic anhydride were added. The mixture was stirred 18 h. at room temperature. The organic solution was washed with water, dried and the solvent was removed yielding 1 g of crude that was purified by chromatography on silica gel (AcOEt: Methanol 5%) to give 0.59 g of a white solid (56%yield).
mp.: 101.2-102.1 °C;
IR(KBr) ν : 2952, 1633, 1421, 1226 $cm^{-1}$;

$^1H$ NMR (80MHz, $CDCl_3$) δ (TMS):
8.73 (m, 2H, pyr),
7.88 (broad d, J=7.8Hz, 1H, pyr),
7.37 (dd, $J_a$=7.8Hz, $J_b$=4.7Hz, 1H, pyr),
4.94 (s, 1H, CHCN),
3.60 (m, 4H, pip),
2,58 (t, J=5.0Hz, 4H, pip),
2.33 (t, J=7.7Hz, 2H),
1,6 (m, 4H),
0.92 (broad t, J=6,2Hz, 3H).
Analysis calcd. for: $C_{16}H_{22}N_4O$: C 67.10%, H 7.74%, N 19.56%.
Found: C 67.08%, H 7.87%, N 19.69%.

**EXAMPLE 19**

**1-(Octanoyl)-4-(3-pyridylcyanomethyl)piperazine hydrochloride**

**a) 1-(octanoyl)-4-(3-pyridylcyanomethyl)piperazine**

Following the procedure described in example 18, but using octanoyl chloride instead of pentanoic anhydride, the title compound of this example was obtained as a colorless oil (98 % yield)
IR(film) ν : 2924, 2853, 1641, 1454, 1420, 1229 $cm^{-1}$;
$^1H$ NMR (80MHz, $CDCl_3$) δ (TMS):
8.79 (d, J=2.4Hz, 1H, pyr),
8.64 (dd, $J_a$=4.7Hz, $J_b$=1.5Hz, 1H, pyr),
7.87 (broad d, J=8.0Hz, 1H, pyr),
7.37 (dd, $J_a$=7.4Hz, $J_b$=5.1Hz, 1H, pyr),
5.02 (s, 1H, CHCN),
3.59 (m, 4H, pip),
2.50 (complex signal, 6H, pip, $CH_2CO$),
1.30 (complex signal, 10H),
0.87 (m, 3H).

**b) Title compound of the example**

To a solution of 0.5 g of the compound obtained in the previous section in 5 mL ethyl acetate, cooled to 0 °C, were added 1.5 mL saturated solution of hydrochloric acid gas in ethyl ether, yielding 0.27 g of a white solid (57% yield)
m.p.: 125.8-132.5 °C;
IR(KBr) ν : 2909, 2844, 2109, 1650, 1454, 1427 $cm^{-1}$;
$^1H$ NMR (80MHz, $CDCl_3$) δ (TMS):
11.8 (m, 2H),
9.29 (m, 1H, pyr),
8.95 (broad d, J=4.6 Hz, 1H, pyr),
8.65 (broad d, J=7.7 Hz, 1H, pyr),
8.12 (t, J=6.0Hz, 1H, pip),
5.69 (s, 1H, CHCN),
3.62 (m, 4H, pip),
2.62 (m, 4H, pip),
2.30 (m, 2H, $CH_2CO$),
1,30 (m, 10H),
0.87 (m, 3H).
Analysis calcd. for : $C_{19}H_{28}N_4O.HCl.3/4H_2O$ : C 60.31%, H 8.12%, N 14.80%.
Found: C 60.69%, H 7.85%, N 14.40%.

**EXAMPLE 20**

**1-(Hexadecanoyl)-4-(3-pyridylcyanomethyl)piperazine**

Following the procedure described in example 18, but using hexadecanoyl chloride instead of pentanoic anhydride, the title compound of this example was obtained as a white solid (56%yield).
m.p.: 67.1-70.1 °C;
IR(KBr)ν: 2911, 2844, 2752, 1650, 1460, 1420, 1321, 1303, 1241, 1225, 1201 $cm^{-1}$;
$^1H$ NMR (80MHz, $CDCl_3$) δ ppm (TMS):
8.78 (broad s, 1H, pyr),
8.66 (broad d J=4.8Hz, 1H, pyr),
7.86 (broad d, J=7.8Hz, 1H, pyr),
7.37 (dd, $J_a$=4.8Hz, $J_b$=7.8Hz, 1H, pyr),
4.92 (s, 1H, CHCN),
3.59 (m, 4H, pip),
2.57 (t, J=5.1Hz, 4H, pip)
2.32 (t, J=8.0Hz, 2H),
1.25 (m, 26H),
0.88 (m, 3H).
Analysis calcd. for $C_{27}H_{44}N_4O$: C 73.59%, H 10,06%, N 12.71%.
Found: C 73.56%, H 10,33%, N 12,44%.

**EXAMPLE 21**

**1-(Trimethylacetyl)-4-(3-pyridylcyanomethyl)piperazine**

Following the procedure described in example 18, but using pivaloyl chloride instead of pentanoic anhydride, the title compound of this example was obtained as a white solid (86% de rend).
m.p.: 134.5-136.4 °C
IR(KBr) ν : 2973, 2820, 1618, 1475, 1417, 1187 $cm^{-1}$;
$^1H$ NMR (80MHz, $CDCl_3$) δ (TMS):
8.77 (broad d, J = 1.6Hz, 1H, pyr),
8.62 (broad d, J = 3.9Hz, 1H, pyr),
7.88 (broad d, J = 8.0Hz, 1H, pyr),
7.39 (dd, $J_a$ = 7.9Hz, $J_b$ = 4.8Hz, 1H, pyr),
5.17 (s, 1H, CHCN),
3.69 (m, 4H, pip),
2.58 (m, 4H, pip),
1.26 (m, 9H).
Analysis calcd. for $C_{16}H_{22}N_4O$: C 67.11%; H 7.74%; N 19.56%.
Found: C 67.37%; H 8.03%; N 19.64%.

**EXAMPLE 22**

**1-(3,4,5-Trimethoxybenzoyl)-4-(3-pyridylcyanomethyl)piperazine, dihydrochloride**

**a) 1-(3,4,5-Trimethoxybenzoyl)-4-(3-pyridylcyanomethyl)piperazine**

Following the procedure described in example 18, but using 3,4,5-trimethoxybenzoyl chloride, a colorless oil was obtained (75%yield)
IR(film) ν : 2935, 2827, 1624, 1580, 1458, 1421, 1327 $cm^{-1}$
$^1H$ NMR (80MHz, $CDCl_3$) δ ppm (TMS):
8.79 (broad s, 1H, pyr),
8.65 (broad d, J=3.6Hz, 1H, pyr),
7.88 (broad d, J=8.0Hz, 1H, pyr),
7.37 (dd, $J_a$=8.2Hz, $J_b$=4.8Hz, 1H, pyr),
6.61 (s, 2H, Ar),
4.97 (s, 1H, CHCN),
3.85 (s, 9H, $CH_3O$),
3.67 (m, 4H, pip),
2.62 (m, 4H, pip),
$^{13}C$ NMR (20.15MHz, $CDCl_3$) δ ppm (TMS): 169.86 (C, CO), 153.17 (C, pyr), 150.29 (CH, pyr), 149.11 (CH,

pyr), 135.20 (CH, pyr), 130.40 (C), 128.22 (C), 123.33 (CH, pyr), 113.72 (CH, CN), 104.38 (CH, Ar), 60.57 ($CH_3$, $OCH_3$), 59.68 (CH, CHCN), 56.10 ($CH_3$, $OCH_3$), 49.43 ($CH_2$, pip), 48.00 ($CH_2$, pip),

**b) Title compound of the example.**

Following the procedure described in example 19b, but using the compound obtained in example 22a, the title compound of this example was obtained as a white solid in a similar yield.
m.p.: 86.6-86.6 ° C
IR(KBr) $\nu$ : 3419, 2935, 2572, 1625, 1580, 1499, 1112 $cm^{-1}$,
Analysis calcd. for : $C_{21}H_{24}H_4O_4$.2HCl.$H_2O$: C 51.75%; H 5.79%; N 11.50%;
Found: C 51.88%; H 5.40%; N 11.64%.

**EXAMPLE 23**

**1-(3,4,5-Trimethoxyphenylacetyl)-4-(3-pyridylcyanomethyl)piperazine**

To a mixture of 0.7 g (3.7 mmol) of the compound obtained in preparation 1, 0.8 g (3.7 mmol) of 3,4,5-trimethoxyphenylacetic acid and 0.75 g (5.5mmol) of 1-hydroxybenzotriazole in 20 mL dimethylformamide, cooled to 0 ° C and under $N_2$ atmosphere, were added 0.78 g (3.78 mmol) of dicyclohexylcarbodiimide. The resulting mixture was stirred 18 h. at room temperature. Then, the solvents were removed in vacuo and the residue was stirred with ethyl acetate. The white solid formed was separated and the organic solution was washed with brine. After drying over sodium sulfate and removal of the solvent, 1.4 g of crude were obtained. Purification by chromatography on silica gel (AcOEt /MeOH 3%) afforded 0.67g of a white solid that was recrystallized from ethyl acetate (44%yield)
mp: 161.1-161.4 ° C,
IR(KBr) $\nu$ : 2993, 2935, 1643, 1586, 1453, 1418, 1229 $cm^{-1}$
$^1$H NMR (80MHz, $CDCl_3$) $\delta$ (TMS):
8.76 (m, 1H, pyr),
8.65 (d, J = 3.6Hz, 1H, pyr),
7.83 (broad d J = 8.1Hz, 1H, pyr),
7.36 (dd, $J_a$ = 8.0Hz, $J_b$ = 4.8Hz, 1H, pyr),
6.43 (s, 2H, Ar),
4.89 (s, 1H, CHCN),
3.84 (s, 9H, $CH_3O$),
3.67 (s, 2H, $CH_2CO$),
3.57 (m, 4H, pip),
2.51 (m, 4H, pip),
Analysis calcd. for : $C_{22}H_{26}N_4O_4$ . 1/2 $H_2O$: C 63.00%; H 6.44%; N 13.36%.
Found: 63.02%; H 6.20%; N 13.14%.

**EXAMPLE 24**

**1-(3-(3,4,5-Trimethoxyphenyl)propanoyl)-4-(3-pyridylcyanomethyl) piperazine, dihydrochloride**

**a) 1-(3-(3,4,5-Trimethoxyphenyl)propanoyl)-4-(3-pyridylcyanomethyl) piperazine**

Following the procedure described in example 23, but using 3-(3,4,5-trimethoxyphenyl)propanoic acid instead of 3,4,5-trimethoxyphenylacetic acid, the title compound of this example was obtained as a colorless oil (60% yield)
IR(film) $\nu$ : 2934, 2827, 2245, 1632, 1586, 1503, 1454, 1420, 1325, 1237 $cm^{-1}$
$^1$H NMR (80MHz, $CDCl_3$) $\delta$ (TMS):
8.76 (broad s, 1H, pyr),
8.63 (broad d, J = 4.8Hz, 1H, pyr),
7.85 (broad d, J = 7.8Hz, 1H, pyr),
7.36 (dd, $J_a$ = 7.8Hz, $J_b$ = 4.8Hz, 1H, pyr),
6.43 (s, 2H, Ar),
4.96 (s, 1H, CHCN),
3.83 (s, 6H, $OCH_3$),

3.80 (s, 3H, $OCH_3$),
3.54 (m, 4H, pip),
2.91 (m, 2H),
2.55 (m, 6H).

**b) Title compound of the example**

Following the procedure described in example 19b, but using the compound obtained in example 24a, the title compound of this example was obtained as a white solid (82%yield)
mp: <30 °C
IR(KBr) $\nu$ : 3417, 2931, 2833, 1625, 1586, 1503, 1456, 1123 $cm^{-1}$
$^1H$ NMR (80MHz, DMSO-$d_6$) $\delta$ (TMS):
8.90 (m, 2H, pyr),
8.51 (broad d, J = 8.8Hz, 1H, pyr),
8.04 (dd, $J_a$ = 8.1Hz, $J_b$ = 5.4Hz, 1H, pyr),
6.54 (s, 2H, Ar),
5.82 (s, 1H, CHCN),
4.56 ($H_2O$),
3.75 (s, 6H, $CH_3O$),
3.61 (s, 3H, $CH_3O$),
3.51 (m, 4H, pip),
2.55 (complex signal, 8H)
Analysis calcd. for : $C_{23}H_{28}N_4O_4.2HCl.1H_2O$: C 53.59%; H 6.25%; N 10.86%.
Found: C 53.45%; H 6.10%; N 10.59%

**EXAMPLE 25**

**1-(3,3-bis(4-methoxyphenyl)propanoyl)-4-(3-pyridylcyanomethyl) piperazine**

Following the procedure described in example 23, but using 3,3-bis(4-methoxyphenyl)propanoic acid instead of 3,4,5-trimethoxyphenylacetic acid, the title compound of this example was obtained as a white solid (66%yield)
mp: 56.3-58.9 °C,
IR(KBr) $\nu$ : 3448, 2930, 2830, 1632, 1605, 1507, 1455, 1437, 1421 $cm^{-1}$
$^1H$ NMR (80MHz, $CDCl_3$) $\delta$ (TMS):
8.74 (d, J = 2.2Hz, 1H, pyr),
8.63 (dd, $J_a$ = 4.8Hz, $J_b$ = 1.6Hz, 1H, pyr),
7.81 (d de t, $J_a$ =8.2Hz, $J_b$ = 1.6Hz, 1H, pyr),
7.33 (dd, $J_a$ = 7.8Hz, $J_b$ = 4.9Hz, 1H, pyr),
7.15 (d, J = 8.6Hz, 4H, Ar),
6.80 (d, J = 8.6Hz, 4H, Ar),
4.85 ( s, 1H, CHCN),
4.52 (t, J = 7.5Hz, 1H, $Ar_2CH$),
3.76 (s, 6H, $OCH_3$),
3.58 (m, 2H, pip),
3.37 (m, 2H, pip),
3.98 (d, J = 7.6 Hz, 2H, $CH_2CO$),
2.36 (m, 4H, pip),
Analysis calcd. for : $C_{28}H_{30}N_4O_3$ . 1/4 $H_2O$: C 70.72%; H 6.42%; N 11.78%.
Found: C 70.73%; H 6.37%; N 11.79%.

**EXAMPLE 26**

**1-(3,3-bis(4-methoxyphenyl)propenoyl)-4-(3-pyridylcyanomethyl)piperazine, dihydrochloride**

**a) 1-(3,3-bis(4-methoxyphenyl)propenoyl)-4-(3-pyridylcyanomethyl)piperazine**

Following the procedure described in example 23, but using 3,3-bis(4-methoxyphenyl)propenoic acid

instead of 3,4,5-trimethoxyphenylacetic acid, the title compound of this example was obtained as a colorless oil (57%yield)
IR(KBr) ν : 2925, 2832, 1622, 1600, 1508, 1246 $cm^{-1}$
$^1H$ NMR (80MHz, $CDCl_3$) δ (TMS):
8.71 (d, J = 2.2Hz, 1H, pyr),
8.61 (dd, $J_a$ = 4.8Hz, $J_b$ = 1,4 Hz, 1H, pyr),
7.80 (broad d, J = 7,2 Hz, 1H, pyr),
7.33 (dd, $J_a$ = 8.2Hz, $J_b$ = 4.6Hz, 1H, pyr),
7.20 (m, 4H, Ar),
6.75 (m, 4H, Ar),
6.11 (s, 1H, C=C),
4.82 (s, 1H, CHCN),
3.83 (s, 3H, $OCH_3$),
3.80 (s, 3H, $OCH_3$),
3.58 (m, 2H, pip),
3.33 (m, 2H, pip),
2.40 (m, 2H, pip),
2.02 (m, 2H, pip).

**b) Title compound of the example**

Following the procedure described in example 19b, but using the compound obtained in example 26a, the title compound of this example was obtained as a white solid (58%yield).
mp: 100.7-110.3° C,
IR(KBr) ν : 3400, 2832, 1600, 1507, 1456, 1434, 1246 $cm^{-1}$
Analysis calcd. for : $C_{28}H_{28}N_4O_3$ . 2HCl:C 62.10%; H 5.58%; N 10.34%. Found: C 61.71%; H 5.52%; N 10.52%.

**EXAMPLE 27**

**1-(2-phenyl-2-hydroxyacetyl)-4-(3-pyridylcyanomethyl)piperazine, dihydrochloride**

**a) 1-(2-phenyl-2-hydroxyacetyl)-4-(3-pyridylcyanomethyl)piperazine**

Following the procedure described in example 23, but using mandelic acid instead of 3,4,5-trimethoxyphenylacetic acid, the title compound of this example was obtained as a colorless oil (32%yield)
IR(KBr)ν : 3397, 2912, 2823, 2227, 1637, 1573, 1472, 1446, 1420, 1391, 1248 $cm^{-1}$
$^1H$ NMR (80MHz, $CDCl_3$) δ (TMS):
8.72 (m, 2H, pyr),
7.82 (broad d, J = 8.4Hz, 1H, pyr),
7.34 (m, 6H, pyr + Ar),
5.19 (s, 1H, CHCN),
4.83 (s, 1/2H, CHCH),
4.80 (s, 1/2H, CHCH),
3.71 (m, 2H, pip),
3.26 (m, 2H, pip),
2.52 (m, 4H, pip),

**b) Title compound of the example**

Following the procedure described in example 19b, but using the compound obtained in example 27a, the title compound of this example was obtained as a white solid (75%yield)
mp: 138.4-154.3° C;
IR(KBr) ν : 3391, 3056, 2823, 1632, 1448, 1272, 1248 $cm^{-1}$
$^1H$ NMR (80MHz, $CDCl_3$) δ (TMS):
8.99 (m, 5H),
8.64 (broad d, J = 8.3Hz, 1H, pyr),
8.12 (dd, $J_a$ = 8.1Hz, $J_b$ = 5.7Hz, 1H, pyr),

7.33 (m, 5H, Ph),
5.76 (s, 1H, CHCN),
5.35 (s, 1H, CHOH),
3.64 (m, 2H, pip),
3.44 (m, 2H, pip),
2.35 (m, 4H, pip).
Analysis calcd. for : $C_{19}H_{20}N_4O_2$ . 2HCl .$H_2O$: C 53.40%; H 5.66%; N 13.11%.
Found: C 53.86%; H 5.30%; N 12.51%.

**EXAMPLE 28**

**1-(3,3-Diphenyl-3-hydroxypropanoyl)-4-(3-pyridylcyanomethyl)piperazine**

Following the procedure described in example 23, but using 3,3-diphenyl-3-hydroxypropanoic acid instead of 3,4,5-trimethoxyphenylacetic acid, the title compound of this example was obtained as a white solid (60%yield)
mp: 62.2-74.3° C;
IR(KBr) $\nu$ : 3345, 3051, 2914, 1612, 1445, 1417, 1230 $cm^{-1}$
$^1H$ NMR (80MHz, $CDCl_3$) $\delta$ (TMS):
8.69 (m, 2H, pyr),
7.85 (broad d, J = 8.5Hz, 1H, pyr),
7.31 (m, 11H, pyr + Ar),
4.87 (s, 1H, CHCN),
3.53 (m, 4H, pyr),
3.20 (s, 2H, $CH_2CO$),
2.45 (t, J = 5.0Hz, 4H, pyr),
Analysis calcd. for: $C_{26}H_{26}N_4O_2$.1/4 $H_2O$:
C 72.11%; H 6.16%; N 12.94%.
Found: C 71.86%; H 6.19%; N 12.57%.

**EXAMPLE 29**

**1-(3,3-Diphenyl-3-hydroxypropanoyl)-4-(4-pyridylcyanomethyl)piperazine**

Following the procedure described in example 23, but using 3,3-diphenyl-3-hydroxypropanoic acid instead of 3,4,5-trimethoxyphenylacetic acid and the compound obtained in preparation 2 instead of the one obtained in preparation 1, the title compound of this example was obtained as a white solid (25%yield)
mp: 73.5-89.2° C
IR(KBr) $\nu$ : 3333, 2914, 2822, 1612, 1445, 1409, 1230 $cm^{-1}$
$^1H$ NMR (80MHz, $CDCl_3$) $\delta$ (TMS):
8.66 (d, J = 6.0Hz, 2H, pyr),
7.33 (m, 12H, pyr + Ar),
6.30 (m, 1H, OH),
4.83 (s, 1H, CHCN),
3.51 (m, 4H, pip),
3.19 (s, 2H, $CH_2CO$)
2.40 (m, 4H, pip)
Analysis calcd. for : $C_{26}H_{26}N_4O_2$ . 1/2 $H_2O$:
C 71.37%; H 6.22%; N 12.80%.
Found: C 71.03%; H 6.08%; N 12.90%.

**EXAMPLE 30**

**1-(Phenoxycarbonyl)-4-(3-pyridylcyanomethyl)piperazine**

Following the procedure described in example 18, but using phenyl chloroformate instead of pentanoic anhydride, the title compound of this example was obtained as a white solid (80%yield)
mp: 144.1-147.7° C;

$^1H$ NMR (80MHz, $CDCl_3$) δ (TMS):
8.80 (m, 1H, pyr),
8.72 (broad d J = 4.8Hz, 1H, pyr),
7.90 (broad d, J = 8.0Hz, 1H, pyr),
7.30 (complex signal, 6H, pyr + Ph),
4.96 (s, 1H, CHCN),
3.69 (m, 4H, pip),
2.68 (t, J = 4.0Hz, 4H, pip);
Analysis calcd. for: $C_{18}H_{18}N_4O_2$
C 67.07%; H 5.63%; N 17.38%.
Found: C 67.03%; H 5.65%; N 17.12%.

**EXAMPLE 31**

**1-((2,2-diphenylethoxy)carbonyl)-4-(3-pyridylcyanomethyl)piperazine, dihydrochloride**

**a) 1-((2,2-diphenylethoxy)carbonyl)-4-(3-pyridylcyanomethyl)piperazine**

A mixture of 1.27 g (6.2 mmol) of the compound obtained in preparation 1, 2 g (6.2 mmol) of 2,2-diphenylethyl phenyl carbonate and 0.7 g of potassium carbonate in 15 mL of dimethylformamide, were heated at 80 °C, under argon atmosphere, for 12 h. The solvent was removed and the mixture worked up with chloroform and 1N sodium hydroxide. The organic phase was separated, dried and the solvent removed yielding 1.85 g of crude that was purified by chromatography on silica gel (hexane: ethyl acetate, 50%). In that way, 0.24 g of a colorless oil were obtained (10%yield)
IR(film) ν : 3022, 2948, 1692, 1488, 1422, 1242 $cm^{-1}$
$^1H$ NMR (80MHz, $CDCl_3$) δ (TMS):
8.75 (m, 2H, pyr),
7.82 (d, J = Hz, 1H, pyr)
7.25 (m, 11H, pyr + Ar),
4.84 (s, 1H, CHCN),
4.55 (complex signal, 3H, $CHCH_2O$),
3.35 (m, 4H, pip),
2.41 (m, 4H, pip).

**b) Title compound of the example**
Following the procedure described in example 19b, but using the compound obtained in example 14a, the title compound of this example was obtained as a white solid (75%yield)
mp: 97.2-97.2 °C
IR(KBr) ν :
3419, 3051, 2824, 1692, 1454, 1427, 1242 $cm^{-1}$
Analysis calcd. for : $C_{26}H_{26}N_4O_2.2HCl.1\ 3/4\ H_2O$:
C 60.87%; H 5.65%; N 10.92%.
Found: C 60.96%; H 5.37%; N 10.96%

**EXAMPLE 32**

**1-((3-phenylpropoxy)carbonyl)-4-(3-pyridylcyanomethyl)piperazine, hydrochloride**

**a) 1-((3-phenylpropoxy)carbonyl)-4-(3-pyridylcyanomethyl)piperazine**

Following the procedure described in example 31, but using 3-phenylpropyl phenyl carbonate instead of 2,2-diphenylethyl phenyl carbonate, the title compound of this example was obtained in a similar yield.
IR(film) ν : 3022, 2943, 1692, 1422, 1243 $cm^{-1}$
$^1H$ NMR (80MHz, $CDCl_3$) δ (TMS):
8.76 (d, J = 2.3Hz, 1H, pyr)
8.62 (dd, $J_a$ = 4.8 Hz, $J_b$ = 1.4Hz, 1H, pyr),
7.86 (broad d, J = 6.3 Hz, 1H, pyr),
7.34 (dd, $J_a$ = 7.7Hz, $J_b$ = 4.9Hz, 1H, pyr),

7.21 (m, 5H, Ph),
4.90 (s, 1H, CHCN),
4.11 (t, J = 6.4Hz, 2H, $CH_2O$),
3.48 (t, J = 4.9Hz, 4H, pip),
2,59 (complex signal, 6H),
1.97 (m, 2H).

**b) Title compound of the example**

Following the procedure described in example 19b, but using the compound obtained in example 32a, the title compound of this example was obtained as a white wax (87%yield)
m.p.< 28° C;
IR(KBr) $\nu$ : 3419, 3054, 2921, 1691, 1461, 1427, 1244 $cm^{-1}$;
$^1H$ NMR (80MHz, $CDCl_3$) $\delta$ (TMS):
9.36 (s, 1H, pyr),
8.01 (broad d, J = 5.2Hz, 1H, pyr),
8.76 (broad d, J = 7.6Hz, 1H, pyr),
8.43 (m, HCl + $H_2O$),
8.10 (dd, $J_a$= 7,8Hz, $J_b$ = 5.8Hz, 1H, pyr),
7.21 (m, 5H, Ph),
5.94 (s, 1H, CHCN),
4.11 (t, J = 6.5Hz, 2H, $CH_2O$),
3.59 (m, 4H, pip),
2.77 (m, 4H, pip),
2.68 (t, J = 7.3Hz, 2H, $CH_2Ph$),
1.97 (q, J = 6.7Hz, 2H, $\overline{CH_2}$-$CH_2$-$CH_2$);
Analysis calcd. for : $C_{21}H_{24}N_4O_2$ . HCl. $H_2O$: C 60.21%, H 6.50%, N 13.37%;
Found: C 60.42%, H 6.57%, N 13.56%.

## EXAMPLE 33

**1-(3-indolylacetyl)-4-(3-pyridylcyanomethyl)piperazine, trihydrochloride**

**a) 1-(3-indolylacetyl)-4-(3-pyridylcyanomethyl)piperazine**

Following the procedure described in example 23, but using 3-indolylacetic acid, instead of 3,4,5-trimethoxyphenylacetic acid, the title compound of this example was obtained as a colorless oil(79%yield).
IR(film) $\nu$ : 3264, 3051, 3002, 2914, 2824, 1624, 1454, 1421, 1336, 1225 $cm^{-1}$
$^1H$ NMR (80MHz, $CDCl_3$) $\delta$ (TMS):
8.71 (m, 3H),
7.78 (broad d, J = 8.0Hz, 1H),
7.54 (m, 1H),
7.25 (m, 4H),
4.80 (s, 1H, CHCN),
3.81 (s, 2H, $CH_2CO$),
3.67 (m, 2H, pip),
3.46 (m, 2H, pip),
2.50 (m, 2H, pip),
2.35 (m, 2H, pip),

**b) Title compound of the example**

Following the procedure described in example 19b, but using the compound obtained in example 33a, the title compound of this example was obtained as a white solid (66%yield)
mp: 111.5 - 120.0° C
IR(KBr) $\nu$ :
3389, 2829, 1624, 1546, 1454, 1337, 1228 $cm^{-1}$
Analysis calcd. for: $C_{21}H_{21}N_5O$ . 3HCl . 1/2$H_2O$: C 52.73%, H 5.12%, N 14.64%; Found: C 52.61%, H

5.02%, N 14.28%.

**EXAMPLE 34**

**1-(*N*-(1-naphthyl)aminoacetyl)-4-(3-pyridylcyanomethyl)piperazine, trihydrochloride.**

**a) 1-(*N*-(1-naphthyl)aminoacetyl)-4-(3-pyridylcyanomethyl)piperazine.**

To a solution of 1 g (3.6 mmol) of the product obtained in preparation 3 in 25 mL anhydrous $CHCl_3$, under argon atmosphere, were added 0.5 mL (3.6 mmol) of triethylamine. The mixture was cooled to 0° C, 1.03 g (7.2 mmol) of 1-naphthylamine were added dropwise and it was stirred one night at room temperature. The resulting mixture was washed with water and dried over sodium sulfate. The solvent was removed yielding 1.7 g of an oil that was purified by chromatography on silica gel eluting with $CHCl_3/MeOH/NH_3$ (60: 2: 0.2) mixtures. 0.37 g of a brown oil were obtained (27% yield).
$^1H$ NMR (80MHz, $CDCl_3$) $\delta$ (TMS):
8.78 (broad s, 1H, pyr),
8.65 (m, 1H, pyr),
8.1-7.2 (m, 8H, pyr, napht.),
6.43 (dd, J = 2.5Hz, J = 6.3Hz, 1H, napht),
4.89 (1H, CHCN),
4.44 (broad s, 1H, NH),
4.00 (s, 2H, $CH_2CO$),
3.60 (m, 4H, pip),
2.61 (m, 4H, pip).

**b) Title compound of the example**

To a solution of 0.37 g of the product obtained in example 34a, in 1 mL anhydrous $CH_2Cl_2$, cooled at 0°, 2 mL saturated solution of hydrochloric acid gas in anhydrous ethyl ether were added. The precipitate formed was filtered and dried in vacuo 24 h. yielding 0.225 g of a yellow solid (47% yield).
mp: 135.1-143.3;
IR(KBr)$\nu$ : 3318, 2925, 2845, 1775, 1729, 1702, 1659, 1620, 1427, 1212, 1100 $cm^{-1}$
Analysis calcd. for : $C_{23}H_{26}Cl_3N_5O$:C 55.83%, H 5.30%, N 14.15%;
Found: C 56.15 %, H 5.31%, N 13.84%.

**EXAMPLE 35**

**1-(*N*-phenylaminoacetyl)-4-(3-pyridylcyanomethyl)piperazine**

Following the procedure described in example 34a, but using aniline instead of 1-naphthylamine the title compound of this example was obtained as a white solid (24% yield).
mp: 138.1 - 141.7° C;
IR(KBr) $\nu$ : 3361, 2938, 2898, 2227, 1649, 1601, 1570, 1509, 1475, 1439, 1421, 1326, 1256, 1129, 1993, 753 $cm^{-1}$;
$^1H$ NMR (80MHz, $CDCl_3$) $\delta$ (TMS):
8.66 (m, 2H, pyr),
7.87 (d, J = 6Hz, 1H, pyr),
7.49 (dd, J = 6Hz, J = 4Hz, 1H, pyr),
7.07 (m, 2H, Ph),
7.62 (m, 3H, Ph),
5.57 (s, 1H, CHCN),
3.91 (s, 2H, $CH_2CO$),
3.55 (m, 4H, pip),
3.31 (broad s, 1H, NH),
2,49 (m, 4H, pip).
Analysis calcd. for : $C_{19}H_{21}N_5O$: C 68.04%, H 6.31%, N 20.88%;
Found: C 67.85%, H 6.65%, N 21.04%.

**EXAMPLE 36**

**1-(*N*-phenyl-*N*-methylaminoacetyl)-4-(3-pyridylcyanomethyl)piperazine, trihydrochloride**

**a) 1-(*N*-phenyl-*N*-methylaminoacetyl)-4-(3-pyridylcyanomethyl)piperazine**

Following the procedure described in example 34a, but using *N*-methylaniline instead of 1-naphthylamine, the title compound of this example was obtained as a colorless oil (43% yield).
IR(film) $\nu$ : 3000, 2911, 2226, 1649, 1595, 1502, 1420, 1233, 999 $cm^{-1}$
$^1H$ NMR (80MHz, $CDCl_3$) $\delta$ (TMS):
8.78 (d, J = 2.5Hz, 1H, pyr),
8.65 (dd, J = 5Hz, 1H, pyr),
7.87 (broad d, J = 6Hz, 1H, pyr),
7.27 (m, 3H),
6.74 (m, 3H),
4.93 (s, 1H, CHCN),
4.09 (s, 2H, $CH_2CO$),
3.59 (m, 4H, pip),
3.01 (s, 3H, $CH_3N$),
2.58 (m, 4H, pip).

**b) Title compound of the example**
Following the procedure described in example 34b, but using the compound obtained in example 36a, the title compound of this example was obtained as a white solid (54% yield).
m.p.: 136.3-138.5 °C;
IR(KBr) $\nu$ : 3413, 2933, 1649, 1456, 1246 $cm^{-1}$
$^1H$ NMR (80MHz, $D_2O$) $\delta$ (TMS):
8.80 (m, 3H, pyr),
8.18 (m, 1H, pyr),
7.62 (s, 5H, Ph),
5.61 (s, 1H, CHCN),
4.85 (s, 2H, $CH_2CO$),
3.68 (m, 4H, pip),
3.38 (s, 3H, $CH_3N$),
2.55 (m, 4H, pip);
Analysis calcd. for: $C_{20}H_{26}Cl_3N_5O$ . $2H_2O$: C 48.53%, H 6.07%, N 14.16%;
Found: C 48.35%, H 5.60%, N 14.37%.

**EXAMPLE 37**

**1-(*N*-benzylaminoacetyl)-4-(3-pyridylcyanomethyl)piperazine, trihydrochloride**

**a) 1-(N-benzylaminoacetyl)-4-(3-pyridylcyanomethyl)piperazine**

Following a procedure analogous to that described in example 34a, but using benzylamine instead of 1-naphthylamine, the title compound of this example was obtained as a white solid ( 44% yield).
$^1H$ NMR (80MHz, $CDCl_3$) $\delta$ (TMS):
8.77 (d, J = 2.5Hz, 1H, pyr),
8.64 (d, J = 5Hz, 1H, pyr),
7.84 (broad d, J = 7.6Hz, 1H, pyr),
7.30 (m, 6H, 1H, pyr, 5HPh),
4.91 (s, 1H, CHCN),
3.60 (m, 8H, 4H, pip, 2H $CH_2Ph$, 2H $CH_2CO$),
2.52 (m, 4H, pip),
2.17 (broad s, 1H, NH).

**b) Title compound of the example**

Following a procedure analogous to the described in example 34b, but using the compound obtained in example 37a, the title compound of this example was obtained as a white solid ( 45% yield).
mp: 135.9-145.7 ° C;
IR(KBr) $\nu$ : 3440, 1643, 1453, 1250, 753 $cm^{-1}$
$^1H$ NMR (80MHz, $CDCl_3$) $\delta$ (TMS):
9.58 (broad s, 3H),
8.84 (m, 2H, pyr),
8.37 (d, J = 7.5Hz, 1H, pyr),
7.93 (dd, J = 5Hz, J = 7.5Hz, 1H, pyr),
7.45 (m, 5H, Ph),
5.85 (s, 1H, CHCN),
4.10 (m, 4H),
3.43 (m, 4H),
2.51 (m, 5H);
Analysis calcd. for : $C_{20}H_{26}Cl_3N_5O$:
C 52.36%, H 5.71 %, N 15.26%;
Found: C 52.19%, H 5.98%, N 14.66%.

**EXAMPLE 38**

**1-(*N*-benzyl-*N*-phenylaminoacetyl)-4-(3-pyridylcyanomethyl)piperazine.**

Following the procedure described in example 23, but using *N*-benzyl-*N*-phenylaminoacetic acid (Bischoff, Chem.Ber., 1898, 31, 2675) instead of 3,4,5-trimethoxyphenylacetic acid, the title compound of this example was obtained as a white solid ( 22% yield ).
mp: 194.4-196.1 ° C;
IR(KBr) $\nu$ : 3051, 2918, 2226, 1644, 1590, 1503, 1222, 998 $cm^{-1}$
$^1H$ NMR (80MHz, $CDCl_3$) $\delta$ (TMS):
7.90 (m, 1H, pyr),
7.29 (m, 10H, pyr + Ph),
6.74 (m, 3H),
4.93 (s, 1H, CHCN),
4.62 (s, 2H, $CH_2N$),
4.10 (s, 2H, $CH_2CO$),
3.56 (m, 4H, pip),
2.57 (m, 4H, pip).
Analysis calcd. for : $C_{26}H_{27}N_5O$: C 73.39%, H 6.40%, N 16.46%.
Found: C 73.34%, H 6.53%, N 16,17%.

**EXAMPLE 39**

**1-(*N*-(3-phenylpropyl)aminoacetyl)-4-(3-pyridylcyanomethyl)piperazine**

Following the procedure described in example 17, but using 3-phenylpropylamine instead of 1-naphthylamine the title compound of this example was obtained as a white solid (14% yield).
mp: 67.2-68.5 ° C;
IR(KBr) $\nu$ : 3309, 2908, 2811, 2226, 1644, 1421, 1232, 1123, 996 $cm^{-1}$
$^1H$ NMR (80MHz, $CDCl_3$) $\delta$ (TMS):
8.78 (d, J = 2.5Hz, 1H, pyr),
8.64 (dd, J = 2.5Hz, J=5Hz, 1H, pyr),
7.84 (broad d, J = 7.6Hz, 1H, pyr),
7.35 (dd, J = 7.6Hz J = 5Hz, 1H, pyr),
7.20 (m, 5H, Ph),
4.91 (s, 1H, CHCN),
3.63 (m, 4H, pip),
3.40 (s, 2H, $CH_2CO$),
2.57 (m, 8H, pip, $CH_2NH$, $CH_2Ph$),
2.03 (s, 1H, NH),

1.85 (quint, J = 7.6Hz, 2H)
Analysis calcd. for : $C_{22}H_{27}N_5O$: C 70.00%, H 7.21%, N 18.55%;
Found: C 69.99%, H 7.31%, N 18.63%.

**EXAMPLE 40**

**1-(*N*-(3-phenylpropyl)-*N*-methylaminoacetyl)-4-(3-pyridylcyanomethyl) piperazine, trihydrochloride**

**a) 1-(*N*-(3-phenylpropyl)-*N*-methylaminoacetyl)-4-(3-pyridylcyanomethyl)piperazine**

Following the procedure described in example 34a, but using *N*-phenylpropyl-*N*-methylamine instead of 1-naphthylamine the title compound of this example was obtained as a colorless oil ( 75% yield).
$^1$H NMR (80MHz, $CDCl_3$) $\delta$ (TMS):
8.79 (d, J = 2.5Hz, 1H, pyr),
8.65 (dd, J = 2.5Hz, J = 5Hz, 1H, pyr),
7.86 (broad d, J = 7.6Hz, 1H, pyr),
7.27 (m, 6H),
4.91 (s, 1H, CHCN),
3.66 (m, 4H, pip),
3.11 (s, 2H, $CH_2CO$),
2.58 (m, 8H),
2.26 (s, 3H, $CH_3N$),
1.82 (m, 2H).

**b)Title compound of the example**

Following the procedure described in example 34b, but using the compound obtained in example 23a, the title compound of this example was obtained as a white solid (35% yield).
mp: 121.3 - 131.0° C;
IR(KBr) $\nu$ : 3415, 2943, 2825, 1649, 1462, 1248, 997 $cm^{-1}$;
$^1$H NMR (80MHz, $D_2O$) $\delta$ (TMS):
8.70 (m, 2H, pyr),
8.15 (m, 1H, pyr),
7.69 (m, 1H, pyr),
7.33 (s, 5H, Ph),
5.40 (s, 1H, CHCN),
4.23 (s, 2H, $CH_2CO$),
3.7-3.1 (m, 8H),
2.94 (s, 3H, $CH_3N$),
2.72 (m, 4H),
2.10 (m, 2H);
Analysis calcd. for : $C_{23}H_{32}Cl_3N_5O$: C 55.14%, H 6.39%, N 13.99%;
Found: C 55.52%, H 7.07%, N 13.65%.

**EXAMPLE 41**

**1-(*N*-benzyl-*N*-(3-phenylpropyl)aminoacetyl)-4-(3-pyridylcyanomethyl) piperazine, trihydrochloride**

**a) 1-(*N*-benzyl-*N*-(3-phenylpropyl)aminoacetyl)-4-(3-pyridylcyanomethyl)piperazine**

To a mixture of 0.34 g (1 mmol) of the product obtained in example 39 in 3 mL methanol, was added 1 drop of hydrochloric acid solution in methanol , maintaining the pH range between 6 and 8. Then0.106 g (1 mmol) of benzaldehyde and 0.063 g (1 mmol) of $NaBH_3CN$ were added and the mixture stirred at room temperature one night. The solvent was removed and the residue dissolved in $CH_2Cl_2$. The solution was washed with water and dried over $Na_2SO_4$, affording 0.384 g of a crude that was chromatographed on silica gel eluting with increasing polarity hexane-ethyl acetate mixtures. 0.10 g of a colorless oil were obtained. (22% yield).
$^1$H NMR (80MHz, $CDCl_3$) $\delta$ (TMS):

8.77 (m, 1H, pyr),
8.65 (d, J = 5Hz, 1H, pyr),
7.28 (m, 12H),
4.87 (s, 1H, CHCN),
3.61 (s, 2H),
3.51 (m, 4H, pip),
3.25 (s, 2H),
2.60 (m, 8H),
1.88 (m, 2H).

**b) Title compound of the example**

Following the procedure described in example 34b, but using the compound obtained in example 41a, the title compound of this example was obtained as a white solid (quant. yield).
mp: 119.6 - 122.3° C;
IR(KBr) $\nu$ : 3414, 3056, 2830, 1649,1454, 1249, 1000, 752, 702 $cm^{-1}$
Analysis calcd. for : $C_{29}H_{36}Cl_3N_5O$: C 60.37%, H 6.29%, N 12.14%.
Found: C 60.09%, H 6.38%, N 12.16%.

**EXAMPLE 42**

**1-(*N*-(1-phenylethyl)aminoacetyl)-4-(3-pyridylcyanomethyl)piperazine, trihydrochloride**

**a) 1-(*N*-(1-phenylethyl)aminoacetyl)-4-(3-pyridylcyanomethyl)piperazine**

Following the procedure described in example 34a, but using 1-phenylethylamine instead of naphthylamine the title compound of this example was obtained as a colorless oil ( 47% yield).
$^1H$ NMR (80MHz, $CDCl_3$) $\delta$ (TMS):
8.76 (d, J = 2.5Hz, 1H, pyr),
8.63 (dd, J = 2.5Hz, J = 5Hz, 1H, pyr).
7.83 (broad d, J = 7.6 Hz, 1H, pyr),
7.30 (m, 6H),
4.91 (s, 1H, CHCN),
3.71 (m, 5H, pip + CHPh),
3.25 (s, 2H, $CH_2CO$),
2.51 (m, 5H, pip + NH),
1.37 (d, J = 7Hz, 3H, $CH_3$).

**b) Title compound of the example**

Following the procedure described in example 34b, but using the product obtained in example 42a the title compound of this example was obtained as a white solid (40% yield).
mp: 106.9 - 116.4° C;
IR(KBr) $\nu$ : 3600-2500, 1649, 1546, 1454, 1248, 1141, 1000 $cm^{-1}$
$^1H$ NMR (80MHz, $D_2O$) $\delta$ (TMS):
8.82 (m, 1H, pyr),
8.36 (m, 1H, pyr),
7.87 (m, 1H, pyr),
7.52 (m, 6H),
5.48 (s, 1H, CHCN),
4.75 (m, 6H),
3.95 (m, 1H),
3.45 (m, 4H).
2.60 (m, 4H),
1.75 (m, 3H);
Analysis calcd. for : $C_{21}H_{28}Cl_3N_5O$ . 1.5 $H_2O$:
C 50.45%, H 6.21%, N 14.01%;
Found: C 50.70%, H 5.92%, N 13.52%.

**EXAMPLE 43**

**1-(*N*-(1-phenylethyl)-*N*-methylaminoacetyl)-4-(3-pyridylcyanomethyl)-piperazine, trihydrochloride.**

**a) 1-(*N*-(1-phenylethyl)-*N*-methylaminoacetyl)-4-(3-pyridylcyanomethyl) piperazine**

Following the procedure described in example 34a, but using *N*-(1-phenylethyl)methylamine instead of 1-naphthylamine the title compound of this example was obtained as a colorless oil (78% yield).
IR(film) ν : 2967, 2824, 2311, 1636, 1448, 1419, 1275, 1142, 1000 $cm^{-1}$
$^{1}H$ NMR (80MHz, $CDCl_3$) δ (TMS):
8.79 (d, J = 2.5Hz 1H, pyr),
8.65 (dd, J = 5Hz, J = 2.5Hz, 1H, pyr),
7.86 (broad d, J = 7.6 Hz, 1H, pyr),
7.42 (m, 1H, pyr),
7.28 (s, 5H, Ph),
4.91 (s, 1H, CHCN),
3.58 (m, 4H, pip),
3.15 (s, 2H, $CH_2CO$),
2.53 (m, 4H, pip),
2.26 (s, 3H, $CH_3N$),
2.1 (broad s, 1H, CHN),
1.37 (d, J = 7Hz, 3H, $CH_3$).

**b) Title compound of the example**

Following the procedure described in example 34b, but using the product obtained in example 26a, the title compound of this example was obtained as a white solid (44% yield).
mp: 135.0-137.8° C;
IR(KBr) ν :
3600-2000, 1649, 1453, 1246, 1139, 1057 $cm^{-1}$
$^{1}H$ NMR (80MHz, $D_2O$) δ (TMS):
8.90 (m, 3H, pyr),
8.20 (dd, J = 7.6 Hz, J = 5Hz, 1H, pyr),
7.56 (s, 5H, Ph),
5.63 (s, 1H, CHCN),
4.8-4.0 (m, 3H),
3:49 (m, 4H, pip),
3.00 (broad s, 3H, $CH_3N$),
2.56 (m, 4H, pip),
1.79 (d, J = 7Hz, 3H, $CH_3$);
Analysis calcd. for: $C_{22}H_{30}Cl_3N_5O$ . $2H_2O$:
C 50.53%, H 6.55%, N 13.39%;
Found: C 50.76%, H 6.18%, N 13.37%.

**EXAMPLE 44**

**1-(*N*-(diphenylmethyl)aminoacetyl)-4-(3-pyridylcyanomethyl) piperazine, trihydrochloride**

**a) 1-(*N*-(diphenylmethyl)aminoacetyl)-4-(3-pyridylcyanomethyl) piperazine**

Following the procedure described in example 23, but using *N*-(diphenylmethyl)aminoacetic acid (H. Nagatomi et al., Chem. Pharm. Bull, 1979, 27, 1021), instead of 3,4,5-trimethoxyphenylacetic acid the title compound of the example was obtained as a colorless oil (92% yield).
IR(film) ν : 3315, 3052, 3019, 2911, 8220, 2310, 1643, 1419, 1235, 748, 706 $cm^{-1}$
$^{1}H$ NMR (80MHz, $CDCl_3$) δ (TMS):
8.75 (m, 1H, pyr),
8.64 (broad d, J = 5Hz, 1H, pyr),
7.83 (d, J = 7.6Hz, 1H, pyr),

7.30 (m, 11H),
4.88 (s, 1H),
4.84 (s, 1H),
3.65 (m, 2H, pip),
3.36 (s, 2H, $CH_2CO$),
3.30 (m, 2H, pip),
2.48 (m, 5H).

**b) Title compound of the example**

Following the procedure described in example 34b, but using the compound prepared in example 44a, the title compound of this example was obtained as a white solid (67% yield).
mp: 165.0-168.2° C;
IR(KBr) $\nu$ : 3600-2400, 1649, 1543, 1449, 1427, 1245, 1000 $cm^{-1}$
$^1H$ NMR (80MHz, $D_2O$) $\delta$ (TMS):
8.87 (m, 3H, pyr),
8.14 (dd, J = 7.6Hz, J = 5Hz, 1H, pyr),
7.52 (s, 10H),
5.64 (s, 1H),
5.58 (s, 1H),
4.10 (s, 2H, $CH_2CO$),
3.59 (m, 2H, pip),
3.38 (m, 2H, pip),
2.60 (m, 4H, pip).
Analysis calcd. for : $C_{26}H_{30}N_5OCl_3$ . $2H_2O$: C 54.79%, H 5.96%, N 12.27%.
Found: C 55.14%, H 5.58%, N 11.88%.

**EXAMPLE 45**

**1-(*N*-diphenylmethyl-*N*-methylaminoacetyl)-4-(3-pyridylcyanomethyl) piperazine, trihydrochloride.**

**a) 1-(*N*-diphenylmethyl-*N*-methylaminoacetyl)-4-(3-pyridylcyanomethyl) piperazine**

Following the procedure described in example 23 but using *N*-diphenylmethyl-*N*-methylaminoacetic acid obtained in preparation 7 instead of 3,4,5-trimethoxyphenylacetic acid, the title compound of this example was obtained as a colorless oil (100% yield ).

**b) Title compound of the example**

Following a procedure analogous to that described in example 34b, but using the compound prepared in example 45a, the title compound of this example was obtained as a white solid (46% yield)
mp: 135.0 - 137.7° C;
IR(KBr) $\nu$ : 3600-2400, 1649, 1449, 1242, 1000 $cm^{-1}$;
$^1H$ NMR (80MHz, $D_2O$) $\delta$ (TMS):
8.90 (m, 3H, pyr),
8.26 (m, 1H, pyr),
7.56 (m, 10H, Ph)
5.73 (s, 1H),
5.58 (s, 1H),
4.35 (s, 2H, $CH_2CO$),
3.43 (m, 4H, pip).
3.03 (s, 3H, $CH_3N$),
2.48 (m, 4H, pip);
Analysis calcd. for : $C_{27}H_{32}Cl_3N_5O$ . $1H_2O$: C 57.09%, H 6.00%, N 12.36%;
Found: C 57.38%, H 6.16%, N 12.22%.

**EXAMPLE 46**

**1-(*N*-(1-(4-chlorophenyl)-1-phenylmethyl)aminoacetyl)-4-(3-pyridylcyanomethyl) piperazine, trihydrochloride.**

**a) 1-(*N*-(1-(4-chlorophenyl)-1-phenylmethyl)aminoacetyl)-4-(3-pyridylcyanomethyl) piperazine**

Following the procedure described in example 23 but using *N*-(1-(4-chlorophenyl)-1-phenylmethyl)-aminoacetic acid (H. Nagatomi et al., Chem, Pharm. Bull, 1979, 27, 1921) instead of 3,4,5-trimethoxyphenylacetic acid, the title compound of this example was obtained as a colorless oil (96% yield).
IR(film)ν: 3600-3100, 3021, 2921, 2846, 2114, 1666, 1481, 1421, 1238, 1000 $cm^{-1}$;
$^1H$ NMR (80MHz, $CDCl_3$) δ (TMS):
8.80 (d, J = 2.5Hz, 1H, pyr),
8.67 (d, J = 5Hz, 1H, pyr),
8.03 (s, broad, 1H, NH),
7.88 (broad d, J = 7.6 Hz, 1H, pyr),
7.35 (m, 10H, pyr, Ph),
4.95 (s, 1H),
4.85 (s, 1H).
3.67 (m, 2H, pip),
3.39 (m, 2H, pip),
3.39 (s, 2H, $CH_2CO$),
2.53 (m, 4H, pip);

**b) Title compound of the example**

Following the procedure described in example 34b, but using the compound prepared in example 46a, the title compound of this example was obtained as a white solid (yield 51%)
mp: 124.0-135.3 °C;
IR(KBr) ν : 3600-2000, 1649, 1546, 1427, 1243, 1091, 1000 $cm^{-1}$;
$^1H$ NMR (80MHz, $D_2O$) δ (TMS):
9.17 (m, 3H, pyr),
8,45 (m, 1H, pyr),
7.69 (m, 9H, Ph),
5.89 (s, 2H, CHCN, $CHAr_2$),
4.35 (s, 2H, $CH_2CO$),
4.0-3.5 (m, 4H, pip),
2.82 (m, 4H, pip);
Analysis calcd. for : $C_{26}H_{29}Cl_4N_5O$ . $1H_2O$:
C 53.17%, H 5.32%, N 12.03%;
Found: C 52.80%, H 5.32%, N 11.92%.

**EXAMPLE 47**

**1-(3-(*N*-(diphenylmethyl)amino)propanoyl)-4-(3-pyridylcyanomethyl) piperazine**

Following a procedure analogous to that described in example 23, but using 3-*N*-(diphenylmethyl)-aminopropanoic acid (H. Nagatomi et al., Chem. Pharm. Bull, 1979, 27, 1021) instead of 3,4,5-trimethoxyphenylacetic acid the title compound of this example was obtained as a white solid (58% yield)
mp: 162.0-163.5 ° C;
IR(KBr) ν : 3273, 3052, 3015, 2935, 2845, 1636, 1593, 1448, 1441, 1326, 1283, 1224, 1134, 1002 $cm^{-1}$;
$^1H$ NMR (80MHz, $D_2O$) δ (TMS):
8.78 (d, J = 7.5Hz, 1H, pyr),
8.65 (dd, J = 2.5Hz, J = 5Hz, 1H, pyr),
7.84 (broad d, J = 7.6Hz, 1H, pyr),
7.30 (m, 11H, 1pyr, Ph),
4.89 (s, 1H),
4.84 (s, 1H),
3.53 (m, 4H, pip),
2.86 (t, J = 7Hz, 2H, $CH_2NH$),

2.54 (m, 6H),
2.28 (broad s, 1H, NH);
Analysis calcd. for: $C_{27}H_{29}N_5O$: C 72.16 %, H 6.46%, N 15.59 %.
Found: C 72.50%, H 6.48%, N 15.13%.

**EXAMPLE 48**

**1-(3-(*N*-(diphenylmethyl)amino)propanoyl)-4-(4-pyridylcyanomethyl)piperazine**

To a solution of 5.1 g (20 mmol) of 3-(*N* -(diphenylmethyl)amino)propanoic acid and 2.3 g (20 mmol) of hydroxysuccinimide in 120 mL anhydrous dimethoxyethane, cooled with an ice bath and under argon atmosphere, were added 4.1 g (20 mmol) of dicyclohexylcarbodiimide. The mixture was stirred 18 h at 4° C. The precipitate was removed, and 4.3 g (21 mmol) of the compound obtained in preparation 2 in 60 mL dimethoxyethane were added to the resulting solution. Then, it was stirred 2 h at room temperature and half an hour at 60° C. The solvents were removed and it was worked up with ethyl acetate and saturated sodium bicarbonate solution. The organic solution was separated and dried. The solvents were removed yielding 10 g of crude that were purified by chromatography on silica gel (chloroform, methanol, ammonia, 60:2:0.2). The obtained product was recrystallized in a mixture of methylene chloride and diethyl ether affording 1.52 g of a white solid (17% yield)
mp: 145.6 - 148.7° C;
IR(KBr) $\nu$: 3313, 3020, 2908, 1636, 1592, 1447 $cm^{-1}$;
$^1H$ NMR (80MHz, $CDCl_3$) $\delta$ (TMS):
8.69 (broad d, J = 6.4Hz, 2H, pyr),
7.35 (m, 12H, pyr + Ar),
4.86 (s, 2H, CHCN, CHNH),
3.51 (m, 4H, pip, 1/3 $(CH_3CH_2)_2O$)
288 (t, J = 5.6Hz, 2H, $CH_2CO$),
2.54 (m, 7H pip, NH, $CH_2N$),
1.19 (t, 1/3 $(CH_3CH_2)_2O$),
Analysis calcd. for : $C_{27}H_{29}N_5O$.1/3 $Et_2O$: C 73.30%, H 7.23%, N 15.08%;
Found: C 73.22%, H 6.95%, N 14.98%.

**EXAMPLE 49**

**1-(*N,N*-dibenzylaminoacetyl)-4-(3-pyridylcyanomethyl)piperazine, trihydrochloride**

**a) 1-(*N,N*-dibenzylaminoacetyl)-4-(3-pyridylcyanomethyl)piperazine**

Following a procedure analogous to that of example 34a, but using dibenzylamine instead of 1-naphthylamine the title compound of this example was obtained as a colorless oil ( 22% yield).
$^1H$ NMR (80MHz,$CDCl_3$) $\delta$ (TMS):
8.70 (m, 2H, pyr),
7.84 (broad d, J = 7Hz, 1H, pyr),
7.31 (m, 11H, 1Hpyr, 10H Ph),
4.87 (s, 1H, CHCN),
3.64 (s, 4H, $CH_2Ph$),
3.7-3.1 (m, 4H, pip),
3.22 (s, 2H, $CH_2CO$),
2.47 (m, 4H, pip).

**b) Title compound of the example**

Following a procedure analogous to that described in example 34b, but starting with the product obtained in example 49a the title compound of this example was obtained as a white solid (100% yield).
mp: 126.7-132.4° C;
IR(KBr) $\nu$ : 3600-2400, 1649, 1453, 1248, 1142, 1001 $cm^{-1}$;
$^1H$ NMR (80MHz, $D_2O$) $\delta$ (TMS):
8.88 (m, 3H, pyr),

8.19 (m, 1H, pyr),
7.57 (m, 10H, Ph),
5.59 (s, 1H, CHCN),
4.65 (broad s, 4H, $CH_2Ph$),
4.10 (s, 2H, $CH_2CO$),
3.30 (m, 4H, pip),
3.54 (m, 4H, pip).
Analysis calcd. for : $C_{27}H_{32}Cl_3N_5O.1.5H_2O$: C 56.29%, H 6.08%, N 12.16%;
Found: C 56.33%, H 6.07%, N 11.78%.

**EXAMPLE 50**

**1-(*N*-(2,2-diphenylethyl)aminoacetyl)-4-(3-pyridylcyanomethyl)piperazine, trihydrochloride**

**a) 1-(*N*-(2,2-diphenylethyl)aminoacetyl)-4-(3-pyridylcyanomethyl) piperazine**

Following a procedure analogous to that described in example 34a, but using 2,2-diphenylethylamine instead of 1-naphthylamine the title compound of this example was obtained as a colorless oil (65% yield).
$^1H$ NMR (80MHz, $CDCl_3$) $\delta$ (TMS):
8.77 (d, J = 2.5Hz, 1H, pyr),
8.64 (dd, J = 2.5Hz, J = 5Hz, 1H, pyr),
7.83 (broad d, J = 7.6Hz, 1H, pyr),
7.30 (m, 11H, 1H pyr, 10H Ph),
4.88 (s, 1H, CHCN),
4.17 (t, J = 7Hz, 2H, $CH_2NH$),
3.41 (m, 7H),
2.52 (m, 4H, pip),
1.72 (s, 1H, NH).

**b) Title compound of the example**

Following a procedure analogous to that described in example 34b, but using the compound synthetized in example 50a, the title compound of this example was obtained as a white solid (58% yield).
mp: 181.0 - 192.1 °C;
IR(KBr) $\nu$ : 3600-2400, 1649, 1487, 1448, 1248, 1000 $cm^{-1}$;
$^1H$ NMR (80MHz, $D_2O$) $\delta$ (TMS):
8.88 (m, 3H, pyr),
8.17 (dd, J = 7.6Hz, J = 5Hz, 1H pyr),
7.42 (m, 10H, Ph),
5.60 (s, 1H, CHCN),
4.50 (m, 2H),
4.13 (s, 2H, $CH_2CO$),
3.9-3.3 (m, 6H),
2.67 (m, 4H);
Analysis calcd. for: $C_{27}H_{32}Cl_3N_5O$ . $1H_2O$: C 57.19%, H 6.00%, N 12.36%;
Found: C 56.91%, H 5.89%, N 11.95%.

**EXAMPLE 51**

**1-(*N*-(3,3-diphenylpropyl)aminoacetyl)-4-(3-pyridylcyanomethyl)-piperazine, trihydrochloride**

**a) 1-(*N*-(3,3-diphenylpropyl)aminoacetyl)-4-(3-pyridylcyanomethyl)-piperazine**

Following a procedure analogous to that described in example 34a, but using 3,3-diphenylpropylamine instead of 1-naphthylamine the title compound of this example was obtained as a colorless oil (71% yield).
IR(film) $\nu$ : 3600-3200.3019, 2919, 2226, 1642, 1488, 1421, 1272, 1237, 1140, 1000, 752, 705 $cm^{-1}$;
$^1H$ NMR (80MHz, $CDCL_3$) $\delta$ (TMS):
8.77 (d, J = 2.5Hz, 1H, pyr),

8.65 (dd, J = 5Hz, J = 2.5Hz, 1H, pyr),
7.85 (broad d, J = 7.6Hz, 1H, pyr),
7.28 (m, 11H),
4.90 (s, 1H, CHCN),
4.04 (t, J = 7Hz, 1H, $CHPh_2$),
3.7-3.3 (m, 4H, pip),
3.36 (s, 2H, $CH_2CO$),
2.55 (m, 6H),
2.6-2.0 (m, 3H);

**b) Title compound of the example**

Following a procedure analogous to that described in example 34b, but using the compound synthetized in example 51a, the title compound of this example was obtained as a white solid (51% yield).
mp: 171.7-176.8 ° C;
IR(KBr) $\nu$ : 3600-2400, 1649, 1447, 1246, 1000 $cm^{-1}$;
$^1H$ NMR (80MHz, $D_2O$) $\delta$ (TMS):
8.90 (m, 3H, pyr),
8.19 (dd, J = 7.6Hz, J = 5Hz, 1H, pyr),
7.39 (m, 10H),
5.62 (s, 1H, CHCN),
4.16 (t, J = 7Hz, 1H),
4.01 (s, 2H, $CH_2CO$),
3.50 (m, 4H),
3.0 (m, 2H),
2.67 (m, 6H);
Analysis calcd. for : $C_{28}H_{34}Cl_3N_5O.0.5H_2O$: C 58.79%, H 6.12%, N 12.25%.
Found: C 58.49%, H 5.93%, N 12.20%.

**EXAMPLE 52**

**1-(*N*-(1,2-diphenylethyl)aminoacetyl)-4-(3-pyridylcyanomethyl)piperazine, trihydrochloride**

**a) 1-(*N*-(1,2-diphenylethyl)aminoacetyl)-4-(3-pyridylcyanomethyl) piperazine.**

Following a procedure analogous to that described in example 34a, but using 1,2-diphenylethylamine instead of 1-naphthylamine the title compound of this example was obtained as a colorless oil (43% yield).
$^1H$ NMR (80MHz, $CDCl_3$) $\delta$ (TMS):
8.71 (d, J = 2.5Hz, 1H, pyr),
8.65 (dd, J = 5Hz, J = 2.5 Hz, 1H, pyr),
7.85 (broad d, J =7.6Hz, 1H, pyr),
7.20 (m, 11H),
4.96 (s, 1H, CHCN),
3.97 (m, 2H, NH, CHPh),
3.56 (m, 2H, pip),
3.18 (m, 2H, pip),
3.15 (s, 2H, $CH_2CO$),
2.94 (d, J = 8Hz, 2H, $CH_2Ph$),
2.46 (m, 4H, pip);

**b) Title compound of the example**

Following a procedure analogous to that described in example 34b, but using the compound prepared in example 52a, the title compound of this example was obtained as a white solid (72% yield).
mp: 162.5-171.0 ° C;
IR(KBr) $\nu$ : 3600-2400, 1649, 1449, 1245, 1140, 1000 $cm^{-1}$;
$^1H$ NMR (80MHz, $D_2O$) $\delta$ (TMS):
8.88 (m, 3H, pyr),

8.17 (m, 1H, pyr),
7.36 (m, 10H, Ph),
5.60 (s, 1H, CHCN),
4.67 (t, J = 8Hz, 1H, CHPh),
3.95 (s, 2H, $CH_2CO$),
3.50 (m, 6H),
2.59 (m, 4H, pip);
Analysis calcd. for : $C_{27}H_{32}Cl_3N_5O$ . $2H_2O$: C 55.43%, H 6.16%, N 11.98%;
Found: C 55.65%, H 5.99%, N 11.52%.

**EXAMPLE 53**

**1-(1-(4-(diphenylmethylpiperazinyl)acetyl)-4-(3-pyridylcyanomethyl) piperazine, tetrahydrochloride**

**a) 1-(1-(4-diphenylmethylpiperazinyl)acetyl)-4-(3-pyridylcyanomethyl) piperazine**

Following a procedure analogous to that described in example 34a, but using 1-diphenylmethylpiperazine instead of 1-naphthylamine the title compound of this example was obtained as a colorless oil (87% yield).
$^1$H NMR (80MHz, $CDCl_3$) $\delta$ (TMS):
8.77 (d, J = 2.5Hz, 1H, pyr),
8.63 (dd, J = 5Hz, J = 2.5Hz, 1H, pyr),
7.32 (m, 12H),
4.90 (s, 1H, CHCN),
4.25 (s, 1H, $CHPh_2$),
3.63 (m, 4H, pip).
3.19 (s, 2H, $CH_2CO$),
2.58 (m, 12H, pip).

**b) Title compound of the example**

Following a procedure analogous to that described in example 34b, but using the compound prepared in example 53a, the title compound of this example was obtained as a white solid (44% yield).
mp: 149.8-156.1 °C;
IR(KBr) $\nu$ : 3600-2400, 1649, 1449, 1427, 1246, 1024 $cm^{-1}$;
$^1$H NMR (80MHz, $D_2O$) $\delta$ (TMS):
8.88 (m, 3H, pyr),
8.17 (m, 1H, pyr),
7.51 (m, 10H, Ph),
5.63 (s, 1H, CHCN),
5.14 (s, 1H, $CHPh_2$),
4.46 (s, 2H, $CH_2CO$),
3.53 (m, 12H, pip),
2.72 (m, 4H, pip);
Analysis calcd. for : $C_{30}H_{38}Cl_4N_6O$ . $1.5H_2O$: C 53.97%, H 6.15%, N 12.59%;
Found: C 53,85%, H 6.26%, N 12,76%.

**EXAMPLE 54**

**1-(*N*-methyl-*N*-cyclohexylaminoacetyl)-4-(3-pyridylcyanomethyl) piperazine trihydrochloride**

**a) 1-(*N*-methyl-*N*-cyclohexylaminoacetyl)-4-(3-pyridylcyanomethyl) piperazine**

Following the procedure described in example 34a, but using *N*-methylcyclohexylamine instead of 1-naphthylamine, the title compound of this example was obtained as a colorless oil (45% yield).
IR(film) $\nu$ : 3441, 2925, 2229, 1632, 1446, 1420 $cm^{-1}$;
$^1$H NMR (80MHz, $D_2O$) $\delta$ (TMS):
8.85 (m, 1H, pyr),

8.68 (broad d, J = 4,8Hz, 1H, pyr),
7.90 (broad d, J = 7.8Hz, 1H, pyr),
7.35 (dd, $J_a$ = 4.8Hz, $J_b$ = 7.8Hz, 1H, pyr),
4.95 (s, 1H, CHCN),
3.68 (m, 4H, pip),
3.27 (s, 2H, $CH_2CO$),
2.58 (t, J = 4.8Hz, 4H, pip),
2.40 (m, 1H, $CHNCH_3$),
2.25 (s, 3H, $CH_3N$),
1.50 (complex signal, 10H).

**b) Title compound of the example**

Following the procedure described in example 19b, but using the compound prepared in example 54a, the title compound of this example was obtained as a white solid in a similar yield.
mp: 41.7-41.7° C;
IR(KBr) ν : 3417, 2937, 2857, 1649, 1248 $cm^{-1}$;
Analysis calcd. for : $C_{20}H_{29}N_5O$. $3HCl.H_2O$: C 49.75%, H 7.10%, N 14.50%;
Found: C 49.89%, H 6.93%, N 14.02%

**EXAMPLE 55**

**1-(1,2,3,4-Tetrahydroisoquinol-2-ylacetyl)-4-(3-pyridylcyanomethyl) piperazine, trihydrochloride**

**a) 1-(1,2,3,4-Tetrahydroisoquinol-2-ylacetyl)-4-(3-pyridylcyanomethyl) piperazine**

Following a procedure analogous to that described in example 34a, but using 1,2,3,4-tetrahydroisoquinoline instead of 1-naphthylamine, the title compound of this example was obtained as a colorless oil (79% yield).
$^1H$ NMR (80MHz, $CDCl_3$) δ (TMS):
8.77(d, J = 2.5Hz, 1H, pyr),
8.63 (dd, J = 2.5Hz, J = 5Hz, 1H, pyr),
7.84 (broad d, J = 7.6Hz, 1H, pyr),
7.4-6.9 (m, 5H),
4.91 (s, 1H, CHCN),
3.68 (m, 6H),
3.38 (s, 2H, $CH_2CO$),
3.0-2.4 (m, 8H).

**b) Title compound of the example**

Following a procedure analogous to that described in example 34b, but using the compound prepared in example 55a, the title compound of this example was obtained as a white solid (65% yield).
mp: 172.7-189.0° C;
IR(KBr) ν : 3600-3200, 1649, 1454, 1367, 1245, 1140, 1000 $cm^{-1}$;
$^1H$ NMR (80MHz, $D_2O$) δ (TMS):
8.87 (m, 2H, pyr),
8.44 (m, 1H, pyr),
8.98 (m, 1H, pyr),
7.25 (s, 4H, Ar),
5.88 (s, 1H, CHCN),
4.54 (broad s, 4H),
3.8-3.0 (m, 6H),
2.51 (m, 6H);
Analysis calcd. for : $C_{22}H_{28}Cl_3N_5O.1.5H_2O$: C 51.61%, H 6.06%, N 13.68%;
Found: C 51.37%, H 5.66%, N 13.29%.

**EXAMPLE 56**

**1-(*N*-benzoylaminoacetyl)-4-(3-pyridylcyanomethyl)piperazine, dihydrochloride**

**a) 1-(*N*-benzoylaminoacetyl)-4-(3-pyridylcyanomethyl)piperazine**

Following the procedure described in example 23, but using *N*-benzoylaminoacetic acid instead of 3,4,5-trimethoxyphenylacetic acid, the title compound of this example was obtained as a colorless oil (47% yield).
$^1H$ NMR (80MHz, $CDCl_3$) $\delta$ (TMS):
8.76 (m, 1H, pyr)
8.63 (broad d, J = 4.0Hz, 1H, pyr),
7.84 (m, 3H),
7,48 (m, 5H),
5.28 (s, 1H, CHCN),
4.24 (d, J = 3.8Hz, 2H, $NHCH_2CO$),
3.60 (m, 4H, pip),
2.58 (m, 4H, pip).

**b) Title compound of the example**

Following the procedure described in example 19b, but using the compound prepared in example 56a, the title compound of this example was obtained as a white solid (57% yield).
mp: 34.1 -41.9° C;
IR(KBr) $\nu$ : 3391, 1649, 1536, 1462, 1305, 1240 $cm^{-1}$;
$^1H$ NMR (80MHz, $DMSOd_6$) $\delta$ ppm(TMS):
8.88 (m, 2H, pyr),
8.43 (m, 2H),
7.80 (complex signal, 5H),
5.79 (s, 1H, CHCN),
4.16 (d, 2H, J = 4.8Hz, $CH_2CO$),
3.56 (m, 4H, pip),
2.50 (m, 4H, pip),
Analysis calcd. for : $C_{20}H_{21}O_2N_5$ . 2HCl . $1H_2O$:
C 52.87%, H 5.54%, N 15.41%;
Found: C 53.04%, H 5.16%, N 14.95%.

**EXAMPLE 57**

**1-(*N*-(diphenylacetyl)aminoacetyl)-4-(4-pyridylcyanomethyl)piperazine**

Following the procedure described in example 23, but using N-(diphenylacetyl)aminoacetic acid instead 3,4,5-trimethoxyphenylacetic acid, the title compound of this example was obtained as a white solid (47% yield).
mp: 80.1 - 109.4° C;
IR(KBr) $\nu$ : 3313, 3021, 2915, 1641, 1593, 1432 $cm^{-1}$;
$^1H$ NMR (80MHz, $CDCl_3$) $\delta$ (TMS):
8.67 (m, 2H, pyr),
7.45 (m, 2H, pyr),
7.28 (s, 10H, Ph),
6.85 (m, 1H, CONH),
4.99 (s, 1H, CHCN),
4,87 (s, 1H, $Ph_2CH$),
4.08 (d, J = 3.2Hz, 2H, $CH_2CO$),
3.62 (m, 2H, pip),
3.41 (m, 2H, pip),
2.51 (t, J =5.6Hz, 4H, pip);
Analysis calcd. for : $C_{27}H_{27}N_5O_2$ . $H_2O$:
C 68.77%, H 6.20%, N 14.85%;
Found: C 68.89%, H 5.64%, N 13.69%.

**EXAMPLE 58**

**1-(3-Phenylpropyl)-4-(3-pyridylcyanomethyl)piperazine, trihydrochloride**

**a) 1-(3-phenylpropyl)-4-(3-pyridylcyanomethyl)piperazine**

Following the procedure described in preparation 1, but using 1-(3-phenylpropyl)piperazine instead of piperazine in a 1:1 molar ratio with pyridine-3-carboxaldehyde, the title compound of this example was obtained as a colorless oil (40% yield).
IR(film) $\nu$ : 2939, 2815, 1587, 1572, 1448, 1418, 1148 $cm^{-1}$;
$^{1}H$ NMR (80MHz, $CDCl_3$) $\delta$ (TMS):
8.76 (d, J = 2.4Hz, 1H, pyr),
8.60 (dd, $J_a$ = 4.8Hz, $J_b$ = 1.4Hz, 1H, pyr),
8.84 (broad d, J =7.9Hz, 1H, pyr),
7.32 (dd, $J_a$ = 8.0Hz, $J_b$ = 4.6Hz, 1H, pyr),
7.20 (s, 5H, Ph),
4.85 (s, 1H, CHCN),
2.54 (m, 12H),
1.77 (m, 2H).

**b) Title compound of the example**

Following the procedure described in example 19b, but starting with the compound prepared in example 58a, the title compound of this example was obtained as a white solid (84%yield).
mp: 31.6-37.2° C;
IR(KBr) $\nu$: 3415, 2927, 1625, 1625, 1546, 1461 $cm^{-1}$;
Analysis calcd. for: $C_{20}H_{24}N_4$ . 3 HCl: C 55.88%, H 6.33%, N 13.03%;
Found: C 56.25%, H 6.63%, N 12.92%.

**EXAMPLE 59**

**1-(3,3-diphenylpropyl)-4-(3-pyridylcyanomethyl)piperazine, trihydrochloride**

**a) 1-(3,3-diphenylpropyl)-4-(3-pyridylcyanomethyl)piperazine**

Following the procedure described in preparation 1 but using 1-(3,3-diphenylpropyl)piperazine instead of piperazine in a 1:1 molar ratio with pyridine-3-carboxaldehyde, the title compound of this example was obtained as a colorless oil (51% yield).
IR(film) $\nu$ : 3021, 2939, 1586, 1488, 1447, 1419 $cm^{-1}$;
$^{1}H$ NMR (80MHz, $CDCl_3$) $\delta$ (TMS):
8.74 (d, J = 2.1Hz, 1H, pyr),
8.59 (dd, $J_a$ = 4.8Hz, $J_b$ = 1.4Hz, 1H, pyr),
7.84 (broad d, J = 7.9Hz, 1H, pyr), 7.23 (m, 11H, pyr + Ar),
4.84 (s, 1H, CHCN),
4.00 (m, 1H, $Ph_2CH$),
2.40 (complex signal, 12H).

**b) Title compound of the example**

Following the procedure described in example 19b, but using the compound prepared in example 59a, the title compound of this example was obtained as a white solid (78% yield).
p.f: 88.5-140.8° C
Analysis calcd. for: $C_{26}H_{28}N_4$.3HCl.1/4$H_2O$: C 61.18%, H 6.22%, N 10.97%;
Found: C 61.18%, H 6.27%, N 10.93%.

**EXAMPLE 60**

**1-diphenylmethyl-4-(3-pyridylcyanomethyl)piperazine**

Following the procedure described in preparation 1, but using 1-diphenylmethylpiperazine instead of piperazine in a 1:1 molar ratio with pyridine-3-carboxaldehyde, the title compound of this example was obtained as a white solid (82%yield).
mp: 151.6-151.8 ° C;
IR(KBr) ν :
3023, 2950, 1590, 1576, 1485, 1447 $cm^{-1}$;
$^1H$ NMR (80MHz $D_2O$) δ (TMS):
8.77 (m, 2H, pyr),
7.84 (broad d, J = 8.0Hz, 1H, pyr),
7.30 (m, 11H, pyr + Ar),
4.86 (s, 1H, CHCN),
3.94 (s, 1H, $CHPh_2$),
2.40 (m, 8H, pip)
Analysis calcd. for : $C_{24}H_{24}N_4$: C 78.23%, H 6.56%, N 15.20%;
Found: C 78.15%, H 6.73%, N 15.40%.

**EXAMPLE 61**

**1-(2-(*N*-(diphenylmethyl)amino)ethyl)-4-(4-pyridylcyanomethyl) piperazine, dihydrochloride**

**a) 1-(2-(*N*-(diphenylmethyl)amino)ethyl)-4-(4-pyridylcyanomethyl) piperazine**

To a solution of 2.0 g (8.1 mmol) of *N*-diphenylmethyl-*N*-(2-chloroethyl)amine hydrochloride and 2.25 mL of triethylamine in 15 mL chloroform, 1.3 g (6.4 mmol) of of the compound obtained in preparation 2 was added. The mixture was heated at reflux under argon atmosphere for two days. The solution was then washed with water, the organic phase was separated and dried. The solvent was removed yielding 2.58 g of crude that was purified by chromatography on silica gel (ethyl acetate: methanol, 3%) affording 0.380 g of a colorless oil (15% yield).
$^1H$ NMR (80MHz, $CDCl_3$) δ (TMS):
8.65 (m, 2H, pyr),
7.34 (m, 12H, pyr + Ar),
4.84 (m, 1H),
4.81 (m, 1H),
2.54 (m, 13H).

**b) Title compound of the example**

Following the procedure described in example 19b, but using the compound obtained in example 61a, the title compound of this example was obtained as a white solid in a similar yield.
mp: 179.7-184.6° C;
IR(KBr) ν : 3406, 2649, 1631, 1605, 1494, 1448 $cm^{-1}$;
Analysis calcd. for : $C_{26}N_5H_{29}$ . 2. 1/2HCl . $H_2O$: C 59.97 %, H 6.43%, N 13.45%; Found: C 59.84%, H 6.62%, N 13.90%.

**EXAMPLE 62**

**1-(2-(*N*-(diphenylacetyl)amino)ethyl)-4-(3-pyridylcyanomethyl)piperazine**

Following the procedure described in example 23 but using as starting materials the compound obtained in preparation 8 and diphenylacetic acid, the title compound of this example was obtained as a white solid (19% yield).
mp: 58.1-66.6 ° C;
IR(KBr) ν : 3306, 3023, 2939, 2820, 2240, 1649, 1573, 1490, 1448, 1419 $cm^{-1}$;
$^1H$ NMR (80MHz, $CDCl_3$) δ (TMS):
8.75 (d, J = 2.4Hz, 1H, pyr),
8.62 (dd, $J_a$ = 4.8Hz, $J_b$ = 1.5Hz, 1H, pyr),
7.84 (d de t, $J_a$ = 8.4Hz, $J_b$ = 2.0Hz, 1H, pyr),
7.29 (m, 11H, pyr + Ar),

6.34 (m, 1H, CONH),
4.95 (s, 1H),
4.86 (s, 1H),
3.34 (q, J = 5.6Hz, 2H, $CH_2NHCO$),
2.41 (m, 10H);
Analysis calcd. for : $C_{27}H_{29}N_5O$ . $1/2H_2O$:C 72.29%, H 6.74%, N 15.61%;
Found: C 72.17%, H 6.62%, N 15.23%.

**EXAMPLE 63**

**1-(2-(3,3-diphenylpropanoylamino)ethyl)-4-(3-pyridylcyanomethyl) piperazine, trihydrochloride**

**a) 1-(2-(3,3-diphenylpropanoylamino)ethyl)-4-(3-pyridylcyanomethyl) piperazine**

Following the procedure described in example 23 but using as starting materials the compound obtained in preparation 8 and 3,3-diphenylpropanoic acid, the title compound of this example was obtained as a colorless oil (48% yield).
IR(film) $\nu$ : 3298, 3054, 3021, 2939, 2820, 1643, 1540, 1488, 1446, 1419 $cm^{-1}$;
$^1H$ NMR (80MHz, $CDCl_3$) $\delta$ (TMS):
8.76 (m, 1H, pyr),
8.62 (broad d, J = 2.4Hz, 1H, pyr),
7.84 (broad d, J = 8.7Hz, 1H, pyr),
7.24 (m, 11H, pyr + Ar),
5.86 (m, 1H, NH),
4.87 (s, 1H, CHCN),
4.54 (t, J = 7.7Hz, 1H, $CHPh_2$),
3.16 (q, J =5.3Hz, 2H, $CONHCH_2$),
2.91 (d, J = 7.8Hz, 2H, $Ph_2CH\underline{CH_2}$)
2.50 (m, 4H, pip),
2.29 (m, 6H).

**b) Title compound of the example**

Following the procedure described in example 19b, but using the compound obtained in example 63a, the title compound of this example was obtained as a white solid (93%yield)
mp: 134.9-151.0 °C;
Analysis calcd. for: $C_{28}H_{31}N_5O$ . 3HCl: C 59.73%, H 6.08%, N 12.43%.
Found: C 59.65%, H 6.23%, N 12.06%.

**EXAMPLE 64**

**1-((*N*-(3,3-diphenylpropyl)aminocarbonyl)methyl)-4-(3-pyridylcyanomethyl)piperazine**

Following the procedure described in example 34 but using the compound obtained in preparation 1 and the one prepared in preparation 9 the title compound of the example was obtained as a white solid (80%yield).
mp: 120.2-120.8 °C;
IR(KBr) $\nu$ : 3357,3020, 2937, 2821, 1659, 1518, 1488, 1448, 1429, 1294 $cm^{-1}$;
$^1H$ NMR (80MHz, $CDCl_3$) $\delta$ (TMS):
8.78 (d, J = 2.0Hz, 1H, pyr),
8.63 (dd, $J_a$ = 4.7Hz, $J_b$ = 1.3Hz, 1H, pyr),
7.82 (d de t, $J_a$ = 1.9Hz, $J_b$ = 7.8Hz, 1H, pyr),
7.27 (m, 11H, Ar + pyr),
4.91 (s, 1H, CHCN),
3.96 (t, J = 7.7Hz, 1H, $Ph_2CH$),
3.27 (q, J = 6.6Hz, 2H, $CH_2NHCO$),
2.93 (s, 2H, $COCH_2N$),
2.57 (m, 9H),

2.33 (t, J = 7.7Hz, 2H, $Ph_2CHCH_2$),
Analysis calcd. for : $C_{28}H_{31}N_5O$ . 3/4 $H_2O$: C 71.93%; H 6.95%; N 14.98%.
Found: C 72.22%, H 6.76%, N 14.79%.

## EXAMPLE 65

### 1-((*N*-diphenylmethyl)aminocarbonyl)-4-(3-pyridylcyanomethyl) piperazine

To a solution of 0.67 g (3.3 mmol) of the compound obtained in preparation 1 in 30 mL pyridine, was added 1 g (3,3 mmol) of the compound prepared in preparation 4. The mixture was heated at reflux for two days. After cooling, it was poured into 200 mL water, extracted with $CH_2Cl_2$ and dried over $Na_2SO_4$. The solvent was removed yielding a crude that was purified by chromatography on silica gel eluting with the mixture $CHCl_3$/MeOH/$NH_3$ (60:2:0.2). 0.28 g (21% yield) of a solid were obtained. Recrystallization in acetonitrile afforded 0.151 g of a white solid (11% yield).
mp: 204.3-205.2° C;
IR(KBr) $\nu$ : 3311, 2996, 2948, 2928, 2227, 1612, 1524, 1492, 1411, 1293, 1236, 1135 $cm^{-1}$;
$^1H$ NMR (80MHz, $CDCl_3$) $\delta$ (TMS):
8.70 (m, 2H, pyr),
7.87 (broad d, J = 7.6 Hz, 1H, pyr),
7.27 (m, 11H),
6.56 (d, J = 7.6Hz, 1H, NH),
6.13 (d, J = 7.6Hz, 1H, $CHPh_2$),
5.07 (s, 1H, CHCN),
3.51 (m, 4H, pip),
2.58 (m, 4H, pip).
Analysis calcd. for : $C_{25}H_{25}N_5O$ . 0.5 $H_2O$: C 71.43%; H 6.19%; N 16.67%.
Found: C 71.53%, H 5.90%, N 16.70%.

## EXAMPLE 66

### 1-(*N*-(3,3-diphenylpropyl)aminocarbonyl)-4-(3-pyridylcyanomethyl) piperazine, dihydrochloride

#### a) 1-(*N*-(3,3-diphenylpropyl)aminocarbonyl)-4-(3-pyridylcyanomethyl) piperazine

Following a procedure analogous to that described in example 65, but using the product obtained in preparation 5 instead of the one prepared in preparation 4, the title compound of this example was obtained as a colorless oil (46% yield).
IR(film) $\nu$:3600-3100, 3020, 2934, 2226, 1624, 1536, 1487, 1447, 1259, 1000 $cm^{-1}$;
$^1H$ NMR (80MHz, $CDCl_3$) $\delta$ (TMS):
8.70 (m, 2H, pyr),
7.84 (broad d, J = 7.6Hz, 1H, pyr),
7.23 (m, 11H),
4.86 (s, 1H, CHCN),
4.47 (t, J = 5Hz, 1H, NH),
3.94 (t, J = 7.5Hz, 1H, $CHPh_2$),
3.21 (m, 6H),
2.46 (m, 6H).

#### b) Title compound of the example

Following a procedure analogous to that described in example 34b, but using the compound prepared in example 66a the title compound of this example was obtained as a white solid (yield 65%).
mp: 95.5-101.7° C;
IR(KBr) $\nu$ : 3600-2000.1625, 1536, 1446, 1260, 1000, 752, 705 $cm^{-1}$;
$^1H$ NMR (80MHz $D_2O$) $\delta$ (TMS):
9.00 (m, 2H, pyr),
8.63 (m, 1H, pyr),

8.15 (m, 1H, pyr),
7.24 (m, 10H, Ph),
5.53 (s, 1H, CHCN),
4.2-2.0 (m, 14H).
Analysis calcd. for : $C_{27}H_{31}Cl_2N_5O.2.5H_2O$: C 58.17%; H 6.46%; N 12.57%.
Found: C 58.20%, H 6.13%, N 12.58%.

**EXAMPLE 67**

**1-(3,3-diphenylpropanoyl)-4-(4-pyridylcyanomethyl)piperazine, dihydrochloride**

**a) 1-(3,3-diphenylpropanoyl)-4-(4-pyridylcyanomethyl)piperazine**

Following the procedure described in example 23, but using the compound obtained in preparation 2 and 3,3-diphenylpropanoic acid, the title compound of this example was obtained as a colorless oil (13% yield).
$^1H$ NMR (80MHz, $CDCl_3$) $\delta$ (TMS):
8.63 (broad d, J = 5.8Hz, 2H, pyr),
7.42 (broad d, J = 5.8Hz, 2H, pyr),
7.22 (m, 10H, Ph),
4.78 (s, 1H, CHCN),
4.61 (t, J = 7.9Hz, 1H, $CHPh_2$),
3.52 (m, 2H, pip),
3.34 (m, 2H, pip),
3.00 (d, J = 8.0Hz, 2H, $\underline{CH_2}$-$CHPh_2$),
2.28 (m, 4H, pip).

**b) Title compound of the example**

Following the procedure described in example 19b, but using the compound prepared in example 67a, the title compound of this example was obtained as a white solid in a similar yield.
mp: 92.7-99.8° C;
Analysis calcd. for : $C_{26}H_{26}N_4O$ . 2 HCl . 1/3 $CH_3CO_2CH_2CH_3$:
C 66.61%; H 5.97%, N 10.92%. Found: C 66.17%, H 6.18%, N 10.61%.

**EXAMPLE 68**

**1-(diphenylacetyl)-4-(1-(3-pyridyl)-1-cyanoethyl)piperazine**

1.4 g (5 mmol) of the compound obtained in preparation 11, 0.5 mL (5 mmol) of 3-acetylpyridine and 0.15 g p-toluenesulfonic acid in 75 mL toluene were heated in a Dean Stark for 48 h. The solvent was removed and the residue was worked up with chloroform and 0.1N sodium hydroxide. The organic phase was separated, dried over sodium sulfate and the solvent was removed yielding 2.5 g of crude. To a solution of this crude in 7 mL glacial acetic acid were added 0.5 g potassium cyanide. The mixture was stirred at room temperature for 12 h. The solvent was removed in vacuo and the residue worked up with chloroform and 1N sodium hydroxide. The organic phase was dried over sodium sulfate and the solvent was removed affording 1,5 g of crude that were purified by chromatography on silica gel (hexane: ethyl acetate 80%) to yield 0.33 g of a white solid (16% yield).
mp: 64.1-73.9° C;
IR(KBr) $\nu$ : 3055, 3022, 2242, 1642, 1595, 1428, 1414 $cm^{-1}$;
$^1H$ NMR (80MHz $D_2O$) $\delta$ (TMS):
8.83 (m, 1H, pyr),
8.60 (m, 1H, pyr),
7.88 (broad d, J = 8.2Hz, 1H, pyr),
7.26 (m, 11H, pyr + Ar),
5.19 (s, 1H, CHCN),
3.70 (m, 2H, pip),
3.47 (m, 2H, pip),

2.48 (m, 4H, pip),
1.72 (s, 3H);
Analysis calcd. for : $C_{26}H_{26}N_4O$ . 1/2 $H_2O$:C 74.43%; H 6.48%; N 13.35%.
Found: C 74.80%, H 6.24%, N 13.01%.

**EXAMPLE 69**

**1-(diphenylacetyl)-4-(2-(3-pyridyl)-2-cyanoethyl) piperazine**

**a) 1-(diphenylacetyl)-4-(2-(3-pyridyl)-2-oxoethyl) piperazine**

To a solution of 4.44 g (15 mmol) of the compound obtained in preparation 11 in 50 mL anhydrous chloroform, was added dropwise a solution previously prepared by addition of 2.36 mL triethylamine into a suspension of 4.23 g (1,5 mmol) of 3-(2-bromoacetyl)pyridine hydrobromide in 80 mL anhydrous chloroform. The mixture was stirred under argon atmosphere, at room temperature for 18 h. Then, it was washed with 1N sodium hydroxide and dried. The solvent was removed affording 8.6 g of crude that was purified by chromatography on silica gel (chloroform: methanol, 3%) to yield 4.56 g of title compound of the section as a colorless oil (73%yield).
IR(film) $\nu$ : 2914, 1692, 1637, 1580, 1427, 1220 $cm^{-1}$;
$^1H$ NMR (80MHz, $CDCl_3$) $\delta$ (TMS):
9.75 (s, 1H, pyr),
9.30 (d, J = 4.5Hz, 1H, pyr),
8.78 (d, J = 7.5Hz, 1H, pyr),
7.79 (m, 11H, pyr + Ar),
5.74 (s, 1H, CHCN),
4.24 (m, 4H, $CH_2CO$ + pip),
4.04 (m, 2H, pip),
3.10 (m, 2H, pip),
2.83 (m, 2H, pip).

**b) 1-(diphenylacetyl)-4-(2-(3-pyridyl)-2-hydroxyethyl)piperazine**

To a solution of 4.56 g (11 mmol) of the compound prepared in example 69a in 20 mL methanol, cooled at 0° C, were added 0.64 g (16.5 mmol) of sodium borohydride and the resulting mixture was stirred at 0° C for 1/2 h. Then, 10 mL 0.1N hydrochloric acid were added. The mixture was concentrated in vacuo and worked up with water and chloroform. The organic phase was separated and dried, affording a crude that was purified by chromatography on silica gel (chloroform: methanol, 5%) to yield 2.7 g of a white solid. (62% yield).
mp: 134.2-135.4° C;
IR(KBr) $\nu$ : 3389, 2811, 1638, 1448, 1220 $cm^{-1}$;
$^1H$ NMR (80MHz, $CDCl_3$) $\delta$ (TMS):
9.20 (m, 2H, pyr),
8.52 ( broad d, J = 7.6Hz, 1H, pyr),
7.99 (m, 11H, pyr + Ar),
5.93 (s, 1H, $CHPh_2$),
5.47 (t, J = 6.4Hz, 1H, CHOH),
4.47 (m, 2H, pip),
4.23 (m, 2H, pip),
3.19 (d, J = 6.5, 2H, $\underline{CH_2}$CHOH),
3.15 (m, 4H, pip);
Analysis calcd. for: $C_{25}H_{27}N_3O_2$:C 74.79%; H 6.78%; N 10.47%.
Found: C 74.48%, H 6.80%, N 10.16%.

**c) 1-(diphenylacetyl)-4-(2-(3-pyridyl)-2-cyanoethyl) piperazine**

To a solution, cooled to 0° C, of 1.6 g (4 mmol) of the compound prepared in example 69b in 8 mL anhydrous chloroform, 0.32 mL of thionyl chloride dissolved in 4 mL dry chloroform were added under argon atmosphere. The mixture was stirred 1 h at room temperature and 1 h at reflux. The solvents were

evaporated and the residue diluted with 20 mL chloroform. Then, 1.7 mL triethylamine were added and the solution washed with water. The organic phase was separated, dried over sodium sulfate and the solvent was removed affording a crude that was dissolved in 10 mL dimethylsulfoxide. To this solution were added 0.58 g potassium cyanide. The mixture was heated at 90° C for 6 h, the solvents were evaporated in vacuo and the residue worked up with chloroform 0.1 N solution of sodium hydroxide. The organic solution was dried and, after removal of the solvent, the crude was purified by chromatography on silica gel (chloroform; methanol, 5%) yielding 0.15 g of the title compound of this example as a white solid (10%yield).
mp: 56.9-64.8° C;
$^1$H NMR ($CDCl_3$ + DMSO $d_6$) $\delta$ (TMS):
9.18 (m, 1H, pyr),
8.81 (m, 2H, pyr),
7.94 (dd, Ja = 8.0 Hz, Jb = 5.5Hz, 1H, pyr),
7.22 (m, 10H, Ph),
5.20 (m, 1H, $ChPh_2$),
4.71 ( t, J = 6.9Hz, 1H, CHCN),
3.85 (m, 2H, pip),
3.67 (m, 2H, pip),
3.45 (d, J = 7.0Hz, 2H, $\underline{CH_2}$CHCN),
2.82 (m, 2H, pip),
2.56 (m, 2H, pip);
$^{13}$CNMR (20.15 MHz, $CDCl_3$) $\delta$ (TMS): 170.03 (C), 161.00 (CH), 149.87 (CH), 149.37 (CH), 139.05 (C), 139.02 (C), 135.20 (CH) 133.05 (C), 129.02 (CH), 128.70 (CH), 128.56 (CH), 127.23 (CH), 127.06 (CH), 123.65 (CH), 117.05 (C), 62.79 (CH), 54.69 (CH), 49.76 ($CH_2$), 45.91 ($CH_2$), 42.09 ($CH_2$), 21.68 ($CH_2$).
Analysis calcd. for : $C_{26}H_{26}N_4O$ . 1/2 $H_2O$: C 74.44%, H 6.48 %, N 13.35%.
Found: C 74.30%, H 6.58%, N 12.82%.

**EXAMPLE 70**

**(1-(3,3-diphenylpropanoyl)-4-piperidinyliden)-3-pyridylacetonitrile**

Following the procedure described in example 23, but using 3,3-diphenylpropanoic acid and the compound obtained in preparation 13 the title compound of this example was obtained as a white solid (65%yield).
mp: 49.7-59.2 ° C;
IR(KBr) $\nu$ : 3021, 2209, 1632, 1580, 1488, 1446, 1268 $cm^{-1}$;
$^1$H NMR (80MHz, $CDCl_3$) $\delta$ (TMS):
8.55 (m, 2H, pyr),
7.58 (d of t, Ja = 8.0Hz, Jb = 1.7Hz, 1H, pyr),
7.24 (m, 11H, pyr + Ar),
4.67 (t, J = 7.7Hz, 1H, $CHPh_2$),
3.58 (m, 4H, pip),
3.10 (m, 2H, pip),
2.40 (m, 4H, pip);
Analysis calcd. for: $C_{27}H_{25}N_3O$ . 1/4 $H_2O$: C 78.90%, H 6.25%, N 10.22%.
Found: C 78.64%, H 5.95%, N 10.22%.

**EXAMPLE 71**

**1-(diphenylacetyl)-4-(3-pyridylcyanomethyl)piperidine**

Following the procedure described in example 23, but using 2,2-diphenylacetic acid and the compound obtained in preparation 15 the title compound of this example was obtained as a white solid (45%yield).
mp: 159.2-160.2° C;
IR(KBr) $\nu$ : 3050, 2920, 2241, 1625, 1491, 1447, 1429 $cm^{-1}$;
$^1$H NMR (80MHz, $D_2O$) $\delta$ (TMS):
8.55 (m, 2H, pyr),
7.60 (broad d, J = 8.2Hz, 1H, pyr),
7.25 (m, 11H, pyr + Ar),

5.16 (s, 1H, $CHPh_2$),
4.78 (m, 1H, pip),
4.00 (m, 1H, pip),
3.60 (d, J = 6.7Hz, 1H, CHCN),
2.68 (m, 2H, pip),
2.0-1.0 (complex signal, 5H);
Analysis calcd. for : $C_{26}H_{25}N_3O$: C 78.96%, H 6.37%, N 10.62%. Found: C 79.46%, H 6.16%, N 10.47%.

**EXAMPLE 72**

**1-(4(2-(1,2,3,4-Tetrahydroisoquinolyl)carbonyloxy)butanoyl)-4-(3-pyridylcyanomethyl)piperazine, monohydrochloride.**

**a)1-(4(2-(1,2,3,4-Tetrahydroisoquinolyl)carbonyloxy)butanoyl)-4-(3-pyridylcyanomethyl)piperazine**

Following the procedure described in example 23, but using the compound obtained in preparation 16 instead of trimethoxyphenylacetic acid the title compound of this example was obtained as an oil (65% yield).
$^1H$ NMR (80MHz, $CDCl_3$) $\delta$ ( TMS):
8.75 (m, 2 H, pyr),
7.92 (m, 2 H, pyr)
7.14 (s, 4 H, Ph),
4.96 (s, 1 H, CHCN),
4.60 (s, 2 H, $CH_2N$),
4.18 (t, J = 5.8 Hz, 2 H, $CH_2O$),
3.64 (m, 6 H),
2.87 (m, 2 H, $CH_2N$),
2.57 (m, 4 H, pip),
2.42 (m, 2 H, $CH_2CO$),
2.97 (m, 2 H, $CH_2$).

**b)1-(4(2-(1,2,3,4-Tetrahydroisoquinolyl)carbonyloxy)butanoyl)-4-(3-pyridylcyanomethyl)piperazine, hydrochloride.**

Following the procedure described in example 34b, but using the compound prepared in example 72a the title compound of this example was obtained as a white solid (54 % yield).
mp: 36.1-39.6° C;
IR(KBr) $\nu$ : 3600-3200, 1685, 1632, 1427, 1227, 1121 $cm^{-1}$;
$^1H$ NMR (80MHz, $D_2O$) $\delta$ (TMS):
8.89 (m, 3 H, pyr),
8.21 (m, 1 H, pyr),
7.16 (s, 4 H, Ph),
5.55 (s, 1 H, CHCN),
4.78 (s, $H_2O$),
4.55 (s, 2 H, $CH_2N$),
4.16 (t, J = 5.7 Hz, 2 H, $CH_2O$),
3.59 (m, 6 H),
2.82 (t, J = 5.8 Hz, 2 H, $CH_2N$),
2.54 (m, 6 H),
2.00 (m, 2 H).
Analysis calcd. for : $C_{25}H_{30}N_5O_3Cl$: C 58.76%, H 6.46%, N 13.71%.
Found: C 59.06%, H6.72%, N 13.41%.

**EXAMPLE 73**

**1-(3(2-(1,2,3,4-Tetrahydroisoquinolyl)carbonyloxy)propanoyl)-4-(3-pyridylcyanomethyl)piperazine, hydrochloride.**

**a)1-(3(2-(1,2,3,4-Tetrahydroisoquinolyl)carbonyloxy)propanoyl)-4-(3-pyridylcyanomethyl)piperazine**

Following the procedure described in example 23, but using the acid obtained in preparation 17 instead of trimethoxyphenylacetic acid the title compound of this example was obtained as a white solid (77%yield).

IR(KBr) $\nu$ : 2921, 2846, 2244, 1691, 1637, 1422, 1224, 1097 $cm^{-1}$;
$^{1}H$ NMR (80MHz, $CDCl_3$) $\delta$ (TMS):
8.70 (m, 2 H, pyr),
7.85 (m, 1H, pyr),
7.43 (m, 1 H, pyr),
7.15 (s, 4 H, Ph),
4.92 (s, 1 H, CHCN),
4.60 (s, 2 H, $CH_2N$),
4.45 (t, J = 6.6 Hz, 2 H $CH_2O$),
3.60 (m, 6 H),
2.70 (m, 8 H).

**b)1-(3(2-(1,2,3,4-Tetrahydroisoquinolyl)carbonyloxy)propanoyl)-4-(3-pyridylcyanomethyl)piperazine**

Following the procedure described in example 34b, but using the compound obtained in example 73a the title compound of this example was obtained as a white solid (39 %yield).
mp: 91.0-93.7 °C;
IR(KBr) $\nu$ : 37001900, 1686, 1632, 1427, 1224, 1120, 1000$cm^{-1}$;
$^{1}H$ NMR (80MHz, $D_2O$) $\delta$ (TMS):
8.97 (m, 3 H, pyr),
8.27 (m, 1 H, pyr),
7.17 (s, 4 H, Ph),
5.66 (s, 1 H, CHCN),
4.87 (s, $H_2O$),
4.54 (m, 4 H),
3.62 (m, 6 H),
2.80 (m, 8H).
Analysis calcd. for : $C_{24}H_{29}N_5O_3Cl_2$: C 54.04%, H 6.00%, N 13.13%.
Found: C 53.92%, H 5.75%, N 12.38%.

## Claims

1. Compounds of formula **I**:

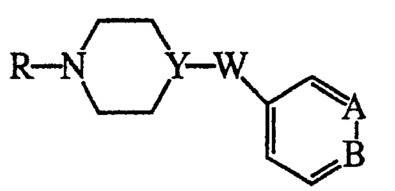

**I**

wherein **A** is nitrogen and **B** is -CH-, or **B** is nitrogen and **A** is -CH-; the group

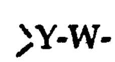

represents a group of formula

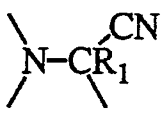 , 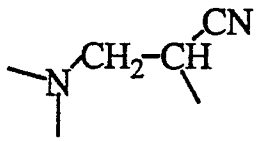 , 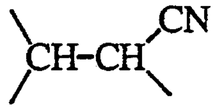 or 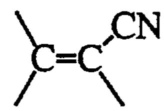

wherein $R_1$ is hydrogen or a $C_1$-$C_6$ alkyl group;
**R** represents a group of formula

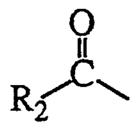 , 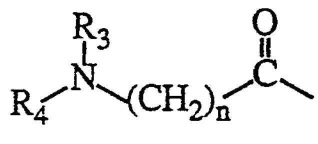 or 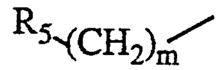

wherein $R_2$ represents $C_1$-$C_{15}$ alkyl, aryl, aryl-($C_1$-$C_6$)-alkyl, aryl-($C_1$-$C_6$)-alkenyl, diaryl-($C_1$-$C_6$)-alkyl, diaryl-($C_1$-$C_6$)-alkenyl, arylhydroxy-($C_1$-$C_6$)-alkyl, diarylhydroxy-($C_1$-$C_6$)-alkyl, aryl-($C_1$-$C_6$)-alkoxy, diaryl-($C_1$-$C_6$)-alkoxy, heteroaryl-($C_1$-$C_6$)-alkyl group or a group of formula

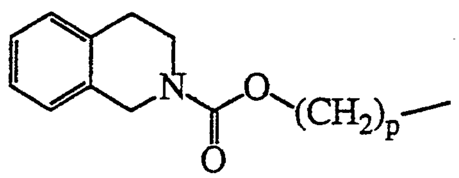

where p = 2 or p = 3;
n is 0, 1, 2 or 3;
$R_3$ is hydrogen, $C_1$-$C_6$ alkyl, aryl or aryl-($C_1$-$C_6$)-alkyl group;
$R_4$ is $C_3$-$C_6$ cycloalkyl, aryl, aryl-($C_1$-$C_6$)-alkyl, diaryl-($C_1$-$C_6$)-alkyl, aryl-($C_1$-$C_6$)-alkylcarbonyl or diaryl-($C_1$-$C_6$)-alkylcarbonyl group, or $R_3R_4N$- is a group of formula

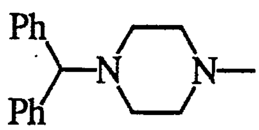 or 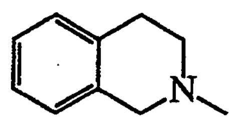

m is 0, 1 or 2;
$R_5$ is aryl-($C_1$-$C_6$)-alkyl, diaryl-($C_1$-$C_6$)-alkyl, diaryl-($C_1$-$C_6$)-alkylcarbonylamino or diaryl-($C_1$-$C_6$)-alkylaminocarbonyl group; being understood that the term "aryl" (or its symbol Ar) means phenyl or a radical from a fused or unfused benzenoid hydrocarbon (e.g. naphtyl, biphenyl, fluorenyl), and can be optionally substituted by one, two or three $C_1$-$C_6$ alkyl, $C_1$-$C_6$ haloalkyl, $C_1$-$C_6$ alkoxy, halogen, nitro, cyano, hydroxy, $C_1$-$C_6$ alkylcarbonyl, $C_1$-$C_6$ alkylcarbonyloxy, $C_1$-$C_6$ alkyloxycarbonyl, $C_1$-$C_6$ alkylsulfonyl, $C_1$-$C_6$ alkylsulfinyl and $C_1$-$C_6$ alkylthio group; and that the term "heteroaryl" means any radical from a 5 or 6 membered heterocycle, isolated or condensed with one benzene ring, with one ring heteroatom chosen from N, O or S, or with two ring heteroatoms chosen from the pairs N/N, N/O or N/S.

2. Compounds according to claim 1 of formula **Ia**

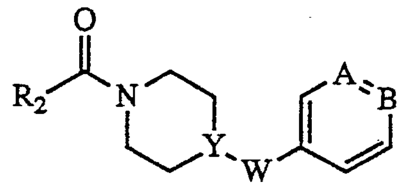

**Ia**

wherein **A**, **B** and the group

>Y-W-

have the previously defined meaning and $R_2$ is a $C_1$-$C_{15}$ alkyl radical, a group of formula $Ar(CH_2)_q$-,ArCHMe-, $(Ar)_2CH$-$(CH_2)_q$-, $(Ar)_2C(Me)$-, $ArCH_2$-CHAr-, $ArCH_2$-$CH_2$-CHAr-, $ArCH_2$-$CH(CH_2Ar)$-, ArCH=CH-, ArCH=CAr-, $(Ar)_2C$=CH-, ArCH(OH)-$(CH_2)_q$-, $(Ar)_2C(OH)$-$(CH_2)_q$-, $(Ar)_2CH$-$(CH_2)_q$-O-, or Ar-$(CH_2)_q$-O- wherein q is an integer from 0 to 3 and Ar has the previously defined meaning, an heteroaryl-($C_1$-$C_6$)-alkyl group, or a group of formula

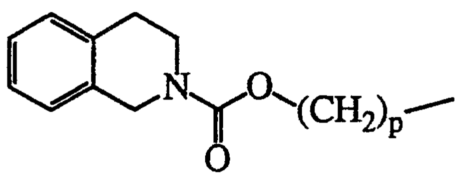

where p is 2 or 3.

**3.** Compounds according to claim 1 of formula **Ib**

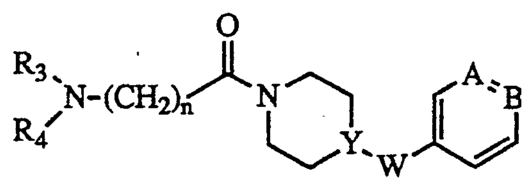

**Ib**

wherein **A**, **B**, the group

>Y-W-,

n and $R_3$ have the previously defined meaning and $R_4$ is a group of formula $Ar(CH_2)_q$-, ArCHMe-, $(Ar)_2CH$-, $(Ar)_2C(Me)$-, $(Ar)_2CH$-$(CH_2)_q$-, $ArCH_2$-CHAr-, Ar(C=O)-, or $(Ar)_2CH(C=O)$-, wherein q and Ar have the previously defined meaning, cyclohexyl, or $R_3R_4N$- is a group of formula

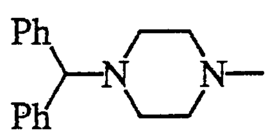 or 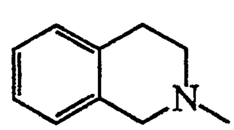

**4.** Compounds according to claim 1 of formula **Ic**

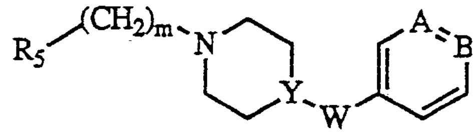

**Ic**

wherein **A**, **B**, the group

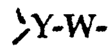

and m have the previously defined meaning and $R_5$ is a group of formula $Ar(CH_2)_q$-, $(Ar)_2CH-(CH_2)_q$-, $(Ar)_2CH$-NH-, $(Ar)_2CH-(CH_2)_q-(C=O)NH$-, or $(Ar)_2CH-(CH_2)_q-NH(C=O)$-, wherein q and Ar have the previously defined meaning.

**5.** Compounds according to claim 2 of formula **Ia**

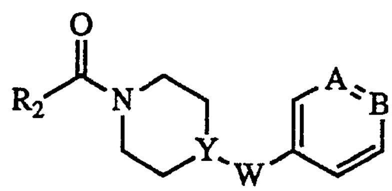

**Ia**

wherein **A** and **B** have the previously defined meaning; the group

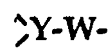

represents:

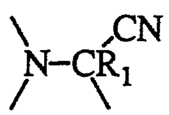 , 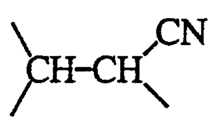 or 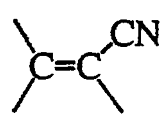

wherein $R_1$ have the previously defined meaning; and $R_2$ is a group of formula ArCHMe-, $(Ar)_2CH-(CH_2)_q$-, $(Ar)_2C(Me)$-, $ArCH_2$-CHAr-, $ArCH_2-CH_2$-CHAr-, $ArCH_2-CH(CH_2Ar)$-, ArCH=CH-, ArCH=CAr-, $(Ar)_2C=CH$-, $(Ar)_2C(OH)-(CH_2)_q$-, or $(Ar)_2CH-(CH_2)_q$-O-, wherein q and Ar have the previously defined meaning.

**6.** Compounds according to claim 3 of formula **Ib**

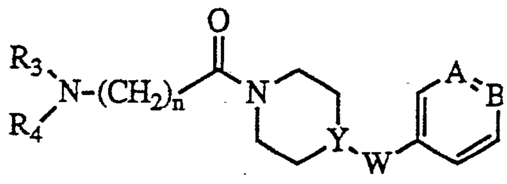

**Ib**

wherein **A**, **B**, and n have the previously defined meaning; the group

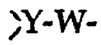

represents:

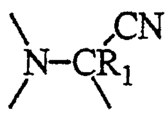 , 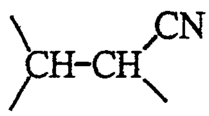 or 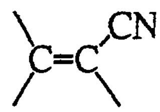

wherein $R_1$ have the previously defined meaning; $R_3$ is hydrogen, Ar or $Ar(CH_2)_q$-, and $R_4$ is a group of formula $Ar(CH_2)_q$-, ArCHMe-, $(Ar)_2CH$-, $(Ar)_2C(Me)$-, $(Ar)_2CH$-$(CH_2)_q$-, $ArCH_2$-CHAr-, Ar(C = O)-, or (Ar)-$_2$CH-(C = O)-, wherein q and Ar have the previously defined meaning.

**7.** Compounds according to claim 5 of formula **Ia**

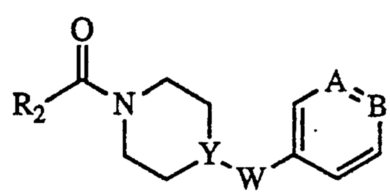

**Ia**

wherein **A** and **B** have the previously defined meaning; the group

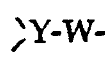

represents:

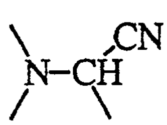 or 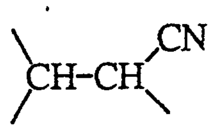

and $R_2$ is a group of formula $(Ph)_2CH$-, $(Ph)_2CH$-$CH_2$, $(Ph)_2C$ = CH- or $(Ph)_2C(OH)$-$CH_2$-.

**8.** Compounds according to claim 6 of formula **Ib**

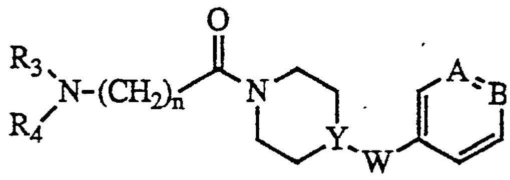

**Ib**

wherein **A** and **B** have the previously defined meaning; the group

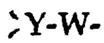

represents:

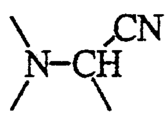 or 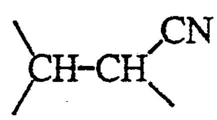

n is 1 or 2; $R_3$ is hydrogen or methyl and $R_4$ is PhCHMe- or $(Ph)_2CH$-.

**9.** 1-(3,3-diphenylpropanoyl)-4-(3-pyridylcyanomethyl)piperazine.

**10.** 1-(2-phenylmethyl-3-phenylpropanoyl)-4-(3-pyridylcyanomethyl)piperazine.

**11.** 1-(diphenylacetyl)-4-(3-pyridylcyanomethyl)piperazine.

**12.** 1-(2-phenylpropanoyl)-4-(3-pyridylcyanomethyl)piperazine.

**13.** 1-(2,2-diphenylpropanoyl)-4-(3-pyridylcyanomethyl)piperazine.

**14.** 1-(3,3-diphenylpropenoyl)-4-(3-pyridylcyanomethyl)piperazine.

**15.** 1-(3,3-bis(4-methoxyphenyl)propanoyl)-4-(3-pyridylcyanomethyl)piperazine.

**16.** 1-(3,3-diphenyl-3-hydroxypropanoyl)-4-(3-pyridylcyanomethyl)piperazine.

**17.** 1-(3,3-diphenyl-3-hydroxypropanoyl)-4-(4-pyridylcyanomethyl)piperazine.

**18.** 1-(*N*-(diphenylmethyl)aminoacetyl)-4-(3-pyridylcyanomethyl)piperazine, trihydrochloride.

**19.** 1-(3-(*N*-(diphenylmethyl)amino)propanoyl)-4-(3-pyridylcyanomethyl)piperazine.

**20.** 1-(3-(*N*-(diphenylmethyl)amino)propanoyl)-4-(4-pyridylcyanomethyl)piperazine.

**21.** (1-(3,3-diphenylpropanoyl)-4-piperidiniliden)-3-pyridylacetonitrile.

**22.** 1-(3,3-diphenylpropanoyl)-4-(4-pyridylcyanomethyl)piperazine, dihydrochloride.

**23.** 1-(diphenylacetyl)-4-(3-pyridylcyanomethyl)piperidine.

**24.** 1-(diphenylacetyl)-4-(1-(3-pyridyl)-1-cyanoethyl) piperazine.

**25.** A process for preparing a compound according to any one of claims 1 to 24, which comprises either

reacting the amine of the following formula:

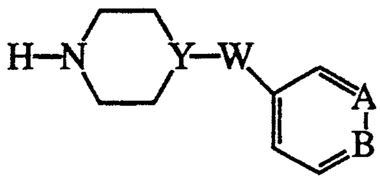

with an acid $R_2COOH$ or an equivalent acylating reagent, when R is $R_2CO$-; with an acid $R_3R_4N-(CH_2)_n-COOH$ or a equivalent acylating reagent, when R is $R_3R_4N-(CH_2)_n-CO$-; with an halide or alkyl sulfonate $R_5(CH_2)_m-X$ or equivalent alkylating reagent, when R is $R_5(CH_2)_m$-;
or reacting the amine of the following formula:

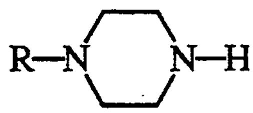

with the aldehyde or ketone of the following formula:

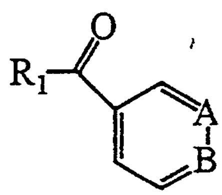

in the presence of potassium cyanide in a suitable solvent, when

>Y-W-

is

>N-$CR_1$(CN)-;

or reacting the intermediate of the following formula:

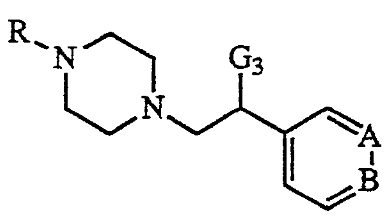

where $G_3$ is a good leaving group, with potassium cyanide in a suitable solvent, when

>Y-W- is >N-$CH_2$-CH(CN)-.

26. A pharmaceutical composition comprising a compound according to any one of claims 1 to 24 and a pharmaceutically acceptable diluent or carrier.

27. A pharmaceutical composition according to claim 26 comprising a compound according to any one of claims 9 to 24 and a pharmaceutically acceptable diluent or carrier.

28. The use of a compound according to any of the claims 1 to 24 in the manufacture of a medicament for the treatment or prophylaxys of PAF-mediated illnesses

**Claims for the following Contracting States: GR, ES**

1. A process for preparing a compound of formula **I**:

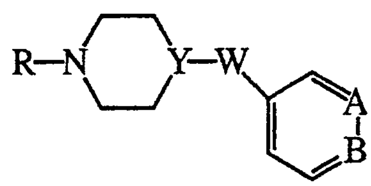

**I**

wherein **A** is nitrogen and **B** is -CH-, or **B** is nitrogen and **A** is -CH-; the group

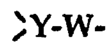

represents a group of formula

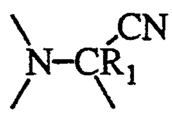 , 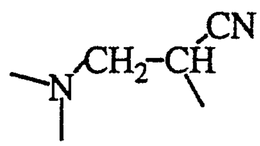 , 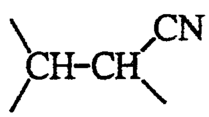 or 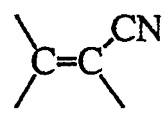

wherein $R_1$ is hydrogen or a $C_1$-$C_6$ alkyl group;
**R** represents a group of formula

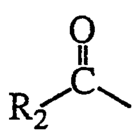 , 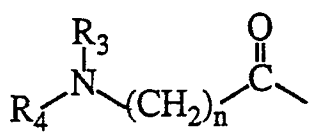 or 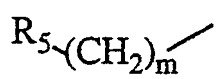

wherein $R_2$ represents $C_1$–$C_{15}$ alkyl, aryl, aryl-($C_1$-$C_6$)-alkyl, aryl-($C_1$-$C_6$)-alkenyl, diaryl-($C_1$-$C_6$)-alkyl, diaryl-($C_1$-$C_6$)-alkenyl, arylhydroxy-($C_1$-$C_6$)-alkyl, diarylhydroxy-($C_1$-$C_6$)-alkyl, aryl-($C_1$-$C_6$)-alkoxy, diaryl-($C_1$-$C_6$)-alkoxy, heteroaryl-($C_1$-$C_6$)-alkyl group or a group of formula

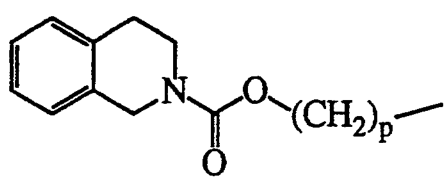

where p = 2 or p = 3;

n is 0, 1, 2 or 3;
$R_3$ is hydrogen, $C_1$-$C_6$ alkyl, aryl or aryl-($C_1$-$C_6$)-alkyl group;
$R_4$ is $C_3$-$C_6$ cycloalkyl, aryl, aryl-($C_1$-$C_6$)-alkyl, diaryl-($C_1$-$C_6$)-alkyl, aryl-($C_1$-$C_6$)-alkylcarbonyl or diaryl-($C_1$-$C_6$)-alkylcarbonyl group, or
$R_3R_4N$- is a group of formula

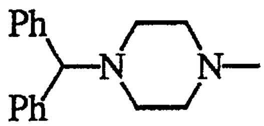

or

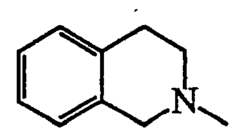

m is 0, 1 or 2;
$R_5$ is aryl-($C_1$-$C_6$)-alkyl, diaryl-($C_1$-$C_6$)-alkyl, diaryl-($C_1$-$C_6$)-alkylcarbonylamino or diaryl-($C_1$-$C_6$)-alkylaminocarbonyl group; being understood that the term "aryl" (or its symbol Ar) means phenyl or a radical from a fused or unfused benzenoid hydrocarbon (e.g. naphtyl, biphenyl, fluorenyl), and can be optionally substituted by one, two or three $C_1$-$C_6$ alkyl, $C_1$-$C_6$ haloalkyl, $C_1$-$C_6$ alkoxy, halogen, nitro, cyano, hydroxy, $C_1$-$C_6$ alkylcarbonyl, $C_1$-$C_6$ alkylcarbonyloxy, $C_1$-$C_6$ alkyloxycarbonyl, $C_1$-$C_6$ alkylsulfonyl, $C_1$-$C_6$ alkylsulfinyl and $C_1$-$C_6$ alkylthio group; and that the term "heteroaryl" means any radical from a 5 or 6 membered heterocycle, isolated or condensed with one benzene ring, with one ring heteroatom chosen from N, O or S, or with two ring heteroatoms chosen from the pairs N/N, N/O or N/S; which comprises:
either reacting the amine of the following formula:

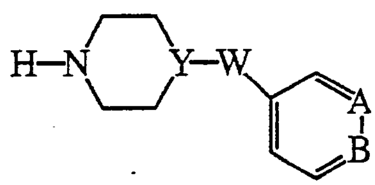

with an acid $R_2COOH$ or an equivalent acylating reagent, when R is $R_2CO$-; with an acid $R_3R_4N$-$(CH_2)_n$-COOH or a equivalent acylating reagent, when R is $R_3R_4N$-$(CH_2)_n$-CO-; with an halide or alkyl sulfonate $R_5(CH_2)_m$-X or equivalent alkylating reagent, when R is $R_5(CH_2)_m$-;
or reacting the amine of the following formula:

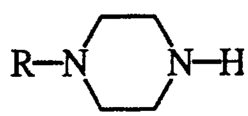

with the aldehyde or ketone of the following formula:

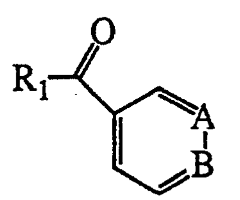

in the presence of potassium cyanide in a suitable solvent, when

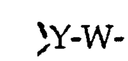

is

$$>N-CR_1(CN)-;$$

or reacting the intermediate of the following formula:

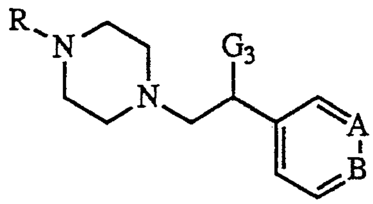

where $G_3$ is a good leaving group, with potassium cyanide in a suitable solvent, when

$$>Y-W- \text{ is } >N-CH_2-CH(CN)-.$$

2. A process according to claim 1 in which the reagents and reaction conditions are so chosen as to prepare a compound of formula **Ia**

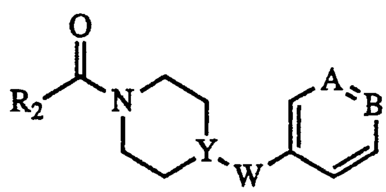

**Ia**

wherein **A**, **B** and the group

$$>Y-W-$$

have the previously defined meaning and $R_2$ is a $C_1$-$C_{15}$ alkyl radical, a group of formula $Ar(CH_2)_q$-,ArCHMe-, $(Ar)_2CH-(CH_2)_q$-, $(Ar)_2C(Me)$-, $ArCH_2$-CHAr-, $ArCH_2-CH_2$-CHAr-, $ArCH_2-CH(CH_2Ar)$-, ArCH = CH-, ArCH = CAr-, $(Ar)_2C$ = CH-, $ArCH(OH)-(CH_2)_q$-, $(Ar)_2C(OH)-(CH_2)_q$-, $(Ar)_2CH-(CH_2)_q$-O-, or $Ar-(CH_2)_q$-O- wherein q is an integer from 0 to 3 and Ar has the previously defined meaning, an heteroaryl-($C_1$-$C_6$)-alkyl group, or a group of formula

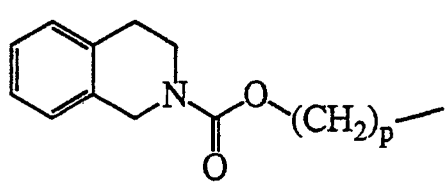

where p is 2 or 3.

3. A process according to claim 1 in which the reagents and reaction conditions are so chosen as to prepare a compound of formula **Ib**

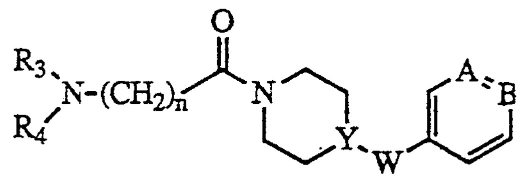

**Ib**

wherein **A, B**, the group

>Y-W-,

n and $R_3$ have the previously defined meaning and $R_4$ is a group of formula $Ar(CH_2)_q$-, ArCHMe-, $(Ar)_2CH$-, $(Ar)_2C(Me)$-, $(Ar)_2CH-(CH_2)_q$-, $ArCH_2$-CHAr-, Ar(C=O)-, or $(Ar)_2CH(C=O)$-, wherein q and Ar have the previously defined meaning, cyclohexyl, or $R_3R_4N$- is a group of formula

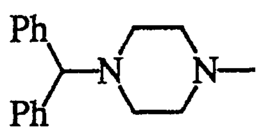

or

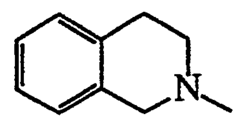

4. A process according to claim 1 in which the reagents and reaction conditions are so chosen as to prepare a compound of formula **Ic**

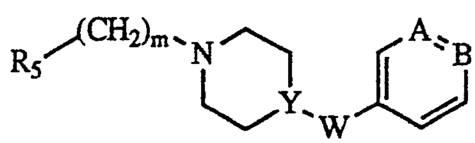

**Ic**

wherein **A**, **B**, the group

>Y-W-

and m have the previously defined meaning and $R_5$ is a group of formula $Ar(CH_2)_q$-, $(Ar)_2CH-(CH_2)_q$-, $(Ar)_2CH$-NH-, $(Ar)_2CH-(CH_2)_q$-(C=O)NH-, or $(Ar)_2CH-(CH_2)_q$-NH(C=O)-, wherein q and Ar have the previously defined meaning.

5. A process according to claim 2 in which the reagents and reaction conditions are so chosen as to prepare a compound of formula **Ia**

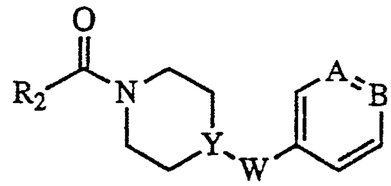

Ia

wherein **A** and **B** have the previously defined meaning; the group

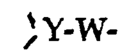

represents:

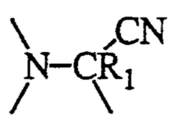 , 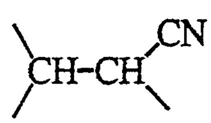 or 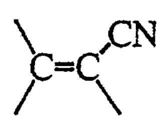

wherein $R_1$ have the previously defined meaning; and $R_2$ is a group of formula ArCHMe-, $(Ar)_2CH-(CH_2)_q$-, $(Ar)_2C(Me)$-, $ArCH_2$-CHAr-, $ArCH_2-CH_2$-CHAr-, $ArCH_2-CH(CH_2Ar)$-, ArCH=CH-, ArCH=CAr-, $(Ar)_2C$=CH-, $(Ar)_2C(OH)-(CH_2)_q$-, or $(Ar)_2CH-(CH_2)_q$-O-, wherein q and Ar have the previously defined meaning.

6. A process according to claim 3 in which the reagents and reaction conditions are so chosen as to prepare a compound of formula **Ib**

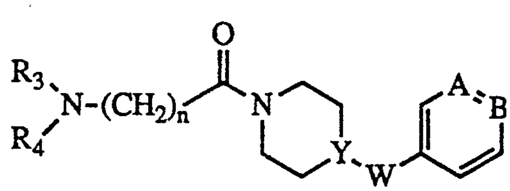

Ib

wherein **A**, **B**, and n have the previously defined meaning; the group

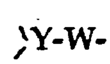

represents:

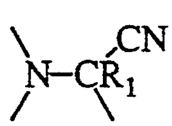 , 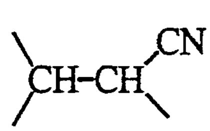 or 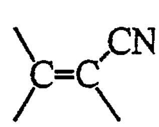

wherein $R_1$ have the previously defined meaning; $R_3$ is hydrogen, Ar or $Ar(CH_2)q$- and $R_4$ is a group of

formula $Ar(CH_2)_q$-, ArCHMe, $(Ar)_2CH$-, $(Ar)_2CMe$-, $(Ar)_2CH-(CH_2)_q$-, $ArCH_2$-CHAr-, Ar(C=O)-, or $(Ar)_2CH$-(C=O)-, wherein q and Ar have the previously defined meaning.

7. A process according to claim 5 in which the reagents and reaction conditions are so chosen as to prepare a compound of formula **Ia**

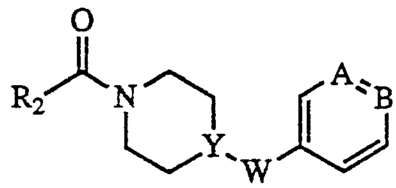

**Ia**

wherein **A** and **B** have the previously defined meaning; the group

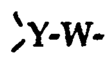

represents:

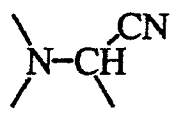 or 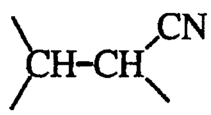

and $R_2$ is a group of formula $(Ph)_2CH$-, $(Ph)_2CH-CH_2$, $(Ph)_2C=CH$- or $(Ph)_2C(OH)-CH_2$-.

8. A process according to claim 6 in which the reagents and reaction conditions are so chosen as to prepare a compound of formula **Ib**

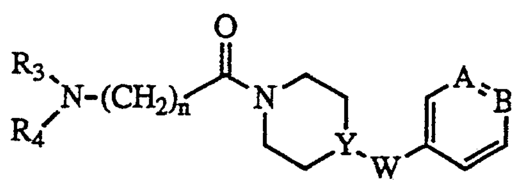

**Ib**

wherein **A** and **B** have the previously defined meaning; the group

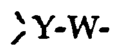

represents:

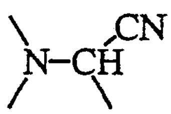 or 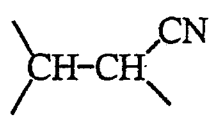

n is 1 or 2; $R_3$ is hydrogen or methyl and $R_4$ is PhCHMe- or $(Ph)_2CH$-.

**9.** A process according to claim 7 in which the reagents and reaction conditions are so chosen as to prepare 1-(3,3-diphenylpropanoyl)-4-(3-pyridylcyanomethyl)piperazine.

**10.** A process according to claim 5 in which the reagents and reaction conditions are so chosen as to prepare 1-(2-phenylmethyl-3-phenylpropanoyl)-4-(3-pyridylcyanomethyl)piperazine.

**11.** A process according to claim 7 in which the reagents and reaction conditions are so chosen as to prepare 1-(diphenylacetyl)-4-(3-pyridylcyanomethyl)piperazine.

**12.** A process according to claim 5 in which the reagents and reaction conditions are so chosen as to prepare 1-(2-phenylpropanoyl)-4-(3-pyridylcyanomethyl)piperazine.

**13.** A process according to claim 5 in which the reagents and reaction conditions are so chosen as to prepare 1-(2,2-diphenylpropanoyl)-4-(3-pyridylcyanomethyl)piperazine.

**14.** A process according to claim 5 in which the reagents and reaction conditions are so chosen as to prepare 1-(3,3-diphenylpropenoyl)-4-(3-pyridylcyanomethyl)piperazine.

**15.** A process according to claim 5 in which the reagents and reaction conditions are so chosen as to prepare 1-(3,3-bis(4-methoxyphenyl)propanoyl)-4-(3-pyridylcyanomethyl)piperazine.

**16.** A process according to claim 7 in which the reagents and reaction conditions are so chosen as to prepare 1-(3,3-diphenyl-3-hydroxypropanoyl)-4-(3-pyridylcyanomethyl)piperazine.

**17.** A process according to claim 7 in which the reagents and reaction conditions are so chosen as to prepare 1-(3,3-diphenyl-3-hydroxypropanoyl)-4-(4-pyridylcyanomethyl)piperazine.

**18.** A process according to claim 8 in which the reagents and reaction conditions are so chosen as to prepare 1-(*N* -(diphenylmethyl)aminoacetyl-4-(3-pyridylcyanomethyl)piperazine, trihydrochloride.

**19.** A process according to claim 8 in which the reagents and reaction conditions are so chosen as to prepare 1-(3-(*N* -(diphenylmethyl)amino)propanoyl)-4-(3-pyridylcyanomethyl)piperazine.

**20.** A process according to claim 8 in which the reagents and reaction conditions are so chosen as to prepare 1-(3-(*N* -(diphenylmethyl)amino)propanoyl)-4-(4-pyridylcyanomethyl)piperazine.

**21.** A process according to claim 5 in which the reagents and reaction conditions are so chosen as to prepare (1-(3,3-diphenylpropanoyl)-4-piperidiniliden)-3-pyridylacetonitrile.

**22.** A process according to claim 8 in which the reagents and reaction conditions are so chosen as to prepare 1-(3,3-diphenylpropanoyl)-4-(4-pyridylcyanomethyl)piperazine, dihydrochloride.

**23.** A process according to claim 7 in which the reagents and reaction conditions are so chosen as to prepare 1-(diphenylacetyl)-4-(3-pyridylcyanomethyl)piperidine.

**24.** A process according to claim 5 in which the reagents and reaction conditions are so chosen as to prepare 1-(diphenylacetyl)-4-(1-(3-pyridyl)-1-cyanoethyl)piperazine.

European Patent Office

# EUROPEAN SEARCH REPORT

Application Number

EP 91101126.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE - C3 - 2 215 545 (EAYT) * Claim 1 * -- | 1 | C 07 D 401/04<br>C 07 D 401/06<br>C 07 D 401/14<br>A 61 K 31/445<br>A 61 K 31/495<br>A 61 K 31/535 |
| A | DE - A1 - 3 524 955 (SANDOZ) * Claims 1,8,9; examples 8, 11-16,20-22 * -- | 1,26, 27 | |
| A | CHEMICAL ABSTRACTS, vol. 111, no. 25, December 18, 1989, Columbus, Ohio, USA KOMOTO, TERUO et al. "Preparation of substituted alkylpiperazine derivatives as drugs" page 798, column 2, abstract -No. 232 866a & Jpn. Kokai Tokkyo Koho JP 01,131,158 -- | 1, 26-28 | **TECHNICAL FIELDS SEARCHED (Int. Cl.5)** |
| A | CHEMICAL ABSTRACTS, vol. 100, no. 3, January 16, 1984, Columbus, Ohio, USA LASSLO, ANDREW et al. "Development of novel surface -active compounds for prophylaxis against and treatment of thromboembolic complications" page 15, column 1, abstract- No. 17 212b & ASAIO J. 1983, 6(2), 47-59 ---- | 1, 26-28 | C 07 D 401/00 |

The present search report has been drawn up for all claims

| Place of search | Date of completion of the search | Examiner |
|---|---|---|
| VIENNA | 02-04-1991 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document